# EUROPEAN PATENT SPECIFICATION

(11) **EP 1 508 043 B1**
(45) Date of publication and mention of the grant of the patent: **02.11.2011**
(21) Application number: 03799763.2
(22) Date of filing: 06.05.2003
(51) Int. Cl.: G01N 33/53, A61K 39/00, C07K 14/00, C07K 16/00

(54) **ANTIGENIC TARGETS OF AUTOIMMUNE SENSORINEURAL HEARING LOSS (AISNHL) AND DEVELOPMENT OF TESTS FRO DIAGNOSIS AND MANAGEMENT OF AISNHL**
ANTIGENZIELE DER AUTOIMMUNBEDINGTEN SCHALLEMPFINDUNGSSCHWERHÖRIGKEIT (AISNHL) UND ENTWICKLUNG VON TESTS ZUR DIAGNOSE UND BEHANDLUNG VON AISNHL
CIBLES ANTIGENIQUES DE LA SURDITE DE PERCEPTION AUTO-IMMUNE (AISNHL) ET CREATION DE TESTS DESTINES AU DIAGNOSTIC ET A LA GESTION DE AISNHL

(30) Priority: 06.05.2002 US 139496
(43) Date of publication of application: 23.02.2005
(73) Proprietor: THE REGENTS OF THE UNIVERSITY OF MICHIGAN, Ann Arbor, Michigan 48109-1280 (US)
(72) Inventor: CAREY, Thomas, E., Dexter, MI 98130 (US); NAIR, Thankam, S., Ann Arbor, MI 48105 (US); BECKMAN, Jennifer, Gray, Falls Church, VA 22042 (US)
(74) Representative: Glawe, Delfs, Moll
(86) International application number: PCT/US2003/014248
(87) International publication number: WO 2004/041152

(56) References cited:
- EP-A- 1 236 739
- WO-A-01/32704
- US-A1- 2001 006 788
- US-A1- 2003 082 646
- DATABASE EMBL [Online] 16 February 2000 (2000-02-16), "Homo sapiens CTL2 gene" XP002377229 retrieved from EBI accession no. EM_PRO:AJ245621 Database accession no. AJ245621

### SEQUENCE LISTING

<110> Carey, Thomas E.
   Nair, Thankum S.
   Gray, Jennifer P.
<120> Antigenic Targets of Autoimmune Sensorineural Hearing Loss (AISNHL) and Development of Tests for Diagnosis and Management of AISNHL
<130> UM-6982
<140> 10/139,496
   <141> 2002-05-06
<150> 09/222,179
   <151> 1998-12-29
<160> 36
<170> PatentIn version 3.1
<210> 1
   <211> 2808
   <212> DNA
   <213> Homo sapiens
<220>
   <221> misc_feature
   <222> (1553)..(1553)
   <223> The n at this position can be a, c, t, or g.
<220>
   <221> misc_feature
   <222> (2682)..(2682)
   <223> The n at this position can be a, c, t, or g.
<220>
   <221> misc_feature
   <222> (2702)..(2702)
   <223> The n at this position can be a, c, t, or g.
<220>
   <221> misc_feature
   <222> (2705)..(2705)
   <223> The n at this position can be a, c, t, or g.
<220>
   <221> misc_feature
   <222> (2743) .. (2743)
   <223> The n at this position can be a, c, t, or g.
<220>
   <221> misc_feature
   <222> (2781) .. (2781)
   <223> The n at this position can be a, c, t, or g.
<400> 1
   gcggccgccg gggctggtcg cctgcaggga tgggggacga gcggccccac tactacggga     60
   aacacggaac gccacagaag tatgatccca ctttcaaagg acccatttac aataggggct    120
   gcacggatat catatgctgt gtgttcctgc tcctggccat tgtgggctac gtggctgtag    180
   gcatcatagc ctggactcat ggagaccctc gaaaggtgat ctaccccact gatagccggg    240
   gcgagttctg cgggcagaag ggcacaaaaa acgagaacaa accctatctg ttttatttca    300
   acattgtgaa atgtgccagc cccctggttc tgctggaatt ccaatgtccc actccccaga    360
   tctgcgtgga aaaatgcccc gaccgctacc tcacgtacct gaatgctcgc agctcccggg    420
   actttgagta ctataagcag ttctgtgttc ctggcttcaa gaacaataaa ggagtggctg    480
   aggtgcttcg agatggtgac tgccctgctg tcctcatccc cagcaaaccc ttggcccgga    540
   gatgcttccc cgctatccac gcctacaagg gtgtcctgat ggtgggcaat gagacgacct    600
   atgaggatgg gcatggctcc cggaaaaaca tcacagacct ggtggagggc gccaagaaag    660
   ccaatggagt cctagaggcg cggcaactcg ccatgcgcat atttgaagat tacaccgtct    720
   cttggtactg gattatcata ggcctggtca ttgccatggc gatgagcctc ctgttcatca    780
   tcctgcttcg cttcctggct ggtattatgg tctgggtgat gatcatcatg gtgattctgg    840
   tgctgggcta cggaatattt cactgctaca tggagtactc ccgactgcgt ggtgaggccg    900
   gctctgatgt ctctttggtg gacctcggct ttcagacgga tttccgggtg tacctgcact    960
   tacggcagac ctggttggcc tttatgatca ttctgagtat ccttgaagtc attatcatct   1020
   tgctgctcat ctttctccgg aagagaattc tcatcgcgat tgcactcatc aaagaagcca   1080
   gcagggctgt gggatacgtc atgtgctcct tgctctaccc actggtcacc ttcttcttgc   1140
   tgtgcctctg catcgcctac tgggccagca ctgctgtctt cctgtccact tccaacgaag 1200
   cggtctataa gatctttgat gacagcccct gcccatttac tgcgaaaacc tgcaacccag 1260
   agaccttccc ctcctccaat gagtcccgcc aatgccccaa tgcccgttgc cagttcgcct 1320
   tctacggtgg tgagtcgggc taccaccggg ccctgctggg cctgcagatc ttcaatgcct 1380
   tcatgttctt ctggttggcc aacttcgtgc tggcgctggg ccaggtcacg ctggccgggg 1440
   cctttgcctc ctattactgg gccctgcgca agccggacga cctgccggcc ttcccgctct 1500
   tctctgcctt tggccgggcg ctcaggtacc acacaggctc cctggccttt ggngcgctca 1560
   tcctggccat tgtgcagatc atccgtgtga tactcgagta cctggatcag cggctgaaag 1620
   gtgcagagaa caagtttgcc aagtgcctca tgacctgtct caaatgctgc ttctggtgcc 1680
   tggagaagtt catcaaattc cttaatagga atgcctacat catgattgcc atctacggca 1740
   ccaatttctg cacctcggcc aggaatgcct tcttcctgct catgagaaac atcatcagag 1800
   tggctgtcct ggataaagtt actgacttcc tcttcctgtt gggcaaactt ctgatcgttg 1860
   gtagtgtggg gatcctggct ttcttcttct tcacccaccg tatcaggatc gtgcaggata 1920
   cagcaccacc cctcaattat tactgggttc ctatactgac ggtgatcgtt ggctcctact 1980
   tgattgcaca cggtttcttc agcgtctatg gcatgtgtgt ggacacgctg ttcctctgct 2040
   tcttggagga cctggagagg aatgacggct cggccgagag gccttacttc atgtcttcca 2100
   ccctcaagaa actcttgaac aagaccaaca agaaggcagc ggagtcctga aggccccgtg 2160
   ctccccacct ctcaaggagt ctcatgccgc agggtgctca gtagctgggt ctgttccccc 2220
   agccccttgg gttcacctga agtcctatca ctgccgctct gcccctcccc atgagccaga 2280
   tcccaccagt ttctggacgt ggagagtctg gggcatctcc ttcttatgcc aaggggcgct 2340
   tggagttttc atggctgccc ctccagactg cgagaaacaa gtaaaaaccc wttggggcct 2400
   cttgatgtct gggatggcac gtggcccgac ctccacaagc tccctcatgc ttcctgtccc 2460
   ccgcttacac gacaacgggc cagaccacag gaaggacggt gtttgtgtct gagggagctg 2520
   ctggccacag tgaacaccca cgtttattcc tgcctgctcc ggccaggact gaaccccttc 2580
   tccacacctg aacagttggc tcaagggcca ccagaagcat ttctttatta ttattatttt 2640
   ttaacctgga catgcattaa agggtctatt agctttcttt yncgtctgtc tcaacagctg 2700
   anatnggggc cgccaaggag tgcctttcct tttgcttcct tcntaggttg gagttaacgg 2760
   gtgggaagtt ttttttccca ngtgggggtg ttttcctggt tgggaagg 2808
<210> 2
   <211> 706
   <212> PRT
   <213> Homo sapiens
<400> 2
   Met Gly Asp Glu Arg Pro His Tyr Tyr Gly Lys His Gly Thr Pro Gln
   1               5                   10                  15
   Lys Tyr Asp Pro Thr Phe Lys Gly Pro Ile Tyr Asn Arg Gly Cys Thr
               20                  25                  30
   Asp Ile Ile Cys Cys Val Phe Leu Leu Leu Ala Ile Val Gly Tyr Val
           35                  40                  45
   Ala Val Gly Ile Ile Ala Trp Thr His Gly Asp Pro Arg Lys Val Ile
       50                  55                  60
   Tyr Pro Thr Asp Ser Arg Gly Glu Phe Cys Gly Gln Lys Gly Thr Lys
   65                  70                  75                  80
   Asn Glu Asn Lys Pro Tyr Leu Phe Tyr Phe Asn Ile Val Lys Cys Ala
                   85                  90                  95
   Ser Pro Leu Val Leu Leu Glu Phe Gln Cys Pro Thr Pro Gln Ile Cys
               100                 105                 110
   Val Glu Lys Cys Pro Asp Arg Tyr Leu Thr Tyr Leu Asn Ala Arg Ser
           115                 120                 125
   Ser Arg Asp Phe Glu Tyr Tyr Lys Gln Phe Cys Val Pro Gly Phe Lys
       130                 135                 140
   Asn Asn Lys Gly Val Ala Glu Val Leu Arg Asp Gly Asp Cys Pro Ala
   145                 150                 155                 160
   Val Leu Ile Pro Ser Lys Pro Leu Ala Arg Arg Cys Phe Pro Ala Ile
                   165                 170                 175
   His Ala Tyr Lys Gly Val Leu Met Val Gly Asn Glu Thr Thr Tyr Glu
               180                 185                 190
   Asp Gly His Gly Ser Arg Lys Asn Ile Thr Asp Leu Val Glu Gly Ala
           195                 200                 205
   Lys Lys Ala Asn Gly Val Leu Glu Ala Arg Gln Leu Ala Met Arg Ile
       210                 215                 220
   Phe Glu Asp Tyr Thr Val Ser Trp Tyr Trp Ile Ile Ile Gly Leu Val
   225                 230                 235                 240
   Ile Ala Met Ala Met Ser Leu Leu Phe Ile Ile Leu Leu Arg Phe Leu
                   245                 250                 255
   Ala Gly Ile Met Val Trp Val Met Ile Ile Met Val Ile Leu Val Leu
               260                 265                 270
   Gly Tyr Gly Ile Phe His Cys Tyr Met Glu Tyr Ser Arg Leu Arg Gly
           275                 280                 285
   Glu Ala Gly Ser Asp Val Ser Leu Val Asp Leu Gly Phe Gln Thr Asp
       290                 295                 300
   Phe Arg Val Tyr Leu His Leu Arg Gln Thr Trp Leu Ala Phe Met Ile
   305                 310                 315                 320
   Ile Leu Ser Ile Leu Glu Val Ile Ile Ile Leu Leu Leu Ile Phe Leu
                   325                 330                 335
   Arg Lys Arg Ile Leu Ile Ala Ile Ala Leu Ile Lys Glu Ala Ser Arg
               340                 345                 350
   Ala Val Gly Tyr Val Met Cys Ser Leu Leu Tyr Pro Leu Val Thr Phe
           355                 360                 365
   Phe Leu Leu Cys Leu Cys Ile Ala Tyr Trp Ala Ser Thr Ala Val Phe
       370                 375                 380
   Leu Ser Thr Ser Asn Glu Ala Val Tyr Lys Ile Phe Asp Asp Ser Pro
   385                 390                 395                 400
   Cys Pro Phe Thr Ala Lys Thr Cys Asn Pro Glu Thr Phe Pro Ser Ser
                   405                 410                 415
   Asn Glu Ser Arg Gln Cys Pro Asn Ala Arg Cys Gln Phe Ala Phe Tyr
               420                 425                 430
   Gly Gly Glu Ser Gly Tyr His Arg Ala Leu Leu Gly Leu Gln Ile Phe
           435                 440                 445
   Asn Ala Phe Met Phe Phe Trp Leu Ala Asn Phe Val Leu Ala Leu Gly
       450                 455                 460
   Gln Val Thr Leu Ala Gly Ala Phe Ala Ser Tyr Tyr Trp Ala Leu Arg
   465                 470                 475                 480
   Lys Pro Asp Asp Leu Pro Ala Phe Pro Leu Phe Ser Ala Phe Gly Arg
                   485                 490                 495
   Ala Leu Arg Tyr His Thr Gly Ser Leu Ala Phe Gly Ala Leu Ile Leu
               500                 505                 510
   Ala Ile Val Gln Ile Ile Arg Val Ile Leu Glu Tyr Leu Asp Gln Arg
           515                 520                 525
   Leu Lys Gly Ala Glu Asn Lys Phe Ala Lys Cys Leu Met Thr Cys Leu
       530                 535                 540
   Lys Cys Cys Phe Trp Cys Leu Glu Lys Phe Ile Lys Phe Leu Asn Arg
   545                 550                 555                 560
   Asn Ala Tyr Ile Met Ile Ala Ile Tyr Gly Thr Asn Phe Cys Thr Ser
                   565                 570                 575
   Ala Arg Asn Ala Phe Phe Leu Leu Met Arg Asn Ile Ile Arg Val Ala
               580                 585                 590
   Val Leu Asp Lys Val Thr Asp Phe Leu Phe Leu Leu Gly Lys Leu Leu
           595                 600                 605
   Ile Val Gly Ser Val Gly Ile Leu Ala Phe Phe Phe Phe Thr His Arg
       610                 615                 620
   Ile Arg Ile Val Gln Asp Thr Ala Pro Pro Leu Asn Tyr Tyr Trp Val
   625                 630                 635                 640
   Pro Ile Leu Thr Val Ile Val Gly Ser Tyr Leu Ile Ala His Gly Phe
                   645                 650                 655
   Phe Ser Val Tyr Gly Met Cys Val Asp Thr Leu Phe Leu Cys Phe Leu
               660                 665                 670
   Glu Asp Leu Glu Arg Asn Asp Gly Ser Ala Glu Arg Pro Tyr Phe Met
           675                 680                 685
   Ser Ser Thr Leu Lys Lys Leu Leu Asn Lys Thr Asn Lys Lys Ala Ala
       690                 695                 700
   Glu Ser
   705
<210> 3
   <211> 2090
   <212> DNA
   <213> Cavia porcellus
<400> 3
   acggaacccc gcagaagtat gaccccacct tcaagggacc catttacaac aggggctgca     60
   cagacatcct ttgctgtgtc ctcctcttcc tggccattgt gggctatgtg gctgtgggcc    120
   tcattgcctg gacgcatggg gaccctcgga aggtgatcta ccccacggac agccgaggcg    180
   aattctgcgg ccagaagggc acgaagaatg cgaacaaacc tttcctgttc tatttcaaca    240
   ttgtaaagtg tgccagcccc ctggtcctgc tggaattcca atgccccact ccccagatct    300
   gcgtggagaa gtgtcccaac cgctacctca ccctcctgaa tgcctggaac acgcctgaat    360
   ttgagtatta caagcagttc tgtgtccctg acttccagaa gaataaaaag ggtgtggccc    420
   aggtgcttcg ggatgggcag tgccctgctg tgctcatccc cagcaagccc ttggcccaga    480
   gatgcttccc ggacatccac gcccacaagg gggtcatcat ggtgggcaat gcgaccacct    540
   acgaggatgg gcacggctcc cggaaaaaca tcacggagct ggtggagggc gccaagcagg    600
   ccaacggcat cctggaggcc cggcagctgg ccatgcgcat atttgaagat tacaccgttt    660
   cctggtactg gattgtcata ggcctggtca tcgccatggt gctcagcctg ctgttcatca    720
   tcctgctgcg cttcctggcc ggcatcatgg tctgggtgat gatcgtcctg gtgatcctgg    780
   tgctgggcta cgggatattt cactgctaca tggagtacgc ccgcctgcgc ggggaggccg    840
   gctcggacat ctcggtcctg gacttgggct tccagacgga cttccgggtg tacctgcacc    900
   tccgccagac ctggctggct ttcatgatca ttctgagcat cctggaagtc atcatcatcc    960
   tgctgctcat cttcctccgc aagcggattc tcatcgccat cgcactcatc aaggaagcca   1020
   gcagggccgt gggatacgtc atgtgctcct tgctgtaccc cctggtcacc ttcttcctgc   1080
   tgtgtctctg cattgcatac tgggccagca ctgctgtctt cctgtctact tctaacgaag   1140
   ctgtctataa gattctgggc gattcctcct gcccacatca ggggcaaacg tgccaccctg   1200
   agaccttctt caattccact gaagcgcacg cgtgcccgaa cgccagatgc cagtttgcct   1260
   tctacggcgg tgaatccggc taccaccgcg cactgctggg cctgcagatc ttcaacgcct   1320
   ttatgttctt ctggctggcc aacttcgtgc tggcgctggg ccaggtcacc ctggccgggg   1380
   ccttcgcctc ctactactgg gccctgcgca agccagatga catgccggcc ttcccgctct   1440
   tcgctgcctt tggccgagcg ctcaggtacc acaccggctc tttggccttt gggtccctca   1500
   ttctcgccat tgtgcagatc atccgagtga ttctggagta tttggatcag cgcctgaaag   1560
   ctgcggaaaa caaatttgct aagtttctca tgacctgcct caagtgttgt ttctggtgcc   1620
   tggagaagtt cattaaattc ctcaacagga acgcttacat catgatcgcc atttatggca   1680
   ccaacttctg cacctcggcc agaaatgcct tcttcctgct catgagaaac atcatcaggg   1740
   tggctgtcct ggacaaagtt accgacttcc tcttcctgct gggcaaactc ctgatcgtgg   1800
   gcagcgtggg gatcctggct ttcttcttct tcacccatcg catcaggatc gtgcacgaca   1860
   cagcgccgcc tctcaactat tactgggtcc ccatactgac agtcatcgtc ggttcctacc   1920
   tgatcgccca tggtttcttt agcgtctatg gcatgtgtgt ggatacactg tttctctgct   1980
   tcttggaaga tctggagcgg aacaacggct cctccgagag gccttacttc atgtcttcca   2040
   ccctcaagaa gctcttgaat aagaccaaca agaagccggt ggagtcctaa              2090
<210> 4
   <211> 695
   <212> PRT
   <213> Cavia porcellus
<400> 4
   Gly Thr Pro Gln Lys Tyr Asp Pro Thr Phe Lys Gly Pro Ile Tyr Asn
   1               5                   10                  15
   Arg Gly Cys Thr Asp Ile Leu Cys Cys Val Leu Leu Phe Leu Ala Ile
               20                  25                  30
   Val Gly Tyr Val Ala Val Gly Leu Ile Ala Trp Thr His Gly Asp Pro
           35                  40                  45
   Arg Lys Val Ile Tyr Pro Thr Asp Ser Arg Gly Glu Phe Cys Gly Gln
       50                  55                  60
   Lys Gly Thr Lys Asn Ala Asn Lys Pro Phe Leu Phe Tyr Phe Asn Ile
   65                  70                  75                  80
   Val Lys Cys Ala Ser Pro Leu Val Leu Leu Glu Phe Gln Cys Pro Thr
                   85                  90                  95
   Pro Gln Ile Cys Val Glu Lys Cys Pro Asn Arg Tyr Leu Thr Leu Leu
               100                 105                 110
   Asn Ala Trp Asn Thr Pro Glu Phe Glu Tyr Tyr Lys Gln Phe Cys Val
           115                 120                 125
   Pro Asp Phe Gln Lys Asn Lys Lys Gly Val Ala Gln Val Leu Arg Asp
       130                 135                 140
   Gly Gln Cys Pro Ala Val Leu Ile Pro Ser Lys Pro Leu Ala Gln Arg
   145                 150                 155                 160
   Cys Phe Pro Asp Ile His Ala His Lys Gly Val Ile Met Val Gly Asn
                   165                 170                 175
   Ala Thr Thr Tyr Glu Asp Gly His Gly Ser Arg Lys Asn Ile Thr Glu
               180                 185                 190
   Leu Val Glu Gly Ala Lys Gln Ala Asn Gly Ile Leu Glu Ala Arg Gln
           195                 200                 205
   Leu Ala Met Arg Ile Phe Glu Asp Tyr Thr Val Ser Trp Tyr Trp Ile
       210                 215                 220
   Val Ile Gly Leu Val Ile Ala Met Val Leu Ser Leu Leu Phe Ile Ile
   225                 230                 235                 240
   Leu Leu Arg Phe Leu Ala Gly Ile Met Val Trp Val Met Ile Val Leu
                   245                 250                 255
   Val Ile Leu Val Leu Gly Tyr Gly Ile Phe His Cys Tyr Met Glu Tyr
               260                 265                 270
   Ala Arg Leu Arg Gly Glu Ala Gly Ser Asp Ile Ser Val Leu Asp Leu
           275                 280                 285
   Gly Phe Gln Thr Asp Phe Arg Val Tyr Leu His Leu Arg Gln Thr Trp
       290                 295                 300
   Leu Ala Phe Met Ile Ile Leu Ser Ile Leu Glu Val Ile Ile Ile Leu
   305                 310                 315                 320
   Leu Leu Ile Phe Leu Arg Lys Arg Ile Leu Ile Ala Ile Ala Leu Ile
                   325                 330                 335
   Lys Glu Ala Ser Arg Ala Val Gly Tyr Val Met Cys Ser Leu Leu Tyr
               340                 345                 350
   Pro Leu Val Thr Phe Phe Leu Leu Cys Leu Cys Ile Ala Tyr Trp Ala
           355                 360                 365
   Ser Thr Ala Val Phe Leu Ser Thr Ser Asn Glu Ala Val Tyr Lys Ile
       370                 375                 380
   Leu Gly Asp Ser Ser Cys Pro His Gln Gly Gln Thr Cys His Pro Glu
   385                 390                 395                 400
   Thr Phe Phe Asn Ser Thr Glu Ala His Ala Cys Pro Asn Ala Arg Cys
                   405                 410                 415
   Gln Phe Ala Phe Tyr Gly Gly Glu Ser Gly Tyr His Arg Ala Leu Leu
               420                 425                 430
   Gly Leu Gln Ile Phe Asn Ala Phe Met Phe Phe Trp Leu Ala Asn Phe
           435                 440                 445
   Val Leu Ala Leu Gly Gln Val Thr Leu Ala Gly Ala Phe Ala Ser Tyr
       450                 455                 460
   Tyr Trp Ala Leu Arg Lys Pro Asp Asp Met Pro Ala Phe Pro Leu Phe
   465                 470                 475                 480
   Ala Ala Phe Gly Arg Ala Leu Arg Tyr His Thr Gly Ser Leu Ala Phe
                   485                 490                 495
   Gly Ser Leu Ile Leu Ala Ile Val Gln Ile Ile Arg Val Ile Leu Glu
               500                 505                 510
   Tyr Leu Asp Gln Arg Leu Lys Ala Ala Glu Asn Lys Phe Ala Lys Phe
           515                 520                 525
   Leu Met Thr Cys Leu Lys Cys Cys Phe Trp Cys Leu Glu Lys Phe Ile
       530                 535                 540
   Lys Phe Leu Asn Arg Asn Ala Tyr Ile Met Ile Ala Ile Tyr Gly Thr
   545                 550                 555                 560
   Asn Phe Cys Thr Ser Ala Arg Asn Ala Phe Phe Leu Leu Met Arg Asn
                   565                 570                 575
   Ile Ile Arg Val Ala Val Leu Asp Lys Val Thr Asp Phe Leu Phe Leu
               580                 585                 590
   Leu Gly Lys Leu Leu Ile Val Gly Ser Val Gly Ile Leu Ala Phe Phe
           595                 600                 605
   Phe Phe Thr His Arg Ile Arg Ile Val His Asp Thr Ala Pro Pro Leu
   610 615 620
   Asn Tyr Tyr Trp Val Pro Ile Leu Thr Val Ile Val Gly Ser Tyr Leu
   625 630 635 640
   Ile Ala His Gly Phe Phe Ser Val Tyr Gly Met Cys Val Asp Thr Leu
   645 650 655
   Phe Leu Cys Phe Leu Glu Asp Leu Glu Arg Asn Asn Gly Ser Ser Glu
   660 665 670
   Arg Pro Tyr Phe Met Ser Ser Thr Leu Lys Lys Leu Leu Asn Lys Thr
   675 680 685
   Asn Lys Lys Pro Val Glu Ser
   690 695
<210> 5
   <211> 7
   <212> PRT
   <213> Artificial sequence
<220>
   <223> Synthetic
<400> 5
   Lys Tyr Asp Pro Thr Phe Lys
   1 5
<210> 6
   <211> 13
   <212> PRT
   <213> Artificial Sequence
<220>
   <223> Synthetic
<400> 6
   Lys Tyr Asp Pro Thr Phe Lys Gly Pro Ile Tyr Asn Arg
   1 5 10
<210> 7
   <211> 9
   <212> PRT
   <213> Artificial Sequence
<220>
   <223> Synthetic
<400> 7
   Lys Val Ile Tyr Pro Thr Asp Ser Arg
   1 5
<210> 8
   <211> 10
   <212> PRT
   <213> Artificial Sequence
<220>
   <223> Synthetic
<400> 8
   Arg Lys Val Ile Tyr Pro Thr Asp Ser Arg
   1 5 10
<210> 9
   <211> 22
   <212> PRT
   <213> Artificial Sequence
<220>
   <223> Synthetic
<400> 9
   Lys Cys Ala Ser Pro Leu Val Leu Leu Glu Phe Gln Cys Pro Thr Pro
   1 5 10 15
   Gln Ile Cys Val Glu Lys
   20
<210> 10
   <211> 8
   <212> PRT
   <213> Artificial Sequence
<220>
   <223> Synthetic
<400> 10
   Lys Gly Val Ala Glu Val Leu Arg
   1 5
<210> 11
   <211> 7
   <212> PRT
   <213> Artificial Sequence
<220>
   <223> Synthetic
<400> 11
   Arg Val Tyr Leu His Leu Arg
   1 5
<210> 12
   <211> 10
   <212> PRT
   <213> Artificial Sequence
<220>
   <223> Synthetic
<400> 12
   Arg Ile Leu Ile Ala Ile Ala Leu Ile Lys
   1 5 10
<210> 13
   <211> 15
   <212> PRT
   <213> Artificial Sequence
<220>
   <223> Synthetic
<400> 13
   Arg Cys Gln Phe Ala Phe Tyr Gly Gly Glu Ser Gly Tyr His Arg
   1 5 10 15
<210> 14
   <211> 9
   <212> PRT
   <213> Artificial Sequence
<220>
   <223> Synthetic
<400> 14
   Arg Asn Ala Phe Phe Leu Leu Met Arg
   1 5
<210> 15
   <211> 11
   <212> PRT
   <213> Artificial Sequence
<220>
   <223> Synthetic
<400> 15
   Lys Val Thr Asp Phe Leu Phe Leu Leu Gly Lys
   1 5 10
<210> 16
   <211> 17
   <212> PRT
   <213> Artificial Sequence
<220>
   <223> Synthetic
<400> 16
   Arg Asn Asp Gly Ser Ala Glu Arg Pro Tyr Phe Met Ser Ser Thr Leu
   1 5 10 15
   Lys
<210> 17
   <211> 18
   <212> PRT
   <213> Artificial Sequence
<220>
   <223> Synthetic
<400> 17
   His Gly Thr Pro Gln Lys Tyr Asp Pro Thr Phe Lys Gly Pro Ile Tyr
   1 5 10 15
   Asn Arg
<210> 18
   <211> 20
   <212> DNA
   <213> Artificial Sequence
<220>
   <223> Synthetic
<400> 18
   tgtgggatac gtcatgtgct 20
<210> 19
   <211> 20
   <212> DNA
   <213> Artificial Sequence
<220>
   <223> Synthetic
<400> 19
   gcagcatttg agacaggtca 20
<210> 20
   <211> 23
   <212> DNA
   <213> Artificial Sequence
<220>
   <223> Synthetic
<400> 20
   ccactttcaa aggacccatt tac 23
<210> 21
   <211> 19
   <212> DNA
   <213> Artificial Sequence
<220>
   <223> Synthetic
<400> 21
   cgaagcacct cagccactc 19
<210> 22
   <211> 20
   <212> DNA
   <213> Artificial Sequence
<220>
   <223> Synthetic
<400> 22
   ctcatcgcga ttgcactcat 20
<210> 23
   <211> 20
   <212> DNA
   <213> Artificial Sequence
<220>
   <223> Synthetic
<400> 23
   ccgactcacc accgtagaag 20
<210> 24
   <211> 20
   <212> DNA
   <213> Artificial Sequence
<220>
   <223> Synthetic
<400> 24
   gccttctacg gtggtgagtc 20
<210> 25
   <211> 20
   <212> DNA
   <213> Artificial Sequence
<220>
   <223> Synthetic
<400> 25
   cccaacagga agaggaagtc 20
<210> 26
   <211> 19
   <212> DNA
   <213> Artificial Sequence
<220>
   <223> Synthetic
<400> 26
   atgccttctt cctgcgcat 19
<210> 27
   <211> 21
   <212> DNA
   <213> Artificial Sequence
<220>
   <223> Synthetic
<400> 27
   tttcttgagg gtggaagaca t 21
<210> 28
   <211> 20
   <212> DNA
   <213> Artificial Sequence
<220>
   <223> Synthetic
<400> 28
   gggctggcac actttacaat 20
<210> 29
   <211> 20
   <212> DNA
   <213> Artificial Sequence
<220>
   <223> Synthetic
<400> 29
   gggattctgg ctttcttctt 20
<210> 30
   <211> 23
   <212> DNA
   <213> Artificial Sequence
<220>
   <223> Synthetic
<400> 30
   ctacctcacg tacctgaatg ctc 23
<210> 31
   <211> 27
   <212> DNA
   <213> Artificial Sequence
<220>
   <223> Synthetic
<400> 31
   aatcatgatg taggcattcc tattagg 27
<210> 32
   <211> 23
   <212> DNA
   <213> Artificial Sequence
<220>
   <223> Synthetic
<400> 32
   aggtgatcta ccccactgat agc 23
<210> 33
   <211> 23
   <212> DNA
   <213> Artificial Sequence
<220>
   <223> Synthetic
<400> 33
   gggacccagt aatagttgag agg 23
<210> 34
   <211> 20
   <212> DNA
   <213> Artificial Sequence
<220>
   <223> Synthetic
<400> 34
   tcatcagagt ggctgtcctg 20
<210> 35
   <211> 20
   <212> DNA
   <213> Artificial Sequence
<220>
   <223> Synthetic
<400> 35
   tttcgagggt ctccatgagt 20
<210> 36
   <211> 52
   <212> PRT
   <213> Artificial Sequence
<220>
   <223> Synthetic
<400> 36
   Arg Asn Asp Gly Ser Ala Glu Arg Pro Tyr Phe Met Ser Ser Thr Leu
   1               5                   10                  15
   Lys His Gly Thr Pro Gln Lys Tyr Asp Pro Thr Phe Lys Gly Pro Ile
               20                  25                  30
   Tyr Asn Arg Thr Cys Asn Pro Glu Thr Phe Pro Ser Ser Asn Glu Ser
           35                  40                  45
   Arg Gln Cys Pro
       50

## Description

This invention was made in part with government support under grant DC 002272 from the National Institutes of Health. The government has certain rights in the invention.

This application is a continuation-in-part of US Patent Application Serial No. 09/222,179, filed on December 29, 1998.

### FIELD OF THE INVENTION

This invention is defined in the claims and relates generally to antigens reactive with autoantibodies associated with autoimmune sensorineural hearing loss (AISNHL), and in particular relates to a purified novel antigen, Inner Ear Supporting Cell Antigen (IESCA) reactive with autoantibodies associated with autoimmune sensorineural hearing loss (AISNHL), and to methods for predicting the response of AISNHL patients to therapeutic treatments, and to methods of detecting genetic lesions in genes encoding such antigens.

### BACKGROUND

**Sensorineural Hearing Loss.** Sensorineural hearing loss (SNHL) is a common disorder affecting millions of Americans. SNHL is the result of damage to either the sensory system within the inner ear or the nerves that carry information from the sensory system to the brain. The inner ear is a tiny organ comprised of specialized sensory cells. These specialized cells are called hair cells because they have stereocilia or long stiff projections from their upper surface. The hair cells are arranged in the inner ear or cochlea within the organ of Corti, in order from the high frequency region in the base of the cochlea to the low frequency region in the apex. Sounds are transmitted from the eardrum to the inner ear by small bones called ossicles. These bones vibrate against a membrane in the cochlea transmitting the energy to the fluid inside. The fluid moves the organ of Corti stimulating hair cells at the appropriate frequency. The stimulated hair cells then stimulate nerves that send the information to the brain for processing. If hair cells are damaged or lost, then hearing is affected for the frequencies encoded by those sensory cells.

**Autoimmune Hearing Loss In Humans.** Unexplained or idiopathic SNHL is troubling to physicians and patients alike because the etiology is unknown and there are few effective treatments. Autoimmunity is suspected to be a cause of some cases of sudden onset, rapidly progressive or fluctuating hearing loss, particularly when bilateral involvement occurs. Autoimmune sensorineural hearing loss (AISNHL) in humans has been suspected in systemic autoimmune diseases, and there are indications that inner ear organ-specific autoimmunity is involved in rapidly progressive hearing loss (Harris JP (1993In: 2nd edition, Otolaryngology Head and Neck Surgery, Vol IV: Ear and Cranial Base (Cummings CW, Krause CJ, Schuller DE, Fredrickson JM, Harker LA (eds), Mosby Yearbook, St. Louis, MO) pp. 2926-2942; McCabe BF (1979) Ann. Otol .88:585-859; McCabe BF (1991) In: Bearing Of Basic Research On Clinical Otolaryngology, Adv. Otorhinolaryngol (Pfaltz CR, Arnold W, Kleinsasser O (eds), Karger Publishing, Basel, Switzerland, Vol. 46) pp. 78-81; Hughes et al. (1988) Laryngoscope 98:251-253; Harris and Ryan (1984) Am. J. Otolaryngol. 5:418-425; Cruz et al. (1990) Am. J. Otol. 11:342-346; Harris (1983) Otolaryngol Head Neck Surg. 91:17; Arnold et al. (1985) Acta Otolaryngol 99:437; Harris and Sharp (1990) Laryngoscope 100:516-524; Moscicki et al. (1994) JAMA 272:611-616; Sismanis et al. (1997) Otolaryngol Head Neck Surg. 116:146-152).

Given the high frequency of idiopathic SNHL, the ability to accurately diagnose autoimmune SNHL (AISNHL) would be of value since this is one of the few potentially treatable causes of SNHL. However, since treatment of autoimmune disease involves toxic drugs such as corticosteroids, cyclophosphamide, methotrexate and cyclosporin A, all of which have significant side effects, most physicians are reluctant to use these agents without a clear indication (Sismanis et al. (1997) Otolaryngol Head Neck Surg. 116:146-152). Treatment of suspected autoimmune hearing loss is complicated, because without treatment there is a high frequency of spontaneous remission, but in cases that don't regress, the hearing loss may become worse and permanent. Immune-mediated hearing loss also may include 30-50% of Meniere's disease (episodic vertigo and fluctuating progressive hearing loss) patients (Rauch et al. (1995) Am. J. Otology 16:648-652; Shin et al. (1997) Laryngoscope 107:222-227).

Early approaches to detecting AISNHL employed cellular assays of immune reactivity, such as lymphocyte transformation and lymphocyte migration inhibition assays using crude inner ear antigens (McCabe BF (1991) In: Bearing Of Basic Research On Clinical Otolaryngology, Adv. Otorhinolaryngol (Pfaltz CR, Arnold W, Kleinsasser O (eds), Karger Publishing, Basel, Switzerland) Vol. 46, pp. 78-81; Hughes et al. (1984) Laryngoscope 94:758-767). Unfortunately, these assays did not correlate well with response to therapy (Hughes et al. (1984) Laryngoscope 94:758-767; Kanzaki J and O-Uchi T (1983) Acta. Otolaryngol. (suppl.), 393:77-84; Hughes et al. (1988) Laryngoscope 98:251-253; Veldman et al. (1984) Laryngoscope 94:501-507). The poor predictability has been explained by a lack of sensitivity and specificity of these assays for identifying organ-specific autoimmune reactivity (Mattox and Lyles (1989) Am. J. Otol. 10:242-247; Mattox and Simmons (1977) Ann. Otol. Rhinol. Laryngol. 86:463-480).

Currently, there is general consensus among researchers in the field that the target antigen of the hearing loss antibody is a 68 kDa protein found in the inner ear. It has been postulated that the 68 kDa protein is HSP-70 (Billings et al. (1995) Ann. Otol. Rhinol. Laryngol. 104:181-188). Dr. Harris patented the use of a Western blot for HSP-70 as a diagnostic tool for AISNHL (U.S. Patent No. 5,422,282) and worked with OtoImmune (IMMCO Diagnostics) to bring a product to market based on his research. However, the correlation between the Western blot result and patient to steroid treatment is low, both as observed by the inventors (53%), and as reported in the literature (Moscicki et al. (1994) JAMA 272:611-616).

Therefore, what is needed are more accurate tests to detect immune-mediated hearing loss. It would also be useful to select patients who have a greater likelihood of responding to steroid treatment.

### SUMMARY OF THE INVENTION

This invention is defined in the claims and generally relates to a purified novel antigen, Inner Ear Supporting Cell Antigen (IESCA) reactive with an autoantibody associated with autoimmune sensorineural hearing loss (AISNHL), and to methods for predicting the response of AISNHL patients to therapeutic treatment. Additionally, the present invention provides a kit containing reagents to assay for an antibody associated with AISNHL in a patient. Furthermore, the present invention relates to methods for drug screens for compounds that are agonistic or antagonistic to IESCA binding and/or function, as well as agonistic or antagonistic to anti-IESCA antibody binding to IESCA. The present invention also relates to methods for screens for IESCA homologs. The present invention also relates to methods of detecting other proteins involved in hearing function and/or loss, and in particular in AISNHL. The present invention also relates to methods of detecting genetic lesions in genes encoding IESCA, IESCA homologs, and other proteins involved in hearing function and/or loss, and in particular in AISNHL.

The present invention represents a major improvement over existing tests for AISNHL, which identify antibodies to a universally distributed substance that is not unique to the inner ear and has never been linked to hearing loss. This substance has been identified as Heat Shock Protein 70 (HSP-70).

Therefore, the present invention provides an immunopurified glycoprotein from the inner-ear organ of Corti reactive with an antibody associated with sensorineural hearing loss, where the glycoprotein is a doublet with apparent molecular weights of about 68,000 and 72,000 daltons as determined by sodium dodecyl sulfate polyacrylamide gel electrophoresis under reducing conditions, and a doublet with apparent molecular weights of about 65,000 and about 68,000 daltons as determined by sodium dodecyl sulfate polyacrylamide gel electrophoresis under non-reducing conditions. In some embodiments, the glycoprotein is reactive with a human autoimmune antibody. In other embodiments, the glycoprotein is reactive with a KHRI-3 monoclonal antibody.

The glycoprotein is encoded by a nucleic acid sequence encoding CTL2; in particular the glycoprotein is encoded by a nucleic acid sequence of SEQ ID NO:1 or an amino acid sequence of SEQ ID NO:2.

The present invention relates to a composition comprising any of the glycoproteins described above.

The present invention also provides a method for predicting the response of an AISNHL patient to therapeutic treatment with steroids, comprising providing the glycoprotein as described above and serum from a patient suspected of having AISNHL, contacting the glycoprotein with the serum, and detecting binding of an antibody in the serum to the glycoprotein under conditions sufficient to effect binding of the antibody to the glycoprotein, where the response of an AISNHL patient to therapeutic treatment with steroids is correlated to the presence of an antibody in the patient serum which binds to the glycoprotein.

The present invention also provides a kit for assaying for the presence of an antibody associated with AISNHL in a patient, where the kit comprises the glycoprotein described above.

The present invention also relates to a method for screening for a compound that binds to IESCA, comprising providing any of the glycoproteins described above, contacting the glycoprotein with the compound under conditions sufficient to effect binding of the compound to the glycoprotein, and detecting binding to the glycoprotein.

The present invention also relates to a method for screening for a compound that alters anti-IESCA antibody binding to IESCA, comprising providing any of the glycoproteins described above and an antibody to the glycoprotein, combining the glycoprotein, the antibody and the compound in any order to form a mixture, under conditions sufficient to effect binding of the antibody to the glycoprotein, and detecting binding of the antibody to the glycoprotein, under conditions sufficient to detect an alteration in anti-IESCA antibody binding to IESCA. For example, the glycoprotein is combined first with the compound, and then with the antibody or the antibody is mixed with the compound, and then with the glycoprotein. The antibody can be an autoimmune antibody; e.g. a human autoimmune antibody.

The present invention also relates to a method for screening for an IESCA homolog, comprising providing an extract from a cell suspected of containing the homolog, and an antibody reactive to IESCA and specific for at least a portion of the protein, and mixing the antibody with the extract under conditions such that the homolog is detected.

The present invention also relates to a method for detecting a protein involved in hearing function and/or damage and/or loss, comprising providing an extract from a cell suspected of containing the protein, and an antibody associated with AISNHL and reactive to IESCA, and in a form such that an antibody-antigen complex can be precipitated, mixing the antibody with the extract under conditions such that an immunoprecipitate is formed, separating proteins of the immunoprecipitate, and detecting a protein involved in hearing function and/or loss. The method can detect a protein involved in AISNHL.

The present invention also relates to a method for detecting an allele associated with hearing damage or loss, comprising providing a sample of DNA from a patient, and assaying the DNA sample for the presence of an allele encoding any of the glycoproteins described above and which is associated with hearing damage or loss.

### DESCRIPTION OF THE FIGURES

Figure 1 shows a graphical representation of protein G purification of KHRI-3 monoclonal antibody.
Figure 2 shows an SDS-PAGE gel of purification of IESCA using a KHRI-3 antibody affinity column.
Figure 3 shows a non-reducing and reducing PAGE of purified IESCA.
Figure 4 shows an SDS-PAGE separation of affinity purified proteins purified with KHRI-3, carried out as described in the methods. The gels were stained with western blotting and Coomassie blue staining; the IESCA 68 and 72 kDa bands can be identified from these stained gels. Affinity-purified proteins were subjected to SDS-PAGE, and the 68 and 72 kDa bands were identified by Western blot and colloidal Coomassie blue staining
Figure 5 (SEQ ID NOS: 6, 7, 9, 10, 11, 12, 13, 14, 15, and 36) shows a diagrammatic representation of the predicted structure of the CTL2 protein in the cell membrane. This structure was predicted using Swiss Prot Expert Protein Analysis System (ExPASy) (at the ca.expaxy.org/sprot web site) based on the various domains within the protein and the conformation with the lowest predicted entropy.
Figure 6 shows immunofluorescence staining of guinea pig inner ear surface preparations stained with either KHRI-3 antibody (left panel) or anti-CTL2 sera (central panel) or preimmune serum as a control (right panel).

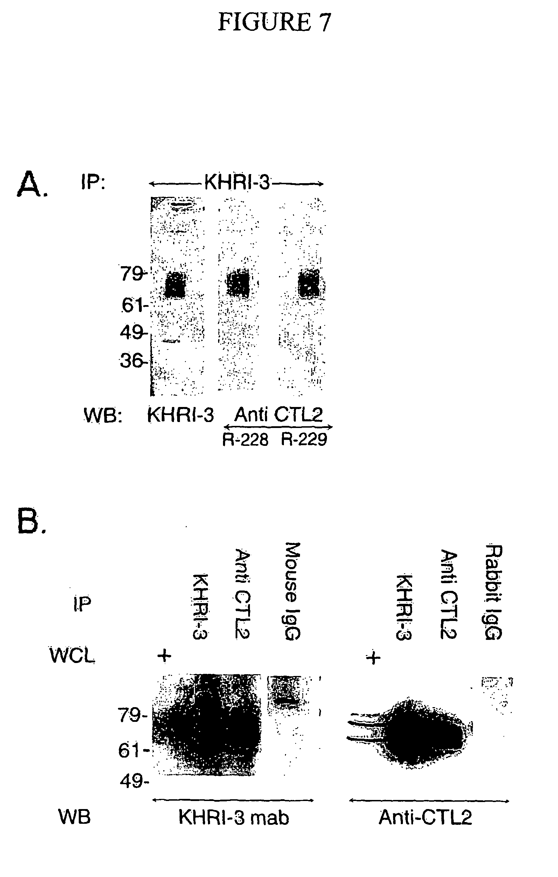

Figure 7A shows the results of proteins immunoprecipitated (IP) from guinea pig inner ear extracts with CNBr coupled KHRI-3 monoclonal antibody, subjected to SDS PAGE under non-reducing conditions, transferred to nitrocellulose filters, and western blotted with either KHRI-3 antibody or with the anti-CTL2 sera from rabbits R228 and R229. Figure 7B shows the results of a second immunoprecipitation (IP) of guinea pig inner ear extracts carried out using anti-CTL2 serum (R229) coupled to CNBr beads, KHRI-3 antibody coupled to CNBr beads, and rabbit and mouse IgG coupled to CNBr beads as a controls. The immunoprecipitates were electrophoresed along with whole cochlear lysates (WCL) and then the blots were probed with either KHRI-3 antibody (left panel) or with the R229 anti-CTL2 antiserum (right panel).
Figure 8 shows primers used to detect CTL2 messenger RNA expression from four human tumor cell lines, human keratinocytes, and inner ear from guinea pig and humans; total RNA was screened.
Figure 9 shows the nucleic acid sequence encoding human CTL2 (hCTL2, SEQ ID NO:1). This sequence was obtained from Pub Med, Accession No. AJ245621, as submitted by Submitted (03-AUG-1999) O'Regan S., Laboratoire de Neurobiologie Cellulaire et Moleculaire, C.N.R.S., Av. de la Terrasse, F-91198 Gif sur Yvette, France, and reported by O'Regan, S. et al. (2000) Proc. Natl. Acad. Sci. U.S.A. 97 (4), 1835-1840; the sequence was obtained from Ewing's sarcoma cell line.
Figure 10 shows the amino acid of human CTL2 (hCTL2, SEQ ID NO:2). This sequence was obtained from Pub Med, Accession No. AJ245621, as submitted by Submitted (03-AUG-1999) O'Regan S., Laboratoire de Neurobiologie Cellulaire et Moleculaire, C.N.R.S., Av. de la Terrasse, F-91198 Gif sur Yvette, France, and reported by O'Regan, S. et al. (2000) Proc. Natl. Acad. Sci. U.S.A. 97 (4), 1835-1840; the sequence was obtained from Ewing's sarcoma cell line. Polynucleotides defining human CTL2 are also known from WO-A-01/32704.
Figure 11 shows the nucleic acid sequence encoding guinea pig CTL2 (gpCTL2, SEQ ID NO:3). This sequence was discovered by the inventors, as described in Example 5.
Figure 12 shows the amino acid sequence of guinea pig CTL2 (gpCTL2, SEQ ID NO:4). This sequence is predicted from the SEQ ID NO:3; it also contains peptide fragments which were sequenced from IESCA, as described in Example 5.

### GENERAL DESCRIPTION OF THE INVENTION

The present invention is defined in the claims and generally relates to a purified novel antigen, Inner Ear Supporting Cell Antigen (IESCA) reactive with an autoantibody associated with autoimmune sensorineural hearing loss (AISNHL), and to methods for predicting the response of AISNHL patients to therapeutic treatment. Additionally, the present invention provides a kit containing reagents to assay for an antibody associated with AISNHL in a patient. Furthermore, the present invention relates to methods for drug screens for compounds that are agonistic or antagonistic to IESCA binding and/or function, as well as agonistic or antagonistic to anti-IESCA antibody binding to IESCA. The present invention also relates to methods for screens for IESCA homologs. The present invention also relates to methods of detecting other proteins involved in hearing function and/or loss, and in particular in AISNHL; at least one of these other proteins, cochlin, appears to form a complex with IESCA. The present invention also relates to methods of detecting genetic lesions in genes encoding IESCA, IESCA homologs, and other proteins involved in hearing function and/or loss.

The present invention represents a major improvement over existing tests for AISNHL, which identify antibodies to a universally distributed substance that is not unique to the inner ear and has never been linked to hearing loss. This substance has been identified as Heat Shock Protein 70 (HSP-70).

The present invention provides a novel, substantially purified antigen from the organ of Corti reactive with autoantibodies and monoclonal antibodies associated with or relating to autoimmune sensorineural hearing loss (ASNHL). The antigen is named inner-ear supporting cells antigen (IESCA). Preferably, IESCA is immunopurified. Purified IESCA is a doublet, with apparent molecular weights of about 68,000 and 70,000 (older molecular weight size standards) or 72,000 (newer molecular weight size standards) daltons as determined by SDS-PAGE analysis under reducing conditions, and 65,000-68,000 daltons under non-reducing conditions, and has reactivity to auto-antibodies associated with AISNHL. IESCA has been isolated and purified with anti-IESCA monoclonal antibody (MAb) KHRI-3 both by immunoprecipitation and antibody affinity chromatography. The invention also provides purified epitopes of IESCA having reactivity with the autoantibodies associated with AISNHL.

The inventors have demonstrated that IESCA is different from another 68 kD antigen previously identified as associated with AISNHL (Harris and Sharp (1990) Laryngoscope 100:516-524). That antigen was later identified to be heat shock protein-70 (HSP-70) (Billings et al. (1995) Laryngoscope 105:1347-1352; Shin et al. (1997) Laryngoscope 107:222-227). The inventors have conducted, for example, immunoprecipitation experiments that clearly show that the antibodies to either IESCA or HSP-70 will not cross react to antigens immunoprecipitated by the other antibody (as described in the Examples).

Furthermore, the inventors have demonstrated that the IESCA. protein which has been identified in example X of US 2001 006 788 A1 is different from the glycoprotein as claimed in the claims.

Moreover, the inventors have characterized, isolated and purified and identified IESCA as a CTL2 protein. Therefore, the present invention also provides compositions comprising nucleic acid sequences encoding CTL2 and/or amino acid sequences of CTL2 as defined in the claims.

These sequences can be used for the production of IESCA and portions of IESCA, as well as antibodies generated to the protein sequences (and portions thereof), and expression constructs and transgenic host cells generated from the nucleotide sequences (and portions thereof). As described further below, it appears that IESCA is a mature form of CTL2. This is based upon the observation of a discrepancy between the predicted size of CTL2 (80 kDa) and the actual size of the protein precipitated by anti-IESCA antibodies (a doublet of molecular weights 68 and 72 kDa under reducing conditions). Moreover, IESCA is a glycosylated protein. Based upon these and additional observations, as described in more detail below, it is contemplated that CTL2 is initially synthesized as a nascent 80 kDa protein, which is post-translationally modified to a glycoprotein with the observed size of IESCA.

The invention also relates to novel compositions. A first composition comprises isolated guinea pig (or other suitable animal) organ of Corti (or other suitable tissue that contains IESCA) and human sera from individuals suspected of having AISNHL. A second composition comprises extracts from guinea pig (or other suitable animal) organ of Corti (or other suitable tissue that contains IESCA), KHRI-3 (a monoclonal antibody reactive with IESCA) or another antibody capable (able to) recognize IESCA, and sera from individuals suspected of having AISNHL.

Furthermore, the present invention also relates to methods using the above-named antigen and compositions in screening assays for AISNHL. The present invention relates to a variety of methods of testing patients suspected of having AISNHL, and of those patients who will most likely respond to steroid treatment. For example guinea pig (or other suitable animal) organ of Corti (or other suitable tissue) is exposed to a patient's sera followed by immunofluorescent staining by methods known to those in the field. A positive result comprises a distinctive staining pattern of "wine glass" shapes on the organ of Corti (or other distinguishable pattern depending on the tissue used). Staining with MAb KHRI-3, or other suitable antibody, serves as a positive control. The method is not limited by the tissue used or the animal from which it was derived, so long as when the tissue is stained with antibody that recognizes IESCA, the staining is distinctive. The invention is not limited by the antibody used as a positive control, so long as the antibody is specific for IESCA or an epitope of IESCA.

Another method contemplates providing purified IESCA from source tissue and determining if sera from a patient suspected of having AISNHL will react with the antigen. The present invention is not limited by the particular method of screening, or by the particular source of IESCA. One embodiment comprises providing purified IESCA, and Western blotting precipitated IESCA with the patient's sera. Another embodiment comprises making tissue extracts from guinea pig organ of Corti, followed by immunoprecipitation of IESCA with monoclonal antibody KHRI-3, or other suitable antibody, and Western blotting precipitated IESCA with patients' sera. A positive result in either embodiment comprises recognition of the precipitated IESCA with the patient's sera. Staining with MAb KHRI-3, or other suitable antibody, may serve as a positive control. The invention is not limited by the tissue used to acquire IESCA, or the animal from which it was derived, so long as the isolated protein stains with antibody that recognizes IESCA. IESCA can be naturally occurring, or obtained from transgenic cells or organisms. The invention is not limited by the antibody used to precipitate the IESCA, or used as a positive control, so long as the antibody is specific for IESCA or an epitope of IESCA (which can be confirmed in competition assays or preclearing assays with MAb KHRI-3).

The invention also relates to methods for drug screens for compounds that are agonistic or antagonistic for binding to IESCA and anti-IESCA antibodies (referred to as "anti-IESCA"). For example, compounds suspected of binding IESCA are mixed with IESCA, under conditions sufficient for the binding of the compound to IESCA, followed by washing, and then the addition of anti-IESCA. Binding of anti-IESCA to IESCA is detected by using assays known to those practiced in the art. Increases or deceases in binding as compared to controls are used to determine if the suspected compound is agonistic or antagonistic to the binding of IESCA and anti-IESCA.

In other methods, a compound suspected of binding anti-IESCA, but not IESCA, is mixed with anti-IESCA, under conditions sufficient for the binding of the compound to IESCA, followed by washing, and then the addition of IESCA. The binding of IESCA to anti-IESCA is detected by assays known to those practiced in the art. Increases or decreases in binding as compared to controls is used to determine if the suspected compound is agonistic or antagonistic to the binding of IESCA to anti-IESCA. Both screening assays described above are adaptable to high throughput assay formats, for example by utilizing microtiter plates and automatic plate readers.

The invention also relates to methods for screening for intra- and inter- specific homologs of IESCA. For example, the method comprises providing in any order an extract from cells or tissues suspected of containing the homolog and antibodies reactive to IESCA and specific for at least a portion of the peptide or glycoconjugate IESCA, and mixing the antibody with the extract under conditions such that the homolog is detected.

The present invention also relates to methods for detecting a protein involved in hearing function and/or loss, and in particular in AISNHL, comprising providing an extract from cells or tissues suspected of containing the protein, and an antibody associated with AISNHL and reactive to IESCA and specific for at least a portion of the peptide or glycoconjugate IESCA, and in a form such that an antibody-antigen complex can be precipitated, mixing the antibody with the extract under conditions such that an immunoprecipitate is formed, separating proteins of the immunoprecipitated, and detecting a protein involved in hearing function and/or loss.

The present invention also relates to methods of detecting a genetic lesion associated with hearing damage or loss, comprising providing a sample of DNA from a patient, and assaying the DNA sample for the presence of a lesion in a gene encoding for the IESCA.

### DEFINITIONS

To facilitate an understanding of the present invention, a number of terms and phrases as used herein are defined below:
The term "sensorineural hearing loss (SNHL)" refers to a disease characterized by progressive unilateral or bilateral deafness resulting from damage to sensory cells or nerves or other components of the inner ear necessary for processing sounds, that, in its incipient stages, may fluctuate or become sudden and profound.

The term "autoimmune sensorineural hearing loss (AISNHL)" refers to sensorineural hearing loss whose etiology includes the generation of autoantibodies directed against inner ear antigenic epitopes.

The term "autoimmune (autoimmunity)" refers to a condition where antibodies recognize self-antigens as foreign and thereby initiate an immune response to cells, tissues or organs often causing the establishment and continuation of a disease state.

The terms "auto-antibody" or "autoimmune antibody" refer to an antibody which recognizes a self-antigen as foreign.

The terms "antibody" or "immunoglobulin" refer to proteins that bind a specific antigen. Immunoglobulins include, but are not limited to, polyclonal, monoclonal, chimeric, and humanized antibodies, Fab Fragments, F(ab')₂ fragments, and includes immunoglobulins of the following classes: IgG, IgA, IgM, IgD, IbE, and secreted immunoglobulins (sIg). Immunoglobulins generally comprise two identical heavy chains and two light chains. However, the terms "antibody" and "immunoglobulin" also encompass single chain antibodies and two chain antibodies. Antibodies may be produced by any of the known methodologies [Current Protocols in Immunology (1998) John Wiley and Sons, Inc., N.Y.].

The term "epitope" refers to tht portion of an antigen that makes contact with a particular antibody or immunoglobulin.

The term "anti-IESCA antibody" refers to an antibody that specifically binds to IESCA.

The term "KHRI-3" refers to a mouse hybridoma monoclonal antibody which is specific to IESCA.

The term "antigen" refers to a protein, glycoprotein, lipoprotein, lipid or other substance that is reactive with an antibody specific for a portion of the molecule.

The term "inner ear antigens" refers to antigens from the inner ear that are reactive with autoantibodies associated with AISNHL.

The term "inner-Ear Supporting Cells Antigen (IESCA)" refers to molecules (proteins, glycoproteins, lipoproteins or other molecules reactive with antibodies), or portions thereof, that are localized with high specificity to the supporting cells of the inner ear and are reactive with KHRI-3 MAb. In the context of this patent application the following shall generally apply: Expression of these molecules in other tissues does not exclude them from being IESCA. Likewise, absence of these molecules from the inner ear supporting cells in certain circumstances (such as disease) does not exclude them from being IESCA. Furthermore, variation in molecular weight of IESCA from that disclosed here, so long as they are reactive with KHRI-3 MAb, does not exclude them from being IESCA.

The term "inner ear organ of Corti" refers to the structure within which specialized sensory cells, or hair cells, are arranged in the inner ear or cochlea; these cells are arranged in order from the high frequency region in the base of the cochlea to the low frequency in the apex. The hair cells are so named because they have stereocilia or long stiff projections from their upper surface. Sounds are transmitted from the eardrum to the inner ear by small bones called ossicles. These bones vibrate against a membrane in the cochlea transmitting the energy to the fluid inside. The fluid moves the organ of Corti stimulating hair cells at the appropriate frequency. The stimulated hair cells then stimulate nerves that send the information to the brain for processing. If hair cells are damaged or lost, then hearing is affected for the frequencies encoded by those sensory cells.

The term "glycoprotein" refers to a protein which has been post-translationally modified by glycosylation, or the addition of carbohydrate moieties to the protein.

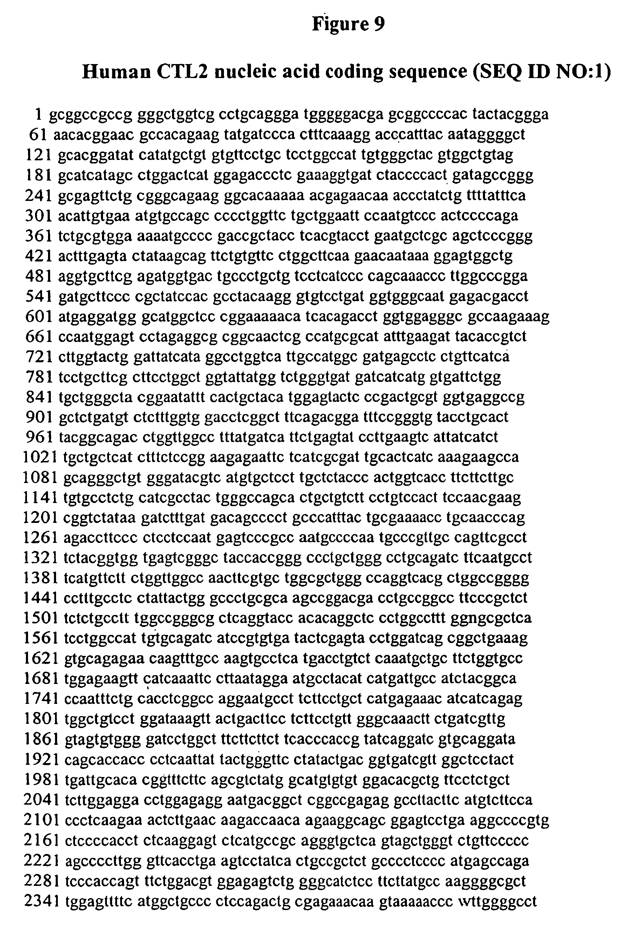

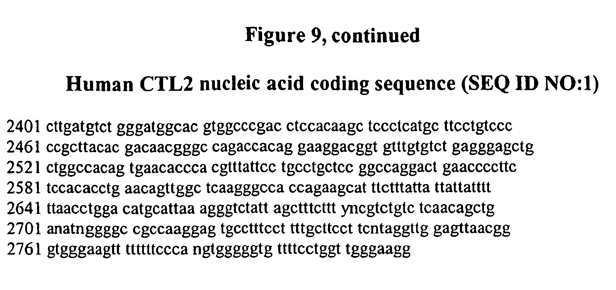

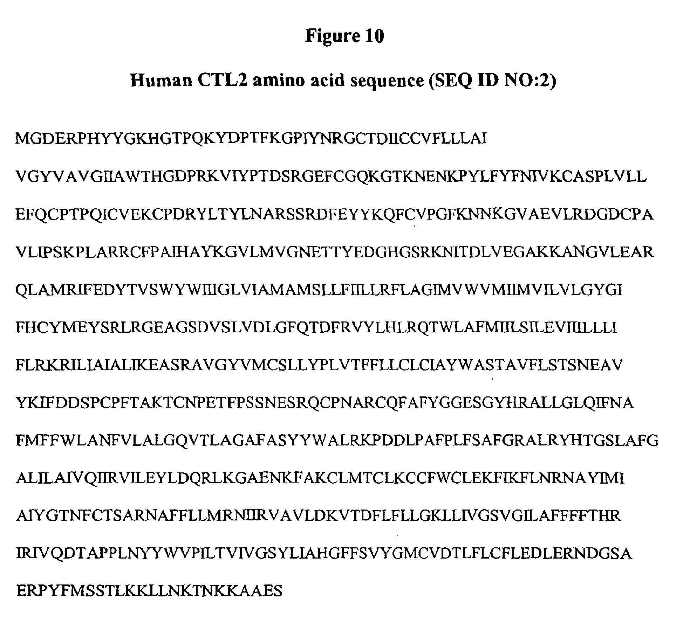

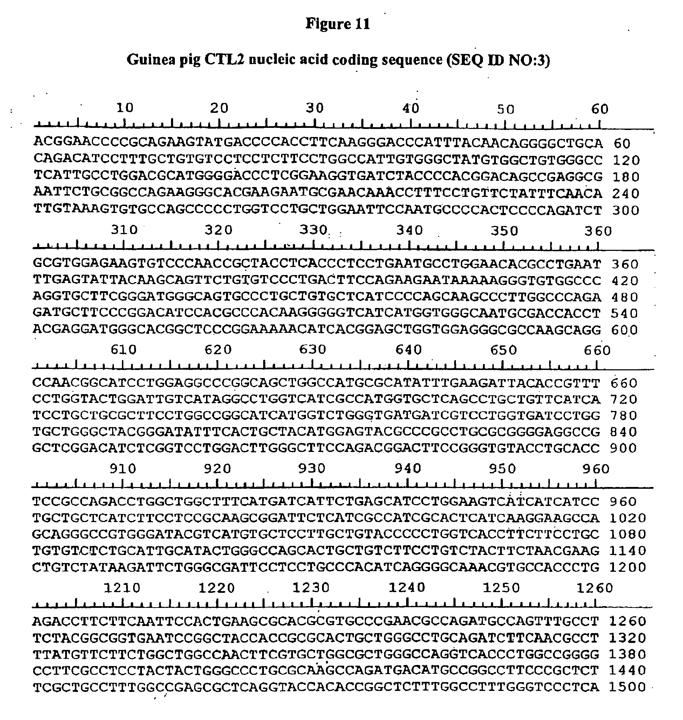

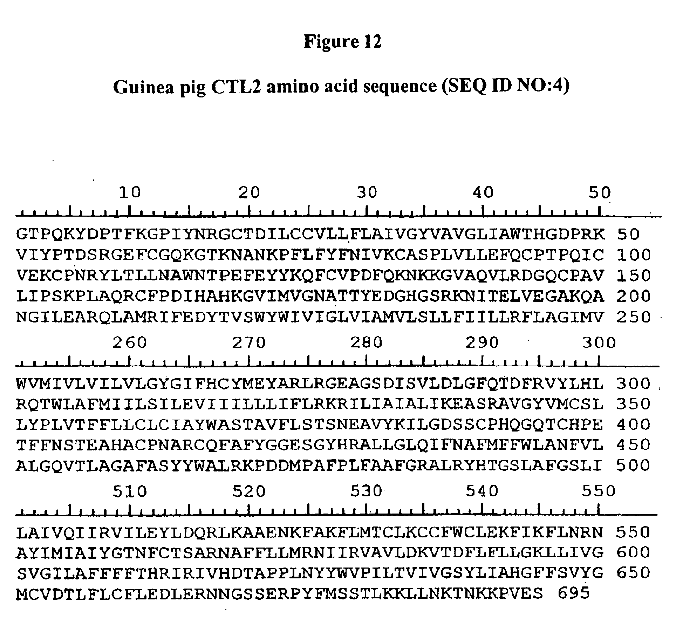

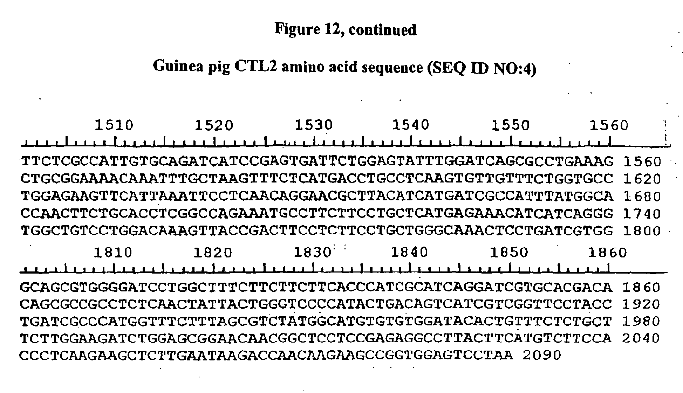

The term "CTL2" refers to a choline-transport-like protein 2, as for example identified by O'Regan (O'Regan,S. et al. (2000) Proc. Natl. Acad. Sci. U.S.A. 97 (4), 1835-1840). An exemplary amino sequence of human CTL2 is SEQ ID NO:2 as shown in Figure 10, and an exemplary nucleic acid sequence encoding CTL2 is SEQ ID NO: 1 as shown in Figure 9. An exemplary amino sequence of guinea pig CTL2 is SEQ ID NO:4 as shown in Figure 12, and an exemplary nucleic acid sequence encoding guinea pig CTL2 is SEQ ID NO:3 as shown in Figure 11, both as discovered by the inventors.

The terms "immunoprecipitate," "immunopurify," and "affinity purify," and grammatical variations such as verbs and adjectives, refer to the use of an antibody to separate its antigen or a portion thereof from a mixture of other molecules.

The term "staining" refers to as any number of processes known to those in the field that are used to better visualize, distinguish or identify a specific component(s) and/or feature(s) of a cell or cells.

The term "immunofluorescence" refers to a staining technique used to identify, mark, label, visualize or make readily apparent by procedures known to those practiced in the art, where a ligand (usually an antibody) is bound to a receptor (usually an antigen) and such ligand, if an antibody, is conjugated to a fluorescent molecule, or the ligand is then bound by an antibody specific for the ligand, and said antibody is conjugated to a fluorescent molecule, where said fluorescent molecule can be visualized with the appropriate instrument (*e.g*., a fluorescent microscope).

The term "morphology" refers to the visual appearance of a cell or organism when viewed, for example, with the eye, a light microscope, a confocal microscope or an electronmicroscope, as appropriate.

The term "patient" refers to a human or other animal, such as a guinea pig or mouse and the like, capable of having AISNHL, either naturally occurring or induced.

The terms "protein" and "polypeptide" refer to compounds comprising amino acids joined via peptide bonds and are used interchangeably. A "protein" or "polypeptide" encoded by a gene is not limited to the amino acid sequence encoded by the gene, but includes post-translational modifications of the protein.

Where the term "amino acid sequence" is recited herein to refer to an amino acid sequence of a protein molecule, "amino acid sequence" and like terms, such as "polypeptide" or "protein" are not meant to limit the amino acid sequence to the complete, native amino acid sequence associated with the recited protein molecule. Furthermore, an "amino acid sequence" can be deduced from the nucleic acid sequence encoding the protein.

The term "nascent" when used in reference to a protein refers to a newly synthesized protein, which has not been subject to post-translational modifications, which includes but is not limited to glycosylation and polypeptide shortening. The term "mature" when used in reference to a protein refers to a protein which has been subject to post-translational processing and/or which is in a cellular location (such as within a membrane or a multi-molecular complex) from which it can perform a particular function which it could not if it were not in the location.

The term "portion" when used in reference to a protein (as in "a portion of a given protein") refers to fragments of that protein. The fragments may range in size from four amino acid residues to the entire amino sequence minus one amino acid (for example, the range in size includes 4, 5, 6, 7, 8, 9, 10, or 11....amino acids up to the entire amino acid sequence minus one amino acid).

The term "chimera" when used in reference to a polypeptide refers to the expression product of two or more coding sequences obtained from different genes, that have been cloned together and that, after translation, act as a single polypeptide sequence. Chimeric polypeptides are also referred to as "hybrid" polypeptides. The coding sequences includes those obtained from the same or from different species of organisms.

The term "fusion" when used in reference to a polypeptide refers to a chimeric protein containing a protein of interest joined to an exogenous protein fragment (the fusion partner). The fusion partner may serve various functions, including enhancement of solubility of the polypeptide of interest, as well as providing an "affinity tag" to allow purification of the recombinant fusion polypeptide from a host cell or from a supernatant or from both. If desired, the fusion partner may be removed from the protein of interest after or during purification.

The term "homolog" or "homologous" when used in reference to a polypeptide refers to a high degree of sequence identity between two polypeptides, or to a high degree of similarity between the three-dimensional structure or to a high degree of similarity between the active site and the mechanism of action. In a preferred embodiment, a homolog has a greater than 60% sequence identity, and more preferably greater than 75% sequence identity, and still more preferably greater than 90% sequence identity, with a reference sequence.

As applied to polypeptides, the term "substantial identity" means that two peptide sequences, when optimally aligned, such as by the programs GAP or BESTFIT using default gap weights, share at least 80 percent sequence identity, preferably at least 90 percent sequence identity, more preferably at least 95 percent sequence identity or more (*e.g*., 99 percent sequence identity). Preferably, residue positions which are not identical differ by conservative amino acid substitutions.

The terms "variant" and "mutant" when used in reference to a polypeptide refer to an amino acid sequence that differs by one or more amino acids from another, usually related polypeptide. The variant may have "conservative" changes, wherein a substituted amino acid has similar structural or chemical properties. One type of conservative amino acid substitutions refers to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, and asparagine-glutamine. More rarely, a variant may have "non-conservative" changes (*e.g*., replacement of a glycine with a tryptophan). Similar minor variations may also include amino acid deletions or insertions (*i.e*., additions), or both. Guidance in determining which and how many amino acid residues may be substituted, inserted or deleted without abolishing biological activity may be found using computer programs well known in the art, for example, DNAStar software. Variants can be tested in functional assays. Preferred variants have less than 10%, and preferably less than 5%, and still more preferably less than 2% changes (whether substitutions, deletions, and so on).

The term "domain" when used in reference to a polypeptide refers to a subsection of the polypeptide which possesses a unique structural and/or functional characteristic; typically, this characteristic is similar across diverse polypeptides. The subsection typically comprises contiguous amino acids, although it may also comprise amino acids which act in concert or which are in close proximity due to folding or other configurations. Examples of a protein domain include the transmembrane domains, and the glycosylation sites.

The term "gene" refers to a nucleic acid (*e.g*., DNA or RNA) sequence that comprises coding sequences necessary for the production of an RNA, or a polypeptide or its precursor (*e.g*., proinsulin). A functional polypeptide can be encoded by a full length coding sequence or by any portion of the coding sequence as long as the desired activity or functional properties (*e.g*., enzymatic activity, ligand binding, signal transduction, etc.) of the polypeptide are retained. The term "portion" when used in reference to a gene refers to fragments of that gene. The fragments may range in size from a few nucleotides to the entire gene sequence minus one nucleotide. Thus, "a nucleotide comprising at least a portion of a gene" may comprise fragments of the gene or the entire gene.

The term "gene" also encompasses the coding regions of a structural gene and includes sequences located adjacent to the coding region on both the 5' and 3' ends for a distance of about 1 kb on either end such that the gene corresponds to the length of the full-length mRNA. The sequences which are located 5' of the coding region and which are present on the mRNA are referred to as 5' non-translated sequences. The sequences which are located 3' or downstream of the coding region and which are present on the mRNA are referred to as 3' non-translated sequences. The term "gene" encompasses both cDNA and genomic forms of a gene. A genomic form or clone of a gene contains the coding region interrupted with non-coding sequences termed "introns" or "intervening regions" or "intervening sequences." Introns are segments of a gene which are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

In addition to containing introns, genomic forms of a gene may also include sequences located on both the 5' and 3' end of the sequences which are present on the RNA transcript. These sequences are referred to as "flanking" sequences or regions (these flanking sequences are located 5' or 3' to the non-translated sequences present on the mRNA transcript). The 5' flanking region may contain regulatory sequences such as promoters and enhancers which control or influence the transcription of the gene. The 3' flanking region may contain sequences which direct the termination of transcription, posttranscriptional cleavage and polyadenylation.

In particular, the term *"CTL2* gene" refers to a full-length *CTL2* nucleotide sequence (*e.g*., as shown in SEQ ID NO:1 or SEQ ID NO:3). However, it is also intended that the term encompass fragments of the *CTL2* sequence, as well as other domains with the full-length *CTL2* nucleotide sequence. Furthermore, the terms *"CTL2 13* nucleotide sequence" or *"CTL2* polynucleotide sequence" encompasses DNA, genomic DNA, cDNA, and RNA (*e.g*., mRNA) sequences.

The term "heterologous" when used in reference to a gene refers to a gene encoding a factor that is not in its natural environment (*i.e*., has been altered by the hand of man). For example, a heterologous gene includes a gene from one species introduced into another species. A heterologous gene also includes a gene native to an organism that has been altered in some way (*e.g*., mutated, added in multiple copies, linked to a non-native promoter or enhancer sequence, etc.). Heterologous genes may comprise gene sequences that comprise cDNA forms of a gene; the cDNA sequences may be expressed in either a sense (to produce mRNA) or anti-sense orientation (to produce an anti-sense RNA transcript that is complementary to the mRNA transcript). Heterologous genes are distinguished from endogenous genes in that the heterologous gene sequences are typically joined to nucleotide sequences comprising regulatory elements such as promoters that are not found naturally associated with the gene for the protein encoded by the heterologous gene or with gene sequences in the chromosome, or are associated with portions of the chromosome not found in nature (*e.g.*, genes expressed in loci where the gene is not normally expressed).

The term "nucleotide sequence of interest" or "nucleic acid sequence of interest" refers to any nucleotide sequence (*e.g*., RNA or DNA), the manipulation of which may be deemed desirable for any reason (*e.g*., treat disease, confer improved qualities, *etc*.), by one of ordinary skill in the art. Such nucleotide sequences include, but are not limited to, coding sequences of structural genes (*e.g*., reporter genes, selection marker genes, oncogenes, drug resistance genes, growth factors, *etc.*), and non-coding regulatory sequences which do not encode an mRNA or protein product (*e.g*., promoter sequence, polyadenylation sequence, termination sequence, enhancer sequence, *etc*.).

The term "structural" when used in reference to a gene or to a nucleotide or nucleic acid sequence refers to a gene or a nucleotide or nucleic acid sequence whose ultimate expression product is a protein (such as an enzyme or a structural protein), an rRNA, an sRNA, a tRNA, etc.

The terms "oligonucleotide" or "polynucleotide" or "nucleotide" or "nucleic acid" refer to a molecule comprised of two or more deoxyribonucleotides or ribonucleotides, preferably more than three, and usually more than ten. The exact size will depend on many factors, which in turn depends on the ultimate function or use of the oligonucleotide. The oligonucleotide may be generated in any manner, including chemical synthesis, DNA replication, reverse transcription, or a combination thereof.

The terms "an oligonucleotide having a nucleotide sequence encoding a gene" or "a nucleic acid sequence encoding" a specified polypeptide refer to a nucleic acid sequence comprising the coding region of a gene or in other words the nucleic acid sequence which encodes a gene product. The coding region may be present in either a cDNA, genomic DNA or RNA form. When present in a DNA form, the oligonucleotide may be single-stranded (*i.e*., the sense strand) or double-stranded. Suitable control elements such as enhancers/promoters, splice junctions, polyadenylation signals, *etc*. may be placed in close proximity to the coding region of the gene if needed to permit proper initiation of transcription and/or correct processing of the primary RNA transcript. Alternatively, the coding region utilized in the expression vectors of the present invention may contain endogenous enhancers/promoters, splice junctions, intervening sequences, polyadenylation signals, *etc.* or a combination of both endogenous and exogenous control elements.

The term "recombinant" when made in reference to a nucleic acid molecule refers to a nucleic acid molecule which is comprised of segments of nucleic acid joined together by means of molecular biological techniques. The term "recombinant" when made in reference to a protein or a polypeptide refers to a protein molecule which is expressed using a recombinant nucleic acid molecule.

The terms "complementary" and "complementarity" refer to polynucleotides (*i.e.,* a sequence of nucleotides) related by the base-pairing rules. For example, for the sequence "5'-A-G-T-3'," is complementary to the sequence "3'-T-C-A-5'." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods which depend upon binding between nucleic acids.

The term "homology" when used in relation to nucleic acids refers to a degree of complementarity. There may be partial homology or complete homology (*i.e*., identity). "Sequence identity" refers to a measure of relatedness between two or more nucleic acids or proteins, and is given as a percentage with reference to the total comparison length. The identity calculation takes into account those nucleotide or amino acid residues that are identical and in the same relative positions in their respective larger sequences. Calculations of identity may be performed by algorithms contained within computer programs such as "GAP" (Genetics Computer Group, Madison, Wis.) and "ALIGN" (DNAStar, Madison, Wis.). A partially complementary sequence is one that at least partially inhibits (or competes with) a completely complementary sequence from hybridizing to a target nucleic acid is referred to using the functional term "substantially homologous." The inhibition of hybridization of the completely complementary sequence to the target sequence may be examined using a hybridization assay (Southern or Northern blot, solution hybridization and the like) under conditions of low stringency. A substantially homologous sequence or probe will compete for and inhibit the binding (*i.e*., the hybridization) of a sequence which is completely homologous to a target under conditions of low stringency. This is not to say that conditions of low stringency are such that non-specific binding is permitted; low stringency conditions require that the binding of two sequences to one another be a specific (*i.e*., selective) interaction. The absence of non-specific binding may be tested by the use of a second target which lacks even a partial degree of complementarity (*e.g*., less than about 30% identity); in the absence of non-specific binding the probe will not hybridize to the second non-complementary target.

The following terms are used to describe the sequence relationships between two or more polynucleotides: "reference sequence", "sequence identity", "percentage of sequence identity", and "substantial identity". A "reference sequence" is a defined sequence used as a basis for a sequence comparison; a reference sequence may be a subset of a larger sequence, for example, as a segment of a full-length cDNA sequence given in a sequence listing or may comprise a complete gene sequence. Generally, a reference sequence is at least 20 nucleotides in length, frequently at least 25 nucleotides in length, and often at least 50 nucleotides in length. Since two polynucleotides may each (1) comprise a sequence (*i.e*., a portion of the complete polynucleotide sequence) that is similar between the two polynucleotides, and (2) may further comprise a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window", as used herein, refers to a conceptual segment of at least 20 contiguous nucleotide positions wherein a polynucleotide sequence may be compared to a reference sequence of at least 20 contiguous nucleotides and wherein the portion of the polynucleotide sequence in the comparison window may comprise additions or deletions (*i.e*., gaps) of 20 percent or less as compared to the reference sequence (which does not comprise additions or deletions) for optimal alignment of the two sequences. Optimal alignment of sequences for aligning a comparison window may be conducted by the local homology algorithm of Smith and Waterman [Smith and Waterman, Adv. Appl. Math. 2: 482 (1981)] by the homology alignment algorithm of Needleman and Wunsch [Needleman and Wunsch, J. Mol. Biol. 48:443 (1970)], by the search for similarity method of Pearson and Lipman [Pearson and Lipman, Proc. Natl. Acad. Sci. (U.S.A.) 85:2444 (1988)], by computerized implementations of these algorithms (GAP, BESTFIT, FASTA, and TFASTA in the Wisconsin Genetics Software Package Release 7.0, Genetics Computer Group, 575 Science Dr., Madison, Wis.), or by inspection, and the best alignment (*i.e*., resulting in the highest percentage of homology over the comparison window) generated by the various methods is selected. The term "sequence identity" means that two polynucleotide sequences are identical (*i.e.*, on a nucleotide-by-nucleotide basis) over the window of comparison. The term "percentage of sequence identity" is calculated by comparing two optimally aligned sequences over the window of comparison, determining the number of positions at which the identical nucleic acid base (*e.g*., A, T, C, G, U, or I) occurs in both sequences to yield the number of matched positions, dividing the number of matched positions by the total number of positions in the window of comparison (i.e., the window size), and multiplying the result by 100 to yield the percentage of sequence identity. The terms "substantial identity" as used herein denotes a characteristic of a polynucleotide sequence, wherein the polynucleotide comprises a sequence that has at least 85 percent sequence identity, preferably at least 90 to 95 percent sequence identity, more usually at least 99 percent sequence identity as compared to a reference sequence over a comparison window of at least 20 nucleotide positions, frequently over a window of at least 25-50 nucleotides, wherein the percentage of sequence identity is calculated by comparing the reference sequence to the polynucleotide sequence which may include deletions or additions which total 20 percent or less of the reference sequence over the window of comparison. The reference sequence may be a subset of a larger sequence, for example, as a segment of the full-length sequences of the compositions claimed in the present invention.

The term "substantially homologous" when used in reference to a double-stranded nucleic acid sequence such as a cDNA or genomic clone refers to any probe that can hybridize to either or both strands of the double-stranded nucleic acid sequence under conditions of low to high stringency as described above.

The term "substantially homologous" when used in reference to a single-stranded nucleic acid sequence refers to any probe that can hybridize (*i.e*., it is the complement of) the single-stranded nucleic acid sequence under conditions of low to high stringency as described above.

The term "hybridization" refers to the pairing of complementary nucleic acids. Hybridization and the strength of hybridization (*i.e.*, the strength of the association between the nucleic acids) is impacted by such factors as the degree of complementary between the nucleic acids, stringency of the conditions involved, the Tₘ of the formed hybrid, and the G:C ratio within the nucleic acids. A single molecule that contains pairing of complementary nucleic acids within its structure is said to be "self-hybridized."

The term "Tₘ" refers to the "melting temperature" of a nucleic acid. The melting temperature is the temperature at which a population of double-stranded nucleic acid molecules becomes half dissociated into single strands. The equation for calculating the Tₘ of nucleic acids is well known in the art. As indicated by standard references, a simple estimate of the Tₘ value may be calculated by the equation: Tₘ = 81.5 + 0.41(% G + C), when a nucleic acid is in aqueous solution at 1 M NaCl (*See e.g.,* Anderson and Young, Quantitative Filter Hybridization, in Nucleic Acid Hybridization [1985]). Other references include more sophisticated computations that take structural as well as sequence characteristics into account for the calculation of Tₘ.

The term "stringency" refers to the conditions of temperature, ionic strength, and the presence of other compounds such as organic solvents, under which nucleic acid hybridizations are conducted. With "high stringency" conditions, nucleic acid base pairing will occur only between nucleic acid fragments that have a high frequency of complementary base sequences. Thus, conditions of "low" stringency are often required with nucleic acids that are derived from organisms that are genetically diverse, as the frequency of complementary sequences is usually less.

"Low stringency conditions" when used in reference to nucleic acid hybridization comprise conditions equivalent to binding or hybridization, for example, at 42°C in a solution consisting of 5X SSPE (43.8 g/l NaCl, 6.9 g/l NaH₂PO₄•H₂O and 1.85 g/l EDTA, pH adjusted to 7.4 with NaOH), 0.1% SDS, 5X Denhardt's reagent [50X Denhardt's contains per 500 ml: 5 g Ficoll (Type 400, Pharmacia), 5 g BSA (Fraction V; Sigma)] and 100 µg/ml denatured salmon sperm DNA followed by washing in a solution comprising 5X SSPE, 0.1% SDS at 42°C when a probe of about 500 nucleotides in length is employed. However, the present invention is not limited to the hybridization of probes of about 500 nucleotides in length. The present invention contemplates the use of probes between approximately 10 nucleotides up to several thousand (*e.g*., at least 5000) nucleotides in length. One skilled in the relevant art understands that stringency conditions may be altered for probes of other sizes (See *e.g*., Anderson and Young (1985) Quantitative Filter Hybridization, in Nucleic Acid Hybridization; and Sambrook et al. (1989) Molecular Cloning: A Laboratory Manual (Cold Spring Harbor Press, NY).

"Medium stringency conditions" when used in reference to nucleic acid hybridization comprise conditions equivalent to binding or hybridization, for example, at 42°C in a solution consisting of 5X SSPE (43.8 g/l NaCl, 6.9 g/l NaH₂PO₄•H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 1.0X SSPE, 1.0% SDS at 42°C when a probe of about 500 nucleotides in length is employed.

"High stringency conditions" when used in reference to nucleic acid hybridization comprise conditions equivalent to binding or hybridization, for example, at 42°C in a solution consisting of 5X SSPE (43.8 g/l NaCl, 6.9 g/l NaH₂PO₄•H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 0.1X SSPE, 1.0% SDS at 42°C when a probe of about 500 nucleotides in length is employed.

It is well known that numerous equivalent conditions may be employed to comprise low stringency conditions; factors such as the length and nature (DNA, RNA, base composition) of the probe and nature of the target (DNA, RNA, base composition, present in solution or immobilized, etc.) and the concentration of the salts and other components (*e.g*., the presence or absence of formamide, dextran sulfate, polyethylene glycol) are considered and the hybridization solution may be varied to generate conditions of low stringency hybridization different from, but equivalent to, the above listed conditions. In addition, the art knows conditions that promote hybridization under conditions of high stringency (*e.g*., increasing the temperature of the hybridization and/or wash steps, the use of formamide in the hybridization solution, etc.).

The term "wild-type" when made in reference to a gene refers to a gene that has the characteristics of a gene isolated from a naturally occurring source. The term "wild-type" when made in reference to a gene product refers to a gene product that has the characteristics of a gene product isolated from a naturally occurring source. The term "naturally-occurring" as applied to an object refers to the fact that an object can be found in nature. For example, a polypeptide or polynucleotide sequence that is present in an organism (including viruses) that can be isolated from a source in nature and which has not been intentionally modified by man in the laboratory is naturally-occurring. A wild-type gene is frequently that gene which is most frequently observed in a population and is thus arbitrarily designated the "normal" or "wild-type" form of the gene. In contrast, the term "modified" or "mutant" when made in reference to a gene or to a gene product refers, respectively, to a gene or to a gene product which displays modifications in sequence and/or functional properties (i.e., altered characteristics) when compared to the wild-type gene or gene product. It is noted that naturally-occurring mutants can be isolated; these are identified by the fact that they have altered characteristics when compared to the wild-type gene or gene product.

The term "allele" refers to different variations in a gene; the variations include but are not limited to variants and mutants, polymorphic loci and single nucleotide polymorphic loci, frameshift and splice mutations. An allele may occur naturally in a population, or it might arise during the lifetime of any particular individual of the population.

Thus, the terms "variant" and "mutant" when used in reference to a nucleotide sequence refer to an nucleic acid sequence that differs by one or more nucleotides from another, usually related nucleotide acid sequence. A "variation" is a difference between two different nucleotide sequences; typically, one sequence is a reference sequence.

The term "polymorphic locus" refers to a genetic locus present in a population that shows variation between members of the population (*i.e.*, the most common allele has a frequency of less than 0.95). Thus, "polymorphism" refers to the existence of a character in two or more variant forms in a population. A "single nucleotide polymorphism" (or SNP) refers a genetic locus of a single base which may be occupied by one of at least two different nucleotides. In contrast, a "monomorphic locus" refers to a genetic locus at which little or no variations are seen between members of the population (generally taken to be a locus at which the most common allele exceeds a frequency of 0.95 in the gene pool of the population).

A "frameshift mutation" refers to a mutation in a nucleotide sequence, usually resulting from insertion or deletion of a single nucleotide (or two or four nucleotides) which results in a change in the correct reading frame of a structural DNA sequence encoding a protein. The altered reading frame usually results in the translated aminoacid sequence being changed or truncated.

A "splice mutation" refers to any mutation that affects gene expression by affecting correct RNA splicing. Splicing mutation may be due to mutations at intron-exon boundaries which alter splice sites.

The term "detection assay" refers to an assay for detecting the presence or absence of a wild-type or variant nucleic acid sequence (*e.g*., mutation or polymorphism) in a given allele of a particular gene (*e.g., CTL2* gene), or for detecting the presence or absence of a particular protein (*e.g*., CTL2 or IESCA) or the activity or effect of a particular protein (*e.g*., choline transport or a role in hearing) or for detecting the presence or absence of a variant of a particular protein.

The term "antisense" refers to a deoxyribonucleotide sequence whose sequence of deoxyribonucleotide residues is in reverse 5' to 3' orientation in relation to the sequence of deoxyribonucleotide residues in a sense strand of a DNA duplex. A "sense strand" of a DNA duplex refers to a strand in a DNA duplex which is transcribed by a cell in its natural state into a "sense mRNA." Thus an "antisense" sequence is a sequence having the same sequence as the non-coding strand in a DNA duplex. The term "antisense RNA" refers to a RNA transcript that is complementary to all or part of a target primary transcript or mRNA and that blocks the expression of a target gene by interfering with the processing, transport and/or translation of its primary transcript or mRNA. The complementarity of an antisense RNA may be with any part of the specific gene transcript, *i.e*., at the 5' non-coding sequence, 3' non-coding sequence, introns, or the coding sequence. In addition, as used herein, antisense RNA may contain regions of ribozyme sequences that increase the efficacy of antisense RNA to block gene expression. "Ribozyme" refers to a catalytic RNA and includes sequence-specific endoribonucleases. "Antisense inhibition" refers to the production of antisense RNA transcripts capable of preventing the expression of the target protein.

"Amplification" is a special case of nucleic acid replication involving template specificity. It is to be contrasted with non-specific template replication (*i.e*., replication that is template-dependent but not dependent on a specific template). Template specificity is here distinguished from fidelity of replication (*i.e*., synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out.

Template specificity is achieved in most amplification techniques by the choice of enzyme. Amplification enzymes are enzymes that, under conditions they are used, will process only specific sequences of nucleic acid in a heterogeneous mixture of nucleic acid. For example, in the case of Q β replicase, MDV-1 RNA is the specific template for the replicase (Kacian et al., Proc. Natl. Acad. Sci. USA, 69:3038 [1972]). Other nucleic acid will not be replicated by this amplification enzyme. Similarly, in the case of T7 RNA polymerase, this amplification enzyme has a stringent specificity for its own promoters (Chamberlin et al., Nature, 228:227 [1970]). In the case of T4 DNA ligase, the enzyme will not ligate the two oligonucleotides or polynucleotides, where there is a mismatch between the oligonucleotide or polynucleotide substrate and the template at the ligation junction (Wu and Wallace, Genomics, 4:560 [1989]). Finally, *Taq* and *Pfu* polymerases, by virtue of their ability to function at high temperature, are found to display high specificity for the sequences bounded and thus defined by the primers; the high temperature results in thermodynamic conditions that favor primer hybridization with the target sequences and not hybridization with non-target sequences (H.A. Erlich (ed.), PCR Technology, Stockton Press [1989]).

The term "amplifiable nucleic acid" refers to nucleic acids that may be amplified by any amplification method. It is contemplated that "amplifiable nucleic acid" will usually comprise "sample template."

The term "sample template" refers to nucleic acid originating from a sample that is analyzed for the presence of "target" (defined below). In contrast, "background template" is used in reference to nucleic acid other than sample template that may or may not be present in a sample. Background template is most often inadvertent. It may be the result of carryover, or it may be due to the presence of nucleic acid contaminants sought to be purified away from the sample. For example, nucleic acids from organisms other than those to be detected may be present as background in a test sample.

The term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (*i.e.,* in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer and the use of the method.

The term "probe" refers to an oligonucleotide (*i.e*., a sequence of nucleotides), whether occurring naturally as in a purified restriction digest or produced synthetically, recombinantly or by PCR amplification, that is capable of hybridizing to another oligonucleotide of interest. A probe may be single-stranded or double-stranded. Probes are useful in the detection, identification and isolation of particular gene sequences. It is contemplated that any probe used in the present invention will be labeled with any "reporter molecule," so that is detectable in any detection system, including, but not limited to enzyme (*e.g*., ELISA, as well as enzyme-based histochemical assays), fluorescent, radioactive, and luminescent systems. It is not intended that the present invention be limited to any particular detection system or label.

The term "target," when used in reference to the polymerase chain reaction, refers to the region of nucleic acid bounded by the primers used for polymerase chain reaction. Thus, the "target" is sought to be sorted out from other nucleic acid sequences. A "segment" is defined as a region of nucleic acid within the target sequence.

The term "polymerase chain reaction" ("PCR") refers to the method of K.B. Mullis U.S. Patent Nos. 4,683,195, 4,683,202, and 4,965,188, that describe a method for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing, and polymerase extension can be repeated many times (*i.e*., denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified."

With PCR, it is possible to amplify a single copy of a specific target sequence in genomic DNA to a level detectable by several different methodologies (*e.g*., hybridization with a labeled probe; incorporation of biotinylated primers followed by avidin-enzyme conjugate detection; incorporation of ³²P-labeled deoxynucleotide triphosphates, such as dCTP or dATP, into the amplified segment). In addition to genomic DNA, any oligonucleotide or polynucleotide sequence can be amplified with the appropriate set of primer molecules. In particular, the amplified segments created by the PCR process itself are, themselves, efficient templates for subsequent PCR amplifications.

The terms "PCR product," "PCR fragment," and "amplification product" refer to the resultant mixture of compounds after two or more cycles of the PCR steps of denaturation, annealing and extension are complete. These terms encompass the case where there has been amplification of one or more segments of one or more target sequences.

The term "amplification reagents" refers to those reagents (deoxyribonucleotide triphosphates, buffer, etc.), needed for amplification except for primers, nucleic acid template, and the amplification enzyme. Typically, amplification reagents along with other reaction components are placed and contained in a reaction vessel (test tube, microwell, etc.).

The term "reverse-transcriptase" or "RT-PCR" refers to a type of PCR where the starting material is mRNA. The starting mRNA is enzymatically converted to complementary DNA or "cDNA" using a reverse transcriptase enzyme. The cDNA is then used as a "template" for a "PCR" reaction.

The term "gene expression" refers to the process of converting genetic information encoded in a gene into RNA (*e.g*., mRNA, rRNA, tRNA, or snRNA) through "transcription" of the gene (*i.e*., via the enzymatic action of an RNA polymerase), and into protein, through "translation" of mRNA. Gene expression can be regulated at many stages in the process. "Up-regulation" or "activation" refers to regulation that increases the production of gene expression products (*i.e*., RNA or protein), while "down-regulation" or "repression" refers to regulation that decrease production. Molecules (*e.g*., transcription factors) that are involved in up-regulation or down-regulation are often called "activators" and "repressors," respectively.

The terms "in operable combination", "in operable order" and "operably linked" refer to the linkage of nucleic acid sequences in such a manner that a nucleic acid molecule capable of directing the transcription of a given gene and/or the synthesis of a desired protein molecule is produced. The term also refers to the linkage of amino acid sequences in such a manner so that a functional protein is produced.

The term "regulatory element" refers to a genetic element which controls some aspect of the expression of nucleic acid sequences. For example, a promoter is a regulatory element which facilitates the initiation of transcription of an operably linked coding region. Other regulatory elements are splicing signals, polyadenylation signals, termination signals, *etc.*

Transcriptional control signals in eukaryotes comprise "promoter" and "enhancer" elements. Promoters and enhancers consist of short arrays of DNA sequences that interact specifically with cellular proteins involved in transcription (Maniatis, et al., Science 236:1237, 1987). Promoter and enhancer elements have been isolated from a variety of eukaryotic sources including genes in yeast, insect, mammalian and plant cells. Promoter and enhancer elements have also been isolated from viruses and analogous control elements, such as promoters, are also found in prokaryotes. The selection of a particular promoter and enhancer depends on the cell type used to express the protein of interest. Some eukaryotic promoters and enhancers have a broad host range while others are functional in a limited subset of cell types *(for review, see* Voss, et al., Trends Biochem. Sci., 11:287, 1986; and Maniatis, *et al., supra 1987).*

The terms "promoter element," "promoter," or "promoter sequence" refer to a DNA sequence that is located at the 5' end (*i.e*. precedes) of the coding region of a DNA polymer. The location of most promoters known in nature precedes the transcribed region. The promoter functions as a switch, activating the expression of a gene. If the gene is activated, it is said to be transcribed, or participating in transcription. Transcription involves the synthesis of mRNA from the gene. The promoter, therefore, serves as a transcriptional regulatory element and also provides a site for initiation of transcription of the gene into mRNA.

The term "regulatory region" refers to a gene's 5' transcribed but untranslated regions, located immediately downstream from the promoter and ending just prior to the translational start of the gene.

The term "promoter region" refers to the region immediately upstream of the coding region of a DNA polymer, and is typically between about 500 bp and 4 kb in length, and is preferably about 1 to 1.5 kb in length.

Promoters may be tissue specific or cell specific. The term "tissue specific" as it applies to a promoter refers to a promoter that is capable of directing selective expression of a nucleotide sequence of interest to a specific type of tissue (*e.g*., inner ear) in the relative absence of expression of the same nucleotide sequence of interest in a different type of tissue (*e.g*., tongue). Tissue specificity of a promoter may be evaluated by, for example, operably linking a reporter gene to the promoter sequence to generate a reporter construct, introducing the reporter construct into the genome of an organism such that the reporter construct is integrated into every tissue of the resulting transgenic organism, and detecting the expression of the reporter gene (*e.g*., detecting mRNA, protein, or the activity of a protein encoded by the reporter gene) in different tissues of the transgenic organism. The detection of a greater level of expression of the reporter gene in one or more tissues relative to the level of expression of the reporter gene in other tissues shows that the promoter is specific for the tissues in which greater levels of expression are detected. The term "cell type specific" as applied to a promoter refers to a promoter which is capable of directing selective expression of a nucleotide sequence of interest in a specific type of cell in the relative absence of expression of the same nucleotide sequence of interest in a different type of cell within the same tissue. The term "cell type specific" when applied to a promoter also means a promoter capable of promoting selective expression of a nucleotide sequence of interest in a region within a single tissue. Cell type specificity of a promoter may be assessed using methods well known in the art, e.g., immunohistochemical staining. Briefly, tissue sections are embedded in paraffin, and paraffin sections are reacted with a primary antibody which is specific for the polypeptide product encoded by the nucleotide sequence of interest whose expression is controlled by the promoter. A labeled (*e.g*., peroxidase conjugated) secondary antibody which is specific for the primary antibody is allowed to bind to the sectioned tissue and specific binding detected (*e.g*., with avidin/biotin) by microscopy.

Promoters may be constitutive or inducible. The term "constitutive" when made in reference to a promoter means that the promoter is capable of directing transcription of an operably linked nucleic acid sequence in the absence of a stimulus (*e.g*., heat shock, chemicals, light, *etc*.). Typically, constitutive promoters are capable of directing expression of a transgene in substantially any cell and any tissue.

In contrast, an "inducible" promoter is one which is capable of directing a level of transcription of an operably linked nucleic acid sequence in the presence of a stimulus (*e.g*., heat shock, chemicals, light, *etc*.) which is different from the level of transcription of the operably linked nucleic acid sequence in the absence of the stimulus.

The term "regulatory element" refers to a genetic element that controls some aspect of the expression of nucleic acid sequence(s). For example, a promoter is a regulatory element that facilitates the initiation of transcription of an operably linked coding region. Other regulatory elements are splicing signals, polyadenylation signals, termination signals, etc.

The enhancer and/or promoter may be "endogenous" or "exogenous" or "heterologous." An "endogenous" enhancer or promoter is one that is naturally linked with a given gene in the genome. An "exogenous" or "heterologous" enhancer or promoter is one that is placed in juxtaposition to a gene by means of genetic manipulation (*i.e*., molecular biological techniques) such that transcription of the gene is directed by the linked enhancer or promoter. For example, an endogenous promoter in operable combination with a first gene can be isolated, removed, and placed in operable combination with a second gene, thereby making it a "heterologous promoter" in operable combination with the second gene. A variety of such combinations are contemplated (*e.g*., the first and second genes can be from the same species, or from different species).

The term "naturally linked" or "naturally located" when used in reference to the relative positions of nucleic acid sequences means that the nucleic acid sequences exist in nature in the relative positions.

The presence of "splicing signals" on an expression vector often results in higher levels of expression of the recombinant transcript in eukaryotic host cells. Splicing signals mediate the removal of introns from the primary RNA transcript and consist of a splice donor and acceptor site (Sambrook, et al., Molecular Cloning: A Laboratory Manual, 2nd ed., Cold Spring Harbor Laboratory Press, New York [1989] pp. 16.7-16.8). A commonly used splice donor and acceptor site is the splice junction from the 16S RNA of SV40.

Efficient expression of recombinant DNA sequences in eukaryotic cells requires expression of signals directing the efficient termination and polyadenylation of the resulting transcript. Transcription termination signals are generally found downstream of the polyadenylation signal and are a few hundred nucleotides in length. The term "poly(A) site" or "poly(A) sequence" as used herein denotes a DNA sequence which directs both the termination and polyadenylation of the nascent RNA transcript. Efficient polyadenylation of the recombinant transcript is desirable, as transcripts lacking a poly(A) tail are unstable and are rapidly degraded. The poly(A) signal utilized in an expression vector may be "heterologous" or "endogenous." An endogenous poly(A) signal is one that is found naturally at the 3' end of the coding region of a given gene in the genome. A heterologous poly(A) signal is one which has been isolated from one gene and positioned 3' to another gene. A commonly used heterologous poly(A) signal is the SV40 poly(A) signal. The SV40 poly(A) signal is contained on a 237 bp *Bam*HI/*Bcl*I restriction fragment and directs both termination and polyadenylation (Sambrook, *supra,* at 16.6-16.7).

The term "vector" refers to nucleic acid molecules that transfer DNA segment(s) from one cell to another. The term "vehicle" is sometimes used interchangeably with "vector."

The terms "expression vector" or "expression cassette" refer to a recombinant DNA molecule containing a desired coding sequence and appropriate nucleic acid sequences necessary for the expression of the operably linked coding sequence in a particular host organism. Nucleic acid sequences necessary for expression in prokaryotes usually include a promoter, an operator (optional), and a ribosome binding site, often along with other sequences. Eukaryotic cells are known to utilize promoters, enhancers, and termination and polyadenylation signals.

The term "transfection" refers to the introduction of foreign DNA into cells. Transfection may be accomplished by a variety of means known to the art including calcium phosphate-DNA co-precipitation, DEAE-dextran-mediated transfection, polybrene-mediated transfection, glass beads, electroporation, microinjection, liposome fusion, lipofection, protoplast fusion, viral infection, biolistics (i.e., particle bombardment) and the like.

The term "stable transfection" or "stably transfected" refers to the introduction and integration of foreign DNA into the genome of the transfected cell. The term "stable transfectant" refers to a cell that has stably integrated foreign DNA into the genomic DNA.

The term "transient transfection" or "transiently transfected" refers to the introduction of foreign DNA into a cell where the foreign DNA fails to integrate into the genome of the transfected cell. The foreign DNA persists in the nucleus of the transfected cell for several days. During this time the foreign DNA is subject to the regulatory controls that govern the expression of endogenous genes in the chromosomes. The term "transient transfectant" refers to cells that have taken up foreign DNA but have failed to integrate this DNA.

The term "calcium phosphate co-precipitation" refers to a technique for the introduction of nucleic acids into a cell. The uptake of nucleic acids by cells is enhanced when the nucleic acid is presented as a calcium phosphate-nucleic acid co-precipitate. The original technique of Graham and van der Eb (Graham and van der Eb, Virol., 52:456 [1973]), has been modified by several groups to optimize conditions for particular types of cells. The art is well aware of these numerous modifications.

The terms "infecting" and "infection" when used with a bacterium refer to co-incubation of a target biological sample, (*e.g*., cell, tissue, *etc*.) with the bacterium under conditions such that nucleic acid sequences contained within the bacterium are introduced into one or more cells of the target biological sample.

The terms "bombarding, "bombardment," and "biolistic bombardment" refer to the process of accelerating particles towards a target biological sample (*e.g*., cell, tissue, *etc.*) to effect wounding of the cell membrane of a cell in the target biological sample and/or entry of the particles into the target biological sample. Methods for biolistic bombardment are known in the art (*e.g*., U.S. Patent No. 5,584,807), and are commercially available (*e.g*., the helium gas-driven microprojectile accelerator (PDS-1000/He, BioRad).

The term "transgene" refers to a foreign gene that is placed into an organism by the process of transfection. The term "foreign gene" refers to any nucleic acid (*e.g*., gene sequence) that is introduced into the genome of an organism by experimental manipulations and may include gene sequences found in that organism so long as the introduced gene does not reside in the same location as does the naturally-occurring gene.

The term "transgenic" when used in reference to a host cell or an organism refers to a host cell or an organism that contains at least one heterologous or foreign gene in the host cell or in one or more of cells of the organism.

The term "host cell" refers to any cell capable of replicating and/or transcribing and/or translating a heterologous gene. Thus, a "host cell" refers to any eukaryotic or prokaryotic cell (*e.g*., bacterial cells such as *E*. *coli,* yeast cells, mammalian cells, avian cells, amphibian cells, plant cells, fish cells, and insect cells), whether located *in vitro* or *in vivo.* For example, host cells may be located in a transgenic animal.

The terms "transformants" or "transformed cells" include the primary transformed cell and cultures derived from that cell without regard to the number of transfers. All progeny may not be precisely identical in DNA content, due to deliberate or inadvertent mutations. Mutant progeny that have the same functionality as screened for in the originally transformed cell are included in the definition of transformants.

The term "selectable marker" refers to a gene which encodes an enzyme having an activity that confers resistance to an antibiotic or drug upon the cell in which the selectable marker is expressed, or which confers expression of a trait which can be detected (*e.g*.., luminescence or fluorescence). Selectable markers may be "positive" or "negative." Examples of positive selectable markers include the neomycin phosphotrasferase (NPTII) gene which confers resistance to G418 and to kanamycin, and the bacterial hygromycin phosphotransferase gene (*hyg*), which confers resistance to the antibiotic hygromycin. Negative selectable markers encode an enzymatic activity whose expression is cytotoxic to the cell when grown in an appropriate selective medium. For example, the HSV-*tk* gene is commonly used as a negative selectable marker. Expression of the HSV-*tk* gene in cells grown in the presence of gancyclovir or acyclovir is cytotoxic; thus, growth of cells in selective medium containing gancyclovir or acyclovir selects against cells capable of expressing a functional HSV TK enzyme.

The term "reporter gene" refers to a gene encoding a protein that may be assayed. Examples of reporter genes include, but are not limited to, luciferase (*See, e.g.,* deWet et al., Mol. Cell. Biol. 7:725 [1987] and U.S. Pat Nos.,6,074,859; 5,976,796; 5,674,713; and 5,618,682,) green fluorescent protein (*e.g.*, GenBank Accession Number U43284; a number of GFP variants are commercially available from CLONTECH Laboratories, Palo Alto, CA), chloramphenicol acetyltransferase, β-galactosidase, alkaline phosphatase, and horse radish peroxidase.

The term "overexpression" refers to the production of a gene product in transgenic organisms that exceeds levels of production in normal or non-transformed organisms. The term "cosuppression" refers to the expression of a foreign gene which has substantial homology to an endogenous gene resulting in the suppression of expression of both the foreign and the endogenous gene. As used herein, the term "altered levels" refers to the production of gene product(s) in transgenic organisms in amounts or proportions that differ from that of normal or non-transformed organisms.

The terms "Southern blot analysis" and "Southern blot" and "Southern" refer to the analysis of DNA on agarose or acrylamide gels in which DNA is separated or fragmented according to size followed by transfer of the DNA from the gel to a solid support, such as nitrocellulose or a nylon membrane. The immobilized DNA is then exposed to a labeled probe to detect DNA species complementary to the probe used. The DNA may be cleaved with restriction enzymes prior to electrophoresis. Following electrophoresis, the DNA may be partially depurinated and denatured prior to or during transfer to the solid support. Southern blots are a standard tool of molecular biologists (J. Sambrook et al. [1989] Molecular Cloning: A Laboratory Manual, Cold Spring Harbor Press, NY, pp 9.31-9.58).

The term "Northern blot analysis" and "Northern blot" and "Northern" refer to the analysis of RNA by electrophoresis of RNA on agarose gels to fractionate the RNA according to size followed by transfer of the RNA from the gel to a solid support, such as nitrocellulose or a nylon membrane. The immobilized RNA is then probed with a labeled probe to detect RNA species complementary to the probe used. Northern blots are a standard tool of molecular biologists (J. Sambrook, *et al.* [1989] *supra,* pp 7.39-7.52).

The terms "Western blot analysis" and "Western blot" and "Western" refers to the analysis of protein(s) (or polypeptides) immobilized onto a support such as nitrocellulose or a membrane. A mixture comprising at least one protein is first separated on an acrylamide gel, and the separated proteins are then transferred from the gel to a solid support, such as nitrocellulose or a nylon membrane. The immobilized proteins are exposed to at least one antibody with reactivity against at least one antigen of interest. The bound antibodies may be detected by various methods, including the use of radiolabeled antibodies.

The term "antigenic determinant" refers to that portion of an antigen that makes contact with a particular antibody (*i.e.*, an epitope). When a protein or fragment of a protein is used to immunize a host animal, numerous regions of the protein may induce the production of antibodies that bind specifically to a given region or three-dimensional structure on the protein; these regions or structures are referred to as antigenic determinants. An antigenic determinant may compete with the intact antigen (*i.e*., the "immunogen" used to elicit the immune response) for binding to an antibody.

The term "isolated" when used in relation to a nucleic acid, as in "an isolated oligonucleotide" refers to a nucleic acid sequence that is identified and separated from at least one contaminant nucleic acid with which it is ordinarily associated in its natural source. Isolated nucleic acid is present in a form or setting that is different from that in which it is found in nature. In contrast, non-isolated nucleic acids, such as DNA and RNA, are found in the state they exist in nature. Examples of non-isolated nucleic acids include: a given DNA sequence (*e.g*., a gene) found on the host cell chromosome in proximity to neighboring genes; RNA sequences, such as a specific mRNA sequence encoding a specific protein, found in the cell as a mixture with numerous other mRNAs which encode a multitude of proteins. However, isolated nucleic acid encoding a particular protein includes, by way of example, such nucleic acid in cells ordinarily expressing the protein, where the nucleic acid is in a chromosomal location different from that of natural cells, or is otherwise flanked by a different nucleic acid sequence than that found in nature. The isolated nucleic acid or oligonucleotide may be present in single-stranded or double-stranded form. When an isolated nucleic acid or oligonucleotide is to be utilized to express a protein, the oligonucleotide will contain at a minimum the sense or coding strand (*i.e*., the oligonucleotide may single-stranded), but may contain both the sense and anti-sense strands (*i.e*., the oligonucleotide may be double-stranded).

The term "purified" refers to molecules, either nucleic or amino acid sequences, that are removed from their natural environment, isolated or separated. An "isolated nucleic acid sequence" may therefore be a purified nucleic acid sequence. "Substantially purified" molecules are at least 60% free, preferably at least 75% free, and more preferably at least 90% free from other components with which they are naturally associated. As used herein, the term "purified" or "to purify" also refer to the removal of contaminants from a sample. The removal of contaminating proteins results in an increase in the percent of polypeptide of interest in the sample. In another example, recombinant polypeptides are expressed in plant, bacterial, yeast, or mammalian host cells and the polypeptides are purified by the removal of host cell proteins; the percent of recombinant polypeptides is thereby increased in the sample.

The term "composition comprising" a given polynucleotide sequence or polypeptide refers broadly to any composition containing the given polynucleotide sequence or polypeptide. The composition may comprise an aqueous solution. Compositions comprising polynucleotide sequences encoding CTL2 (*e.g*., SEQ ID NO:1 or SEQ ID NO:3) or fragments thereof may be employed as hybridization probes. In this case, the CTL2 encoding polynucleotide sequences are typically employed in an aqueous solution containing salts *(e.g.,* NaCl), detergents (*e.g*., SDS), and other components (*e.g*., Denhardt's solution, dry milk, salmon sperm DNA, etc.).

The term "test compound" refers to any chemical entity, pharmaceutical, drug, and the like that can be used to treat or prevent a disease, illness, sickness, or disorder of bodily function, or otherwise alter the physiological or cellular status of a sample. Test compounds comprise both known and potential therapeutic compounds. A test compound can be determined to be therapeutic by screening using the screening methods of the present invention. A "known therapeutic compound" refers to a therapeutic compound that has been shown (*e.g*., through animal trials or prior experience with administration to humans) to be effective in such treatment or prevention.

As used herein, the term "response," when used in reference to an assay, refers to the generation of a detectable signal (*e.g.*, accumulation of reporter protein, increase in ion concentration, accumulation of a detectable chemical product).

The term "sample" is used in its broadest sense. In one sense it can refer to an animal cell or tissue. In another sense, it is meant to include a specimen or culture obtained from any source, as well as biological and environmental samples. Biological samples may be obtained from plants or animals (including humans) and encompass fluids, solids, tissues, and gases. Environmental samples include environmental material such as surface matter, soil, water, and industrial samples. These examples are not to be construed as limiting the sample types applicable to the present invention.

### DESCRIPTION OF THE INVENTION

The present invention is defined in the claims and relates generally to antigens reactive with autoantibodies associated with autoimmune sensorineural hearing loss (AISNHL). In particular, the present invention provides a purified novel antigen, Inner Ear Supporting Cell Antigen (IESCA) which are reactive with autoantibodies associated with autoimmune sensorineural hearing loss (AISNHL). The invention also relates to compounds and methods for the screening of sera for antibodies reactive to the antigens. The presence of these antibodies in the patient's sera is predictive of a patient's potential to respond to steroid treatment for AISNHL. Additionally, the invention relates to methods for the detection of compounds that are agonistic and/or antagonistic for IESCA binding and/or anti-IESCA binding, as well as methods for the identification of intra- and inter-specific homologs of IESCA. The present invention also relates to methods of detecting other proteins involved in hearing function and/or loss, and in particular in AISNHL; at least one of these other protein, cochlin, appears to form a complex with IESCA. The invention also relates to methods of detecting genetic lesions in genes encoding for such antigens.

### I. Autoimmune Sensorineural Hearing Loss (AISNHL)

The evidence of antibody to inner-ear antigens in sera from patients with the clinical diagnosis of autoimmune sensorineural hearing loss (AISNHL) and experimental models show a causal relationship between autoimmunity and sensorineural hearing loss (SNHL) (Harris (1983) Otolaryngol. Head Neck Surg. 91:18-23; Yoo et al. (1983) Am. J. Otolaryngol 6:209-216; Harris (1987) Laryngoscope 97:63-76; Harris and Sharp (1990) Laryngoscope 100:516-524; Orozco et al. (1990) Laryngoscope 100:941-947; Cruz et al., (1990) Am. J. Otol. 11:342-346; McCabe (1991) Adv. Otorhinolaryngol 46:78-81; Kusakari et al. (1992) Ann. Otol. Rhinol. Laryngol. 101:82-86; Tago et al. (1992) Ann. Otol. Rhinol. Laryngol, 157(Suppl.):87-91; Wong et al. (1992) Hear. Res. 59:93-100; Sone et al. (1994) Hear. Res. 83:26-36; Moscicki et al. (1994) JAMA 272:611-616; Harris and Ryan (1995) Otolaryngol. Head Neck Surg. 112:639-653; Billings et al. (1995) Ann. Otolol. Rhinol. Larngol. 104:181-188; Gottschlich et al. (1995) Laryngoscope 105:1347-1352; Cao et al. (1995) Mol. Cell Biochem. 146:157-163; Nair et al. (1995) Hear. Res. 83:101-113; Cao et al. (1996) Laryngoscope 106:207-212). In this regard, the importance of identifying antigens specific for AISNHL for the establishment of accurate method for diagnoses becomes critical.

### A. Animal Models of AISNHL

In 1987 Harris (Harris (1997) Laryngoscope 97:63-76) demonstrated hearing loss and inner-ear lesions in guinea pigs immunized with bovine inner-ear extract. Subsequently, Orosco *et al.* (Orozco et al. (1990) Laryngoscope 100:941-947) immunized mice and guinea pigs with chick and guinea pig inner-ear tissues and found that the animals developed transient hearing loss and serum antibodies to hair cell stereocilia.

The present inventors followed these experiments with the development of monoclonal antibodies to inner-ear antigens by immunizing mice with chick and guinea pig inner-ear tissue (Zajic et al. (1991) Hear. Res. 52:59-72; Ptok et al. (1991) Hear. Res. 57:79-90). Two classes of monoclonal antibodies (MAb) to inner-ear antigens were characterized. One class (KHRI-5 and KHRI-6) stains stereocilia (Ptok et al. (1991) Hear. Res. 57:79-90). The other class is represented by MAb KHRI-3, which binds to inner ear supporting cells in a characteristic punctate "stacked wine glass" staining pattern (Zajic et al. (1991) Hear. Res. 52:59-72). MaB KHRI-3 also identifies a protein in Western blots of inner-ear extracts (Nair et al. (1995) Hear. Res. 83:101-113). The protein to which KHRI-3 binds was termed Inner Ear Supporting Cell Antigen, or IESCA.

*In vivo* studies showed that mice carrying the KHRI-3 hybridoma develop high-frequency hearing loss (Nair et al. (1995) Hear. Res. 83:101-113; Disher et al. (1997) Ann. NY Acad. Sci. 830:253-265). The hearing loss is associated with high circulating KHRI-3 antibody titers and loss of outer hair cells in the basal turn of the cochlea, the region that encodes high-frequency sounds. More recent studies using *in* vivo infusion of KHRI-3 antibody directly into the guinea pig cochlea have shown that KHRI-3 binds to supporting cells *in vivo,* and that animals receiving this antibody, but not an isotype-matched IgG1 myeloma protein, develop hearing loss (Nair et al. (1997) Hear. Res. 107:93-101). These experimental studies provide strong evidence for antibody-mediated disruption of hearing.

### B. AISNHL In Humans

Support for antibody-mediated hearing loss in humans also has accumulated. Harris and his colleagues demonstrated that serum antibodies from patients with AISNHL bind to a 68 kD protein in bovine inner-ear tissue extracts (Harris and Sharp (1990) Laryngoscope 100:516-524; Harris and Ryan (1995) Otolaryngol. Head Neck Surg. 112:639-653; Billings et al. (1995) Ann. Otolol. Rhinol. Larngol. 104:181-188; Gottschlich et al. (1995) Laryngoscope 105:1347-1352). The cumulative data were summarized in a recent report (Harris and Ryan (1995) Otolaryngol. Head Neck Surg. 112:639-653). Of 279 patients from patients with rapidly progressive sensorineural hearing loss, 32% were positive for the 68 kD antigen on Western blots (Harris and Ryan (1995) Otolaryngol. Head Neck Surg. 112:639-653). Hughes *et al.* (Hughes et al. (1994) Am. J. Otology. 15:198-202) reported finding antibodies to a 68 kD antigen in 86% of patients with what they termed idiopathic, progressive, bilateral sensorineural hearing loss (IPBSNHL), whereas Moscicki *et al*. (Moscicki et al. (1994) JAMA 272:611-616) found this type of antibody activity in 58% of patients. These groups also concluded that the presence of antibody predicted a clinical response to treatment with steroids.

The nature of the target antigen of human AISNHL autoimmune sera has been suggested to be heat shock protein 70 (HSP-70) (Billings et al. (1995) Ann. Otolol. Rhinol. Larngol. 104:181-188). This was based upon evidence from ion exchange chromatography, adenosine triphosphate affinity chromatography, and one- and two-dimensional electrophoresis. Additionally, immunoblotting with AISNHL patient sera showed dramatically increased expression of the 68 kD antigen by bovine kidney cells following heat shock in culture.

HSP-70 is a ubiquitous protein that is not specific for the inner-ear. However, reactivity with stress proteins of various classes has been reported in a number of autoimmune diseases as well as non-autoimmune disease states. When cells or organisms are placed under stress, synthesis of HSPs increases presumably to protect critical proteins from denaturation. Thus, antibodies to HSPs may be produced as a result of release of cellular contents of damaged cells under the same stress that leads to formation of antibodies to autoimmune specific antigens. In fact, surveys of patients with other autoimmune diseases and patients with some types of infection also frequently have antibodies to heat shock proteins. In spite of the lack of specificity, HSP-70 is being used in some studies, including a national clinical trial sponsored by the NIH, as a target antigen for assessing AISNHL.

### C. IESCA is Associated with AISNHL In Humans, and

### is not HSP-70

In contrast to the suggestion that HSP-70 is a target antigen of human AISNHL autoimmune sera, the present inventors have discovered that another antigen, referred to as IESCA, is a target antigen of human AISNHL autoimmune sera. Moreover, the present inventors have demonstrated that IESCA and HSP-70 are different proteins. 1. IESCA is a target antigen in human AINSL. In preliminary studies, the present inventors used guinea pig inner ear tissue as the substrate for detection of inner ear reactive antibodies in sera from patients with AISNHL. This selection was made based upon extensive experience with this model (Zajic et al. (1991) Hearing Res. 52:59-72; Ptok et al. (1991) Hearing Res. 57:79-90; Nair et al. (1995) Hearing Res. 83:101-113; Nair et al. (1997) Hearing Res. 107:93-101; Disher et al. (1997) Ann. NY Acad. Sci. 830:253-265), the ability to control precisely the preparation of the inner ear extract, and previous work which has indicated that inner ear reactive antibodies bind to phylogenetically conserved antigens (Harris (1993) In: 2nd edition Otolaryngology Head and Neck Surgery Vol. IV: Ear and Cranial Base (Cummings et al. (eds.) Mosby Yearbook, St. Louis, MO) pp. 2926-2942; and Harris and Ryan (1984) Am. J. Otolaryngol.).

Moreover, as described above, the inventors had prepared a monoclonal antibody, KHRI-3, which bound to an inner ear supporting cell antigen (IESCA) both *in vitro* and *in vivo,* and which when present in animals, resulted in the development of hearing loss in the animals. These results provided strong evidence for antibody-mediated disruption of hearing, and evidence that antibody binding to IESCA might be involved in hearing loss.

Initial experiments (described in more detail in the Examples) indicated that 50-60% of patients clinically assessed as having AISNHL have antibodies that bind to a 68-70 kD protein in guinea pig inner ear extracts. The same sera also stain supporting cells in the organ of Corti with a pattern like that of the KHRI-3 monoclonal antibody. This is the first demonstration that Western blot positive sera from humans with rapidly progressive hearing loss have a reproducible binding pattern in the inner ear (Disher et al. (1997) Ann. NY Acad. Sci. 830:253-265). These initial experiments also demonstrated that a 68-70 kD inner ear protein immunoprecipitated by KHRI-3 is strongly stained by antibodies in AISNHL patients' sera, but not sera from normal control donors. This protein, as described above, is referred to as IESCA (for inner ear supporting cell antigen). Furthermore, human inner ear tissue, but not blood cells from the same donor, absorbs the human serum antibody reactivity to guinea pig inner ear substrate, indicating that the antibodies define a phylogenetically conserved protein expressed in human and guinea pig ears. 2. IESCA and HSP-70 are different proteins. The present inventors have demonstrated that IESCA and HSP-70 are different proteins, even though they appear to have similar molecular weights. The supporting evidence is summarized as follows (and is described in more detail in the Examples). The inventors have shown that antigen purified from patient tissue or guinea pig inner ear with the IESCA specific MAb KHRI-3 does not react with antibodies to HSP-70. Similarly, protein precipitated by HSP-70 antibody does not react with the KHRI-3 MAb. Likewise, anti-HSP-70 does not stain guinea pig organ of Corti supporting cells, as does MAb KHRI-3.

### D. Treatment of AISNHL

Based upon these initial experimental results, the inventors hypothesized that the KHRI-3 monoclonal antibody and human sera from patients with the provisional diagnosis of AISNHL bind to the same antigen, which is referred to as IESCA. The inventors further postulated that human antibodies against this antigen, or IESCA, are likely to be a cause of hearing loss. These hypotheses were supported by other experimental studies undertaken by the inventors (described above) which demonstrated that the KHRI-3 antibody can bind to supporting cells in *vivo,* and when present in *vivo* at high titre or administered in *vivo,* can result in both loss of sensory cells in the organ of Corti and the development of significant hearing loss (Nair, TS et al. (1995) Hear. Res. 83: 101-113; and Nair, TS et al. (1997) Hear. Res. 107: 93-101).

The inventors have now discovered that the presence of antibodies to IESCA in AISNHL patients is predictive of the response of patients to treatment with immunosuppressive therapy; those patients with antibodies are more likely to respond to treatment than those without. This discovery is based upon the results of a prospective study, which demonstrated that patients with antibody to IESCA were statistically significantly more likely to have improvement in hearing after steroid therapy than those without such antibody.

In the study, the presence of antibody to IESCA was determined by one of two methods, Western blotting (where the separated proteins from the patient sera were stained with Mab KHRI-3), and immunofluroescence (where guinea pig organs of Corti were stained by antibody present in the sera). In the patient subset, 67% of the patients were antibody positive by Western blot and 75% were positive by immunofluorescence staining. About half of the patients demonstrated clinical improvement, and about half did not. Of those who were Western blot positive, 53% improved; similarly, 48% of the Western blot negative patients showed hearing improvement (p=0.66). In contrast, 60% of patients who were immunofluroescence positive had hearing improvement, as compared to improvement in only 25% of those who were immunofluorescence negative (p=0.021). In fact, 88% of those who improved were positive for antibody to supporting cells by the immunofluorescence assay, and only 12.1% of those who improved were immunofluroescence negative. The presence of antibody to supporting cells is statistically significantly associated with hearing improvement after steroid therapy (Relative Risk: 2.4). Therefore, patients whose acute phase sera are immunofluorescence (IF) positive are greater than two times more likely to experience improved hearing with steroid treatment than patients whose sera are immunofluorescence negative.

This data provides further support for the importance of the supporting cell antigen in the pathogenesis of autoimmune hearing loss. A subset of the immunofluorescence positive patients fail to improve, since some may have already developed irreversible damage at the time therapy was initiated. It is also likely that those patients who were Western blot negative but immunofluroescence positive and who improved after steroid treatment probably had too low a titer of antibody to be seen on the Western blots, as described in further detail in the Examples.

### E. IESCA As Screening Agent For AISNHL

The presence of IESCA antibodies in patients sera has been shown to have a high correlation with the probable response of a patient to corticosteroid treatment for AISNHL, as described above. Therefore, a diagnostic screen based on the detection of anti-IESCA antibodies would identify these patients while protecting those that do not express anti-IESCA from unnecessary treatment and any associated side effects.
Additionally, the identification of compounds that are agonistic and/or antagonistic to IESCA binding and/or anti-IESCA binding would be advantageous in determining methodologies for the treatment of AISNHL.

### F. Characterization of IESCA

Characterization and identification of IESCA would facilitate the implementation of diagnostic screens based upon the detection of anti-IESCA antibodies, as well as the identification of compounds that are agonistic and/or antagonistic to IESCA binding and/or anti-IESCA binding, as described above. Therefore, the inventors set out to identify this inner ear supporting cell antigen.

Immunoaffinity purified guinea pig inner ear protein was isolated by SDS PAGE and sequenced using tandem mass spectroscopy (MS/MS). Ten peptide sequences were obtained which were identical to sequences in human CTL2, a 706 amino acid member of the choline transporter-like family of proteins (O'Regan, S et al. (2000) Proc Natl Acad Sci 97:1835-1840). The nucleic acid sequence encoding human CTL2 (hCTL2) is shown in Figure 9 (SEQ ID NO: 1), and the predicted amino acid sequence of hCTL2 is shown in Figure 10 (SEQ ID NO:2).

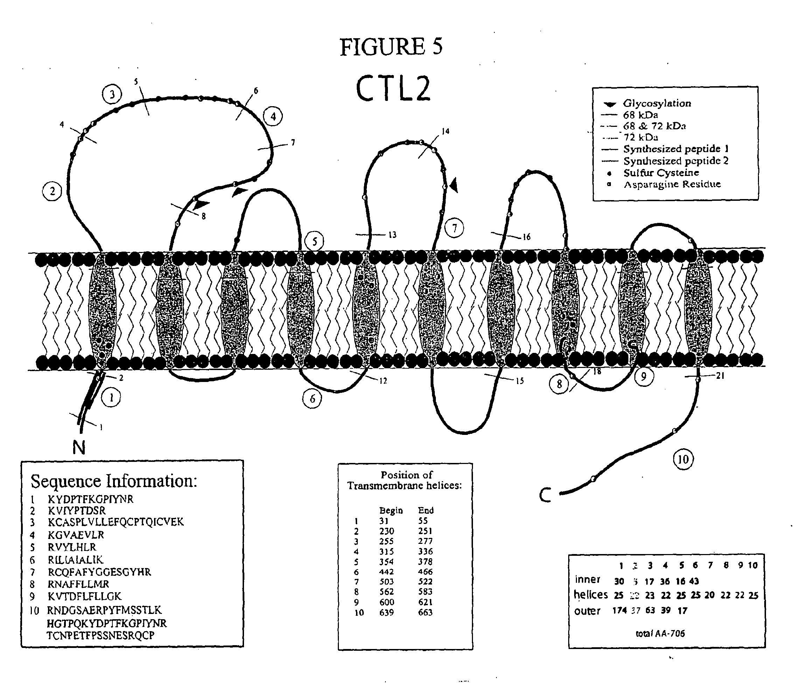

The function of CTL2 is not known. Presumably, as a member of the family of choline transporter-like proteins, it could transport choline. O'Regan et al. ((2000) Proc Natl Acad Sci 97:1835-1840) cloned CTL1 using cDNA from the electric organ of the torpedo fish to complement a yeast strain deficient in choline transport. Using CTL1 as a probe, three other members of this family were found, CTL2, CTL3 and CTL4 (O'Regan et al. (2000) Proc Natl Acad Sci 97:1835-1840). However, there are no reports that CTL2 been studied for function. It may, like CTL1 serve as a choline transporter. Choline is required for biosynthesis of acetylcholine, which is a primary neurotransmitter in cholinergic nerve terminals. Choline is also required for biosynthesis of phosphatidylcholine, a major component of membranes. Human CTL2 is predicted to contain ten membrane-spanning regions, with both the N and C terminus residing on the cytoplasmic side of the cell membrane (as shown in Figure 5). Such a structure is consistent with a transporter function.

Rabbit antibodies raised to a synthetic peptide based on a conserved antigenic peptide of CTL2 stained inner ear supporting cells with the same pattern as KHRI-3, and strongly stained human vestibular supporting cells with a pattern quite similar to that obtained with KHRI-3 on guinea pig vestibular tissue. CTL2 and KHRI-3 antibodies both identify the same 68 and 72kDa bands in immunoprecipitation and western blot experiments. Note that the currently identified molecular weight of 72kDa for IESCA is the same as the previously identified molecular weight of 70kDa described above; the difference is due to a change in calibration of the commercially available molecular weight standards.

RT-PCR using RNA from human inner ear tissue and guinea pig inner ear demonstrated that full length CTL2 mRNA is expressed in guinea pig and human inner ear tissue, as well as in a number of human cell lines. The guinea pig CTL2 is 72% homologous to the protein encoded by human cDNA. These results show that the inner ear supporting cell antigen (IESCA) defined by KHRI-3 is the guinea pig homologue of hCTL2, suggesting that a possible mechanism of action of the KHRI-3 antibody is blockade of the transporter function of this molecule. Sera from patients suspected to have autoimmune hearing loss bound to the antigenic peptide corresponding to the N terminal domain of CTL2 (exons 1-3) in ELISA assays. Thus, the CTL2 protein in the human inner ear is a candidate antigenic target in autoimmune hearing loss in humans.

The overall sequence in guinea pigs and humans diverges, as might be expected of species that are evolutionarily distant from one another, but for the most part the sequences are highly homologous, suggesting that the function remains the same in both species. Several lines of evidence demonstrate that CTL2 is as abundantly expressed in the human inner ear as is the guinea pig homologue identified by KHRI-3. First, there is sufficient messenger RNA in both the guinea pig cochlea and the human vestibular tissue to amplify the full sequence. Additionally, antibody to the N-terminal domain of CTL2 strongly stains the supporting cells in the cochlea of the guinea pig and the vestibular system of the human. Furthermore, the pattern of CTL2 expression in the human ampulla is the same as that previously observed in the guinea pig vestibular system using the KHRI-3 antibody (Ptok, M. et al. (1993a) Hear. Res. 66, 245-252). The CTL2 protein is predicted to be a membrane protein, and the immunofluorescence studies are consistent with this prediction.

In prior studies of the expression of the IESCA in guinea pig inner ears that had been infused with purified KHRI-3 antibody, the inventors observed a redistribution of the antigen in regions of scar formation and hair cell loss (Nair TS et al. (1999) Hearing Res 129:50-60). Presumably, as the supporting cells form scars to seal gaps left in the reticular lamina of the organ of Corti left by the involution of damaged hair cells, they must synthesize new membrane. This redistribution of IESCA is consistent with CTL2 having a role in choline transport for the purpose of membrane synthesis. It is therefore contemplated that CTL2 does function as a choline transporter.

The KHRI-3 antibody binds to an N-linked carbohydrate moiety on the extracellular loops. It is predicted, from the model of CTL2 shown in Figure 5, that these carbohydrates are linked to arginine residues N187 and N200 on extracellular loop one and/or N417 on extracellular loop three. There are actually four predicted N glycosylation sites in the molecule but the fourth, N697, is in the C-terminal domain, which the model predicts to be in the cytoplasm. The inventors' previous *in vivo* studies of KHRI-3 infusion into the cochlea have shown that binding of KHRI-3 to these targets leads to hair cell loss and ultimately hearing loss. Although it is not necessary to understand the mechanism to practice the invention, and the invention is not limited to any particular hypothesis, it is hypothesized that the antibody blocks the function of the antigenic protein, perhaps by sterically hindering transport of choline or some other molecule transported by CTL2. The hypothesis that human antibodies binding to the same molecule might result in damage to the human inner ear and could be a cause of autoimmune hearing loss is also consistent with these results. On the other hand, the inventors have also demonstrated that CTL2 is part of a multimolecular complex, as is described in more detail below, and that antibodies to other members of this complex might also result in similar *in vivo* staining patterns and damage to the inner ear and to hearing.

There is a discrepancy in the predicted size of the CTL2 protein and the actual size of the protein precipitated by either the CTL2 or KHRI-3 antibodies. The predicted CTL2 protein of 706 amino acids is expected to be 80 kDa. However, the actual mass of the protein doublet routinely observed by the inventors is 68 kDa and70 kDa (in the older sizing) or 68 kDa and 72 kDA (in the newer sizing), and this doublet contains carbohydrate modifications. Thus, the size discrepancy and the presence of glycosylation suggests that there are posttranslational modifications of newly synthesized CTL2 to become the mature protein that is inserted into the membrane. In this regard, it was observed by the inventors that an 80 kDa protein band was immunoprecipitated by the CTL2 antiserum, but not by KHRI-3. It is postulated that the 80 kDa protein is the nascent protein, which is then cleaved and glycosylated to form the final membrane embedded form.

### G. CTL2 is Part of a Multimolecular Complex

The inventors have discovered that IESCA is part of a multimolecular complex. At least one other protein is co-precipitated with IESCA during immunoprecipitation with Mab KHRI-3.

Another prominent 60 kDa protein was observed to co-precipitate with IESCA by both immunoprecipitation and affinity column purification. This protein band was also cut out and sent for sequencing; the results indicated that the protein is cochlin. Cochlin is the protein product of the COCH-5B2 gene, which is also the target gene of DFNA9, a congenital hearing loss locus on human chromosome 14. Individuals affected by a mutation in COCH-5B2 develop progressive hearing loss (Manolis, EN et al. (1996) Hum. Mol. Gen. 1996, Vol. 5, No. 7, 1047-1050; Nahid G et al. (1997) Genomics 46, 345-354. 1997. Art. No. GE975067; Nahid G et al. (1998) Nat. Gen. Vol. 20; Heller, S et al. (1998) Proc. Natl. Acad. Sci. USA. Vol. 95, pp. 11400-11405, Neurobiology; Yvette J. M. et al. (1999) Hum Mol Gen, Vol. 8, No. 2, 361-366; and Erik Fransen, E et al. (1999) Hum Mol Gen, Vol. 8, No.8 1425-1429).

Other investigators have reported that sera from patients with rapidly progressive sensorineural hearing loss (SNHL) or Meniere's disease react with a guinea pig or bovine inner ear protein of approximately 58 kDa in Western blot analysis (as summarized by Boulassel et al. (2001) Otol Neurol 22:614-618). One of these groups recently identified this protein as the COCH5B2 protein (Boulassel et al. (2001) Otol Neurol 22:614-618). They report that the exact function of this protein remains to be determined, but report that it is proposed to have a structural role. This proposal is based upon characteristics of the protein, which include the presence of a predicted signal peptide and two regions of homology to von Willebrand factor A domains, which are known to be involved in various functions, and whose presence in a protein appears invariably associated with secretion of the protein. Thus, it has been suggested that the COCH5B2 protein interacts via type A domains with the abundant fibrillar collagens for ECM assembly in the cochlea.

The inventors undertook several experiments to further examine the expression of these proteins in the inner ear, and to further characterize the relationship between the KHRI-3 antigen and the proteins identified by MS/MS sequencing. To that end, rabbit antisera were raised against highly antigenic regions of cochlin, by the methods as described elsewhere for CTL2. These antibodies were used to precipitate proteins from guinea pig inner ear extracts, to stain human and guinea pig inner ear tissue, and to identify the protein bands on western blots of proteins immunoprecipitated by KHRI-3, by the methods as described elsewhere for CTL2. The immunofluorescence analysis demonstrated that CTL2 and cochlin have identical distribution patterns on the surface of supporting cells in the cochlea.

Subsequently, in the course of developing a high throughput western blot screening assay for testing sera of patients suspected of autoimmune hearing loss, the inventors noted that some patients' sera produce a staining pattern against inner ear extracts that is remarkably similar to the banding pattern produced by the rabbit anti-cochlin antisera. Based upon these results, together with the results described above for the behavior of AISNHL patient sera with respect to IESCA, it is contemplated that patients with autoimmune hearing loss have antibodies to inner ear antigens that are either identical to or at least are co-expressed with IESCA and cochlin. Based upon the co-precipitation of cochlin with CTL-2 by both immunoprecipitation and affinity column purification, and the similarities of the staining patterns of AISNHL patient sera against inner ear extracts and the banding patterns produced by rabbit anti-cochlin, it is contemplated that cochlin and CTL2 are part of a macromolecular complex. It is further contemplated that this complex has a probable transporter function in the inner ear that is the target of autoimmune attack, and that antibodies to these proteins can cause hearing loss.

### II. CTL2 Nucleic Acid and Amino Acid Sequences

The present invention provides the identification of IESCA as CTL2, where IESCA is contemplated to be a mature form of CTL2. The maturation of CTL2 is contemplated to involve glycosylation and possibly also shortening of the polypeptide sequence. The present invention also identifies a role of IESCA in hearing function. Therefore, the present invention provides a nucleic acid sequence encoding a protein which has a role in hearing, where the sequence encodes CTL2. Exemplary sequences include SEQ ID NO:1 as shown in Figure 9, which encodes human CTL2, and SEQ ID NO:3 as shown in Figure 11, which encodes guinea pig CTL2.

The present invention also provides an amino acid sequence of a protein which has a role in hearing, where the protein is IESCA. IESCA is a glycoprotein from the inner-ear organ of Corti which is reactive with an antibody associated with autoimmune sensorineural hearing loss, and which is a doublet when separated by SDS-PAGE, with apparent molecular weights of about 68,000 and 72,000 daltons under reducing conditions, and with apparent molecular weights of about 65,000 and about 68,000 daltons under non-reducing conditions. Examples of antibodies include KHRI-3. Sequencing by tandem mass spectroscopy of immunoaffinity purified IESCA isolated by SDS-PAGE identified IESCA as a CTL2, as described above. Because IESCA has a lower molecular weight than that predicted for CTL2, and is a glycoprotein, it is contemplated that IESCA is a mature form of CTL2, where the newly synthesized CTL2 undergoes post-translational modification, including glycosylation. Exemplary sequences include the amino acid sequence of a mature or nascent form of IESCA (SEQ ID NO:2 or SEQ ID NO:4) as shown in Figures 10 and 12.

### III. Use of CTL2 Nucleic Acid and Amino Acid Sequences

### A. Predicting response of AISNHL patients to treatment.

The present invention also relates to a variety of methods of testing patients suspected of having AISNHL, and of those patients who will most likely respond to steroid treatment. In some methods, guinea pig (or other suitable animal) organ of Corti (or other suitable tissue) is exposed to a patient's sera followed by immunofluorescent staining by methods known to those in the field. A positive result comprises a distinctive staining pattern of "wine glass" shapes on the organ of Corti (or other distinguishable pattern depending on the tissue used). Staining with MAb KHRI-3, or other suitable antibody, serves as a positive control. The invention is not limited by the tissue used or the animal from which it was derived, so long as when the tissue is stained with antibody that recognizes IESCA, the staining is distinctive. The invention is not limited by the antibody used as a positive control, so long as the antibody is specific for IESCA or an epitope of IESCA.

Another method contemplates providing purified IESCA from source tissue and determining if sera from a patient suspected of having AISNHL will react with the antigen. The present invention is not limited by the particular method of screening, or by the particular source of IESCA. One embodiment comprises providing purified IESCA, and Western blotting precipitated IESCA with the patient's sera. Another embodiment comprises making tissue extracts from guinea pig organ of Corti, followed by immunoprecipitation of IESCA with monoclonal antibody KHRI-3, or other suitable antibody, and Western blotting precipitated IESCA with patients' sera. A positive result in either embodiment comprises recognition of the precipitated IESCA with the patient's sera. Staining with MAb KHRI-3, or other suitable antibody, may serve as a positive control. The invention is not limited by the tissue used to acquire IESCA, or the animal from which it was derived, so long as the isolated protein stains with antibody that recognizes IESCA. IESCA can be naturally occurring, or obtained from transgenic cells or organisms. The invention is not limited by the antibody used to precipitate the IESCA, or used as a positive control, so long as the antibody is specific for IESCA or an epitope of IESCA (which can be confirmed in competition assays or preclearing assays with MAb KHRI-3).

The presence of auto-antibodies to IESCA is associated with AISNHL, and is associated with hearing improvement after steroid treatment.

### B. Drug Screening Using IESCA

The present invention relates to methods and compositions for using IESCA and anti-IESCA antibodies ("anti-IESCA") as a target for screening drugs that can alter, for example, IESCA and anti-IESCA binding (*e.g*., and thus alleviate AISNHL symptoms). For example, drugs that induce or inhibit anti-IESCA binding to IESCA can be identified by screening for compounds that bind to either IESCA or anti-IESCA, or that affect the binding of the antigen to the antibody.

The present invention is not limited to a particular mechanism of action. Indeed, an understanding of the mechanism of action is not necessary to practice the present invention. Nevertheless, it is contemplated that the presence of antibodies to IESCA results in AISNHL. Thus, it is contemplated that drugs which inhibit the binding of the antibody to IESCA can be used to prevent symptoms of AISNHL.

In one screening method, candidate compounds are evaluated for their ability to alter anti-IESCA binding to IESCA by adding the compound to either the antibody or to the antigen, then mixing the antibody (plus compound, if present) and the antigen (plus compound, if present), and then measuring the effects of the compound on the binding of the antibody to the antigen. In another screening method, candidate compounds are evaluated for their ability to disrupt anti-IESCA binding to IESCA by adding the compound to a mixture of the antibody and antigen, and then measuring the effects of the compound on the binding of the antibody to the antigen.

The present invention relates to many other means of screening compounds. The examples provided above are presented merely to illustrate a range of techniques available. One of ordinary skill in the art will appreciate that many other screening methods can be used.

In particular, the present invention relates to the use of cell lines transfected with *CTL2* and variants thereof for screening compounds for activity, and in particular to high throughput screening of compounds from combinatorial libraries (*e.g*., libraries containing greater than 10⁴ compounds). The cell lines of the present invention can be used in a variety of screening methods.

### C. Detecting alleles and genetic lesions resulting in hearing damage or loss.

It is contemplated that IESCA is involved in hearing, based upon the evidence described above. For example, the presence of autoantibodies against IESCSA and/or a complex of which IESCA and cochlin are members results in damage to hearing. Therefore, it is contemplated that the absence of expression of IESCA or a defect in protein results in hearing damage or loss. Thus, it is contemplated that the nucleic acid sequence encoding CTL2, and the amino acid sequence encoding CTL2, are useful in detecting genetic lesions resulting loss. Moreover, it is contemplated that genetic therapy might be useful to restore a functional IESCA protein, and to prevent and/or reverse such hearing loss or damage.

Therefore, the present invention relates to alleles of CTL2 that predict a patient's likelihood of suffering hearing loss or damage. It is contemplated that analysis of naturally occurring human CTL2 alleles reveal that patients with increased susceptibility to hearing loss or damage have a mutant CTL2 allele. Mutant CTL2 alleles result in the loss of IESCA or a functional IESC A. It is contemplated that a non-functional IESCA results from many types of mutations, including but not limited to the absence of a cleavage site, resulting in the loss or processing of CTL2, or in a modification of the amino acid residues which are glycosylated, which result in different glycosylation patterns or no glycosylation. Any mutation that results in the undesired phenotype (*e.g.*, progressive damage to or loss of hearing) is within the scope of the present invention. Assays for determining the presence of a mature IESCA protein are described in the Examples, and in particular in Examples 4 and 5.

The present invention is not limited to a particular mechanism of action. Indeed, an understanding of the mechanism of action is not necessary to practice the present invention. Nevertheless, it is contemplated that the IESCA is a mature form of CTL2, and involved in choline transport or phosphatidyl choline synthersis and membrane synthesis or function. The loss of IESCA or a functional IESCA leads to the damage to or loss of hearing.

Accordingly, the present invention provides methods for determining whether a patient has an increased susceptibility to hearing damage or loss by determining whether the individual has a variant CTL2 gene. In other embodiments, the present invention provides methods for providing a prognosis of increased risk for hearing loss or damage to an individual based on the presence or absence of one or more variant alleles of CTL2.

A number of methods are available for analysis of variant (*e.g*., mutant or polymorphic) nucleic acid sequences. Assays for detections variants (*e.g*., polymorphisms or mutations) fall into several categories, including, but not limited to direct sequencing assays, fragment polymorphism assays, hybridization assays, and computer based data analysis. Protocols and commercially available kits or services for performing multiple variations of these assays are available. In some embodiments, assays are performed in combination or in hybrid (*e.g*., different reagents or technologies from several assays are combined to yield one assay). The following assays are useful in the present invention.

### 1. Direct sequencing Assays

In some methods, variant sequences are detected using a direct sequencing technique. In these assays, DNA samples are first isolated from a subject using any suitable method. The region of interest is cloned into a suitable vector and amplified by growth in a host cell (*e.g.,* a bacteria) or DNA in the region of interest is amplified using PCR.

Following amplification, DNA in the region of interest (*e.g*., the region containing the mutation of interest) is sequenced using any suitable method, including but not limited to manual sequencing using radioactive marker nucleotides, or automated sequencing. The results of the sequencing are displayed using any suitable method. The sequence is examined and the presence or absence of a given mutation is determined.

### 2. PCR Assay

In some methods, variant sequences are detected using a PCR-based assay. The PCR assay comprises the use of oligonucleotide primers that hybridize only to the variant or wild type allele of CTL2 (*e.g*., to the region of polymorphism or mutation). Both sets of primers are used to amplify a sample of DNA. If only the mutant primers result in a PCR product, then the patient has the mutant CTL2 allele. If only the wild-type primers result in a PCR product, then the patient has the wild type allele of CTL2.

### 3. Fragment Length Polymorphism Assays

In some methods, a variant sequence is detected using a fragment length polymorphism assay. In a fragment length polymorphism assay, a unique DNA banding pattern based on cleaving the DNA at a series of positions is generated using an enzyme. DNA fragments from a sample containing a mutation will have a different banding pattern than wild type.

### a. RFLP Assay

In some methods, variant sequences are detected using a restriction fragment length polymorphism assay (RFLP). The region of interest is first isolated using PCR. The PCR products are then cleaved with restriction enzymes known to give a unique length fragment for a given polymorphism. The restriction-enzyme digested PCR products are separated by agarose gel electrophoresis and visualized by ethidium bromide staining. The length of the fragments is compared to molecular weight markers and fragments generated from wild-type and mutant controls.

### b. CFLP Assay

In other methods, variant sequences are detected using a CLEAVASE fragment length polymorphism assay (CFLP; Third Wave Technologies, Madison, WI; *See e.g.,* U.S. Patent Nos. 5,843,654; 5,843,669; 5,719,208; and 5,888,780). This assay is based on the observation that when single strands of DNA fold on themselves, they assume higher order structures that are highly individual to the precise sequence of the DNA molecule. These secondary structures involve partially duplexed regions of DNA such that single stranded regions are juxtaposed with double stranded DNA hairpins. The CLEAVASE I enzyme, is a structure-specific, thermostable nuclease that recognizes and cleaves the junctions between these single-stranded and double-stranded regions.

The region of interest is first isolated, for example, using PCR. Then DNA strands are separated by heating. Next, the reactions are cooled to allow intrastrand secondary structure to form. The PCR products are then treated with the CLEAVASE I enzyme to generate a series of fragments that are unique to a given mutation. The CLEAVASE enzyme treated PCR products are separated and detected (*e.g*., by agarose gel electrophoresis) and visualized (*e.g*., by ethidium bromide staining). The length of the fragments is compared to molecular weight markers and fragments generated from wild-type and mutant controls.

### 4. Hybridization Assays

In other methods variant sequences are detected a hybridization assay. In a hybridization assay, the presence or absence of a given mutation is determined based on the ability of the DNA from the sample to hybridize to a complementary DNA molecule (*e.g*., a oligonucleotide probe). A variety of hybridization assays using a variety of technologies for hybridization and detection are available. A description of a selection of assays is provided below.

### a. Direct Detection of Hybridization

In some methods, hybridization of a probe to the sequence of interest (*e.g.,* a mutation) is detected directly by visualizing a bound probe (*e.g*., a Northern or Southern assay; *See e.g.,* Ausabel et al. (eds.), Current Protocols in Molecular Biology, John Wiley & Sons, NY [1991]). In a these assays, genomic DNA (Southern) or RNA (Northern) is isolated from a subject. The DNA or RNA is then cleaved with a series of restriction enzymes that cleave infrequently in the genome and not near any of the markers being assayed. The DNA or RNA is then separated (*e.g*., on an agarose gel) and transferred to a membrane. A labelled (*e.g.*, by incorporating a radionucleotide) probe or probes specific for the mutation being detected is allowed to contact the membrane under a condition or low, medium, or high stringency conditions. Unbound probe is removed and the presence of binding is detected by visualizing the labelled probe.

### b. Detection of Hybridization Using "DNA Chip" Assays

In other methods, variant sequences are detected using a DNA chip hybridization assay. In this assay, a series of oligonucleotide probes are affixed to a solid support. The oligonucleotide probes are designed to be unique to a given mutation. The DNA sample of interest is contacted with the DNA "chip" and hybridization is detected.

In some embodiments, the DNA chip assay is a GeneChip (Affymetrix, Santa Clara, CA; *See e.g.,* U.S. Patent Nos. 6,045,996; 5,925,525; and 5,858,659) assay. The GeneChip technology uses miniaturized, high-density arrays of oligonucleotide probes affixed to a "chip." Probe arrays are manufactured by Affymetrix's light-directed chemical synthesis process, which combines solid-phase chemical synthesis with photolithographic fabrication techniques employed in the semiconductor industry. Using a series of photolithographic masks to define chip exposure sites, followed by specific chemical synthesis steps, the process constructs high-density arrays of oligonucleotides, with each probe in a predefined position in the array. Multiple probe arrays are synthesized simultaneously on a large glass wafer. The wafers are then diced, and individual probe arrays are packaged in injection-molded plastic cartridges, which protect them from the environment and serve as chambers for hybridization.

The nucleic acid to be analyzed is isolated, amplified by PCR, and labeled with a fluorescent reporter group. The labeled DNA is then incubated with the array using a fluidics station. The array is then inserted into the scanner, where patterns of hybridization are detected. The hybridization data are collected as light emitted from the fluorescent reporter groups already incorporated into the target, which is bound to the probe array. Probes that perfectly match the target generally produce stronger signals than those that have mismatches. Since the sequence and position of each probe on the array are known, by complementarity, the identity of the target nucleic acid applied to the probe array can be determined.

In other methods, a DNA microchip containing electronically captured probes (Nanogen, San Diego, CA) is utilized (*See e.g.,* U.S. Patent Nos. 6,017,696; 6,068,818; and 6,051,380). Through the use of microelectronics, Nanogen's technology enables the active movement and concentration of charged molecules to and from designated test sites on its semiconductor microchip. DNA capture probes unique to a given mutation are electronically placed at, or "addressed" to, specific sites on the microchip. Since DNA has a strong negative charge, it can be electronically moved to an area of positive charge.

First, a test site or a row of test sites on the microchip is electronically activated with a positive charge. Next, a solution containing the DNA probes is introduced onto the microchip. The negatively charged probes rapidly move to the positively charged sites, where they concentrate and are chemically bound to a site on the microchip. The microchip is then washed and another solution of distinct DNA probes is added until the array of specifically bound DNA probes is complete.

A test sample is then analyzed for the presence of target DNA molecules by determining which of the DNA capture probes hybridize, with complementary DNA in the test sample (*e.g*., a PCR amplified gene of interest). An electronic charge is also used to move and concentrate target molecules to one or more test sites on the microchip. The electronic concentration of sample DNA at each test site promotes rapid hybridization of sample DNA with complementary capture probes (hybridization may occur in minutes). To remove any unbound or nonspecifically bound DNA from each site, the polarity or charge of the site is reversed to negative, thereby forcing any unbound or nonspecifically bound DNA back into solution away from the capture probes. A laser-based fluorescence scanner is used to detect binding.

In still further methods an array technology based upon the segregation of fluids on a flat surface (chip) by differences in surface tension (ProtoGene, Palo Alto, CA) is utilized (*See e.g.,* U.S. Patent Nos. 6,001,311; 5,985,551; and 5,474,796; ). Protogene's technology is based on the fact that fluids can be segregated on a flat surface by differences in surface tension that have been imparted by chemical coatings. Once so segregated, oligonucleotide probes are synthesized directly on the chip by ink-jet printing of reagents. The array with its reaction sites defined by surface tension is mounted on a X/Y translation stage under a set of four piezoelectric nozzles, one for each of the four standard DNA bases. The translation stage moves along each of the rows of the array and the appropriate reagent is delivered to each of the reaction site. For example, the A amidite is delivered only to the sites where amidite A is to be coupled during that synthesis step and so on. Common reagents and washes are delivered by flooding the entire surface and then removing them by spinning.

DNA probes unique for the mutation of interest are affixed to the chip using Protogene's technology. The chip is then contacted with the PCR-amplified genes of interest. Following hybridization, unbound DNA is removed and hybridization is detected using any suitable method (e.g., by fluorescence de-quenching of an incorporated fluorescent group).

In yet other methods, a "bead array" is used for the detection of polymorphisms (Illumina, San Diego, CA; *See e.g.,* PCT Publications WO 99/67641 and WO 00/39587). Illumina uses a BEAD ARRAY technology that combines fiber optic bundles and beads that self-assemble into an array. Each fiber optic bundle contains thousands to millions of individual fibers depending on the diameter of the bundle. The beads are coated with an oligonucleotide specific for the detection of a given mutation. Batches of beads are combined to form a pool specific to the array. To perform an assay, the BEAD ARRAY is contacted with a prepared subject sample (*e.g*., DNA). Hybridization is detected using any suitable method.

### c. Enzymatic Detection of Hybridization

In some methods, genomic profiles are generated using a assay that detects hybridization by enzymatic cleavage of specific structures (INVADER assay, Third Wave Technologies; *See e.g.,* U.S. Patent Nos. 5,846,717; 6,090,543; 6,001,567; 5,985,557; and 5,994,069). The INVADER assay detects specific DNA and RNA sequences by using structure-specific enzymes to cleave a complex formed by the hybridization of overlapping oligonucleotide probes. Elevated temperature and an excess of one of the probes enable multiple probes to be cleaved for each target sequence present without temperature cycling. These cleaved probes then direct cleavage of a second labeled probe. The secondary probe oligonucleotide can be 5'-end labeled with fluorescein that is quenched by an internal dye. Upon cleavage, the de-quenched fluorescein labeled product may be detected using a standard fluorescence plate reader.

The INVADER assay detects specific mutations in unamplified genomic DNA. The isolated DNA sample is contacted with the first probe specific either for a mutation or wild type sequence and allowed to hybridize. Then a secondary probe, specific to the first probe, and containing the fluorescein label, is hybridized and the enzyme is added. Binding is detected by using a fluorescent plate reader and comparing the signal of the test sample to known positive and negative controls.

In some methods, hybridization of a bound probe is detected using a TaqMan assay (PE Biosystems, Foster City, CA; *See e.g.,* U.S. Patent Nos. 5,962,233 and 5,538,848). The assay is performed during a PCR reaction. The TaqMan assay exploits the 5'-3' exonuclease activity of the AMPLITAQ GOLD DNA polymerase. A probe, specific for a given allele or mutation, is included in the PCR reaction. The probe consists of an oligonucleotide with a 5'-reporter dye (*e.g.,* a fluorescent dye) and a 3'-quencher dye. During PCR, if the probe is bound to its target, the 5'-3' nucleolytic activity of the AMPLITAQ GOLD polymerase cleaves the probe between the reporter and the quencher dye. The separation of the reporter dye from the quencher dye results in an increase of fluorescence. The signal accumulates with each cycle of PCR and can be monitored with a fluorimeter.

In still further methods, polymorphisms are detected using the SNP-IT primer extension assay (Orchid Biosciences, Princeton, NJ; *See e.g.,* U.S. Patent Nos. 5,952,174 and 5,919,626). In this assay, SNPs are identified by using a specially synthesized DNA primer and a DNA polymerase to selectively extend the DNA chain by one base at the suspected SNP location. DNA in the region of interest is amplified and denatured. Polymerase reactions are then performed using miniaturized systems called microfluidics. Detection is accomplished by adding a label to the nucleotide suspected of being at the SNP or mutation location. Incorporation of the label into the DNA can be detected by any suitable method (*e.g*., if the nucleotide contains a biotin label, detection is via a fluorescently labelled antibody specific for biotin).

### 5. Mass Spectroscopy Assay

In some methods, a MassARRAY system (Sequenom, San Diego, CA.) is used to detect variant sequences (*See e.g.,* U.S. Patent Nos. 6,043,031; 5,777,324; and 5,605,798). DNA is isolated from blood samples using standard procedures. Next, specific DNA regions containing the mutation of interest, about 200 base pairs in length, are amplified by PCR. The amplified fragments are then attached by one strand to a solid surface and the non-immobilized strands are removed by standard denaturation and washing. The remaining immobilized single strand then serves as a template for automated enzymatic reactions that produce genotype specific diagnostic products.

Very small quantities of the enzymatic products, typically five to ten nanoliters, are then transferred to a SpectroCHIP array for subsequent automated analysis with the SpectroREADER mass spectrometer. Each spot is preloaded with light absorbing crystals that form a matrix with the dispensed diagnostic product. The MassARRAY system uses MALDI-TOF (Matrix Assisted Laser Desorption Ionization - Time of Flight) mass spectrometry. In a process known as desorption, the matrix is hit with a pulse from a laser beam. Energy from the laser beam is transferred to the matrix and it is vaporized resulting in a small amount of the diagnostic product being expelled into a flight tube. As the diagnostic product is charged when an electrical field pulse is subsequently applied to the tube they are launched down the flight tube towards a detector. The time between application of the electrical field pulse and collision of the diagnostic product with the detector is referred to as the time of flight. This is a very precise measure of the product's molecular weight, as a molecule's mass correlates directly with time of flight with smaller molecules flying faster than larger molecules. The entire assay is completed in less than one thousandth of a second, enabling samples to be analyzed in a total of 3-5 second including repetitive data collection. The SpectroTYPER software then calculates, records, compares and reports the genotypes at the rate of three seconds per sample.

### 6. Variant Analysis by Differential Antibody Binding

In other methods, antibodies are used to determine if an individual contains an allele encoding a variant CTL2 gene. In some methods, antibodies are utilized that discriminate between mutant (*e.g.*, non-glycosylated proteins, or incorrectly processed proteins) and wild-type proteins.

### 7. Kits for Analyzing Risk of Hearing Loss or Damage

The present invention also relates to kits for determining whether an individual contains a wild-type or variant (*e.g*., polymorphic or mutant) allele of CTL2. The kits are useful determining whether the subject is at risk of developing hearing loss or damage. The diagnostic kits are produced in a variety of ways. The kits can contain at least one reagent for specifically detecting a mutant CTL2 allele or protein. For example, the reagent is a nucleic acid that hybridizes to nucleic acids containing the mutation and that does not bind to nucleic acids that do not contain the mutation or the reagents are primers for amplifying the region of DNA containing the mutation or the reagents are antibodies which preferentially bind either the wild-type or truncated CTL2 proteins. In some embodiments, the kit contains instructions for determining whether the subject is at risk for developing hearing loss or damage. In some embodiments, the kits include ancillary reagents such as buffering agents, nucleic acid stabilizing reagents, protein stabilizing reagents, and signal producing systems (*e.g*., florescence generating systems as Fret systems). The test kit may be packaged in any suitable manner, typically with the elements in a single container or various containers as necessary along with a sheet of instructions for carrying out the test. In some embodiments, the kits also preferably include a positive control sample.

### D. Discovering other proteins involved in hearing damage or loss

It is contemplated that IESCA is part of a multi-molecular complex which comprises IESCA and cochlin. Autoantibodies to members of this complex are associated with hearing damage and/or loss. Therefore, it is contemplated that use of antibodies to either IESCA or cochlin can be used to immunoprecipitate other members of this complex. These members are contemplated to be involved in hearing, such that the present of auto-antibodies to these members result in damage to and/or loss of hearing. Use of these other members of the complex then allows detection of other genetic lesions which result in damage to and/or loss of hearing. Thus, these other members can be sequenced and the amino acid sequences used to determine the coding sequences and genes. Lesions in these genes can then be identified as described above. Exemplary antibodies include KHRI-3, and those present in rabbit antisera to CTL2, including R228 and R229 as described in Example 5.

It is also contemplated that other members of this complex can be identified by using the yeast two-hybrid system to identify binding partners (Chien *et al.* (1991); Fields and Song (1989) Nature 1989 Jul 20; 340(6230): 245-6). In this method, a protein fused to a DNA-binding domain (the bait) and a protein fused to an activation domain (the prey) are expressed in two different haploid yeast strains of opposite mating type (MATa and MATα), and the strains are mated to determine if the two proteins interact. Mating occurs when haploid yeast strains of opposite mating type come into contact, and results in fusion of the two haploids to form a diploid yeast strain. If the prey protein binds to the bait protein, a fully functional transcriptional activator is formed, allowing transcription of a reporter gene under control of a promoter requiring the presence of the activator. Thus, a protein binding interaction can be determined by measuring activation of a two hybrid reporter gene in the diploid strain. In these experiments, the bait protein is IESCA, and the prey protein a candidate member of the IESCA/cochlin multi-molecular complex.

### IV. Methods of Treating Hearing Loss or Damage

### A. Treatment of AISNHL

The present invention relates to methods of treating AISNHL, where the first step is determining whether a patient experiencing hearing damage or loss has serum antibodies to IESCA. The presence of antibodies indicates a generally favorable response to treatment with steroids; thus, patients with anti-IESCA antibodies in their serum are treated with steroids.

Methods of determining whether a patient has AISNHL include mixing a patient's serum sample with purified IESCA, and detecting antibody binding to IESCA by Western blotting, as described in the methods. The source of IESCA includes other animals, such as guinea pig; other sources include recombinant IESCA, produced by well known methods. Other methods for detecting antibody binding to a protein are well known and also useful.

Patients who test positive for the presence of autoantibodies against IESCA are then treated based upon the physician's clinical judgment. Some steroid treatment regimes consist of prednisone at 1mg per kilogram body weight per day (maximum of 60 mg) for a minimal period of 7 days, followed by a tapering schedule. Patients who demonstrate an initial improvement in hearing that deteriorated as the steroid dosage tapered are maintained on steroids until successful weaning is possible. Other treatment regimes consist of Medrol® dose packs (methylprednisolone 24 mg as a loading dose that was tapered by 4 milligrams daily for 6 days). Those patients who demonstrate sufficient improvement are not treated further. Patients who do not demonstrate sufficient improvement receive additional treatment with prednisone (60 mg per day). Some patients receive 60mg/day for seven days followed by a rapid taper, and other patients receive a 30 day burst at 60mg/day followed by a slow taper.

### B. Gene Therapy Using CTL2

The present invention also relates to methods of treating, hearing damage or loss due to mutations present in CTL2 genes. Thus, the invention relates to methods and compositions suitable for gene therapy to alter CTL2 expression, production, or function. As described above, the present invention provides CTL2 genes. Thus, the methods described below are generally applicable. For example the gene therapy is performed by providing a subject with a wild-type allele of CTL2 (*i.e*., an allele that does not contain a mutation which results in hearing damage or loss). Subjects in need of such therapy are identified by the methods described above.

Viral vectors commonly used for *in vivo* or *ex vivo* targeting and therapy procedures are DNA-based vectors and retroviral vectors. Methods for constructing and using viral vectors are known in the art *(See e.g.* (1992) Miller and Rosman, BioTech., 7:980-990). Preferably, the viral vectors are replication defective, that is, they are unable to replicate autonomously in the target cell. In general, the genome of the replication defective viral vectors that are used within the scope of the present invention lack at least one region that is necessary for the replication of the virus in the infected cell. These regions can either be eliminated (in whole or in part), or be rendered non-functional by any technique known to a person skilled in the art. These techniques include the total removal, substitution (by other sequences, in particular by the inserted nucleic acid), partial deletion or addition of one or more bases to an essential (for replication) region. Such techniques may be performed *in vitro* (*i.e*., on the isolated DNA) or *in situ,* using the techniques of genetic manipulation or by treatment with mutagenic agents.

Preferably, the replication defective virus retains the sequences of its genome that are necessary for encapsidating the viral particles. DNA viral vectors include an attenuated or defective DNA viruses, including, but not limited to, herpes simplex virus (HSV), papillomavirus, Epstein Barr virus (EBV), adenovirus, adeno-associated virus (AAV), and the like. Defective viruses, that entirely or almost entirely lack viral genes, are preferred, as defective virus is not infective after introduction into a cell. Use of defective viral vectors allows for administration to cells in a specific, localized area, without concern that the vector can infect other cells. Thus, a specific tissue can be specifically targeted. Examples of particular vectors include, but are not limited to, a defective herpes virus 1 (HSV1) vector (Kaplitt et al. (1991) Mol. Cell. Neurosci., 2:320-330), defective herpes virus vector lacking a glycoprotein L gene *(See e.g.,* Patent Publication RD 371005 A), or other defective herpes virus vectors *(See e.g.,* WO 94/21807; and WO 92/05263); an attenuated adenovirus vector, such as the vector described by Stratford-Perricaudet et al. (J. Clin. Invest., 90:626-630 (1992); *See also,* La Salle et al. (1993) Science 259:988-990); and a defective adeno-associated virus vector (Samulski et al. (1987) J. Virol., 61:3096-3101; Samulski et al. (1989) J. Virol., 63:3822-3828; and Lebkowski et al. (1988) Mol. Cell. Biol., 8:3988-3996).

Preferably, for *in vivo* administration, an appropriate immunosuppressive treatment is employed in conjunction with the viral vector (*e.g*., adenovirus vector), to avoid immuno-deactivation of the viral vector and transfected cells. For example, immunosuppressive cytokines, such as interleukin-12 (IL-12), interferon-gamma (IFN-γ), or anti-CD4 antibody, can be administered to block humoral or cellular immune responses to the viral vectors. In addition, it is advantageous to employ a viral vector that is engineered to express a minimal number of antigens.

The vector can be an adenovirus vector. Adenoviruses are eukaryotic DNA viruses that can be modified to efficiently deliver a nucleic acid of the invention to a variety of cell types. The present invention contemplates adenoviruses of both human and animal origin. *(See e.g.,* WO94/26914). Various serotypes of adenovirus exist. Those adenoviruses of animal origin that can be used include adenoviruses of canine, bovine, murine (*e.g*., Mav1, Beard et al. (1990) Virol., 75-81), ovine, porcine, avian, and simian (*e.g*., SAV) origin. Preferably, the adenovirus of animal origin is a canine adenovirus, more preferably a CAV2 adenovirus (*e.g*. Manhattan or A26/61 strain (ATCC VR-800)).

Preferably, the replication defective adenoviral vectors comprise the ITRs, an encapsidation sequence and the nucleic acid of interest. Still more preferably, at least the E1 region of the adenoviral vector is non-functional. The deletion in the E1 region preferably extends from nucleotides 455 to 3329 in the sequence of the Ad5 adenovirus (*Pvu*II-*Bgl*II fragment) or 382 to 3446 *(Hinf*II-*Sau*3A fragment). Other regions may also be modified, in particular the E3 region (*e.g*., WO95/02697), the E2 region (*e*.*g*., WO94/28938), the E4 region (*e.g*., WO94/28152, WO94/12649 and WO95/02697), or in any of the late genes L1-L5.

The adenoviral vector may have a deletion in the E1 region (Ad 1.0). Examples of E1-deleted adenoviruses are disclosed in EP 185,573 or the adenoviral vector has a deletion in the E1 and E4 regions (Ad 3.0). Examples of E1/E4-deleted adenoviruses are disclosed in WO95/02697 and WO96/22378. The adenoviral vector may have a deletion in the E1 region into which the E4 region and the nucleic acid sequence are inserted.

The replication defective recombinant adenoviruses can be prepared by any technique known to the person skilled in the art *(See e.g.,* Levrero et al. (1991) Gene 101:195; EP 185 573; and Graham (1984) EMBOJ., 3:2917). In particular, they can be prepared by homologous recombination between an adenovirus and a plasmid that carries, *inter alia,* the DNA sequence of interest. The homologous recombination is accomplished following co-transfection of the adenovirus and plasmid into an appropriate cell line. The cell line that is employed should preferably (i) be transformable by the elements to be used, and (ii) contain the sequences that are able to complement the part of the genome of the replication defective adenovirus, preferably in integrated form in order to avoid the risks of recombination. Examples of cell lines that may be used are the human embryonic kidney cell line 293 (Graham et al. (1977) J. Gen. Virol., 36:59), which contains the left-hand portion of the genome of an Ad5 adenovirus (12%) integrated into its genome, and cell lines that are able to complement the E1 and E4 functions, as described in applications WO94/2691 and WO95/02697. Recombinant adenoviruses are recovered and purified using standard molecular biological techniques that are well known to one of ordinary skill in the art.

The adeno-associated viruses (AAV) are DNA viruses of relatively small size that can integrate, in a stable and site-specific manner, into the genome of the cells that they infect. They are able to infect a wide spectrum of cells without inducing any effects on cellular growth, morphology or differentiation, and they do not appear to be involved in human pathologies. The AAV genome has been cloned, sequenced and characterized. It encompasses approximately 4700 bases and contains an inverted terminal repeat (ITR) region of approximately 145 bases at each end, which serves as an origin of replication for the virus. The remainder of the genome is divided into two essential regions that carry the encapsidation functions: the left-hand part of the genome, that contains the *rep* gene involved in viral replication and expression of the viral genes; and the right-hand part of the genome, that contains the *cap* gene encoding the capsid proteins of the virus.

The use of vectors derived from the AAVs for transferring genes *in vitro* and *in vivo* has been described (*See e.g*., WO 91/18088; WO 93/09239; US Pat. No. 4,797,368; US Pat. No., 5,139,941; and EP 488 528). These publications describe various AAV-derived constructs in which the *rep* and/or *cap* genes are deleted and replaced by a gene of interest, and the use of these constructs for transferring the gene of interest *in vitro* (into cultured cells) or *in vivo* (directly into an organism). The replication defective recombinant AAVs according to the invention can be prepared by co-transfecting a plasmid containing the nucleic acid sequence of interest flanked by two AAV inverted terminal repeat (ITR) regions, and a plasmid carrying the AAV encapsidation genes (*rep* and *cap* genes), into a cell line that is infected with a human helper virus (for example an adenovirus). The AAV recombinants that are produced are then purified by standard techniques.

The gene can be introduced in a retroviral vector (*e.g*., as described in U.S. Pat. Nos. 5,399,346, 4,650,764, 4,980,289 and 5,124,263 ; Mann et al. (1983) Cell 33:153; Markowitz *et al*. (1988) J. Virol., 62:1120; PCT/US95/14575; EP 453242; EP178220; Bernstein et al. (1985) Genet. Eng., 7:235; McCormick, (1985) BioTechnol., 3:689; WO 95/07358; and Kuo *et al*., (1993) :845). The retroviruses are integrating viruses that infect dividing cells. The retrovirus genome includes two LTRs, an encapsidation sequence and three coding regions (*gag, pol* and *env*)*.* In recombinant retroviral vectors, the *gag, pol* and env genes are generally deleted, in whole or in part, and replaced with a heterologous nucleic acid sequence of interest. These vectors can be constructed from different types of retrovirus, such as, HIV, MoMuLV ("murine Moloney leukaemia virus" MSV ("murine Moloney sarcoma virus"), HaSV ("Harvey sarcoma virus"); SNV ("spleen necrosis virus"); RSV ("Rous sarcoma virus") and Friend virus. Defective retroviral vectors are also disclosed in WO95/02697.

In general, in order to construct recombinant retroviruses containing a nucleic acid sequence, a plasmid is constructed that contains the LTRs, the encapsidation sequence and the coding sequence. This construct is used to transfect a packaging cell line, which cell line is able to supply in trans the retroviral functions that are deficient in the plasmid. In general, the packaging cell lines are thus able to express the *gag, pol* and *env* genes. Such packaging cell lines have been described in the prior art, in particular the cell line PA317 (US Pat. No. 4,861,719 ), the PsiCRIP cell line (*See,* WO90/02806), and the GP+envAm-12 cell line (*See,* WO89/07150). In addition, the recombinant retroviral vectors can contain modifications within the LTRs for suppressing transcriptional activity as well as extensive encapsidation sequences that may include a part of the *gag* gene (Bender et al. (1987) Virol., 61:1639). Recombinant retroviral vectors are purified by standard techniques known to those having ordinary skill in the art.

Alternatively, the vector can be introduced *in vivo* by lipofection. For the past decade, there has been increasing use of liposomes for encapsulation and transfection of nucleic acids *in vitro.* Synthetic cationic lipids designed to limit the difficulties and dangers encountered with liposome mediated transfection can be used to prepare liposomes for *in vivo* transfection of a gene encoding a marker (Felgner et. al. (1987) Proc. Natl. Acad. Sci. USA 84:7413-7417; *See also,* Mackey, et al. (1988) Proc. Natl. Acad. Sci. USA 85:8027-8031; Ulmer et al. (1993) Science 259:1745-1748). The use of cationic lipids may promote encapsulation of negatively charged nucleic acids, and also promote fusion with negatively charged cell membranes (Felgner and Ringold (1989) Science 337:387-388). Particularly useful lipid compounds and compositions for transfer of nucleic acids are described in WO95/18863 and WO96/17823, and in U.S. Pat. No. 5,459,127.

Other molecules are also useful for facilitating transfection of a nucleic acid in *vivo*, such as a cationic oligopeptide (*e.g*., WO95/21931), peptides derived from DNA binding proteins (*e.g*.. WO96/25508), or a cationic polymer (e.g., WO95/21931). It is also possible to introduce the vector *in vivo* as a naked DNAplasmid. Methods for formulating and administering naked DNA to mammalian muscle tissue are disclosed in U.S. Pat. Nos. 5,580,859 and 5,589,466.

DNA vectors for gene therapy can be introduced into the desired host cells by methods known in the art, including but not limited to transfection, electroporation, microinjection, transduction, cell fusion, DEAE dextran, calcium phosphate precipitation, use of a gene gun, or use of a DNA vector transporter (*See e.g*., Wu et al. (1992) J. Biol. Chem., 267:963; Wu and Wu (1988) J. Biol. Chem., 263:14621; and Williams et al. (1991) Proc. Natl. Acad. Sci. USA 88:2726). Receptor-mediated DNA delivery approaches can also be used (Curiel et al. (1992) Hum. Gene Ther., 3:147; and Wu and Wu (1987) J. Biol. Chem., 262:4429).

### EXPERIMENTAL

The following examples are provided in order to demonstrate and further illustrate certain preferred embodiments and aspects of the present invention and are not to be construed as limiting the scope thereof.

In the experimental disclosures which follow, the following abbreviations apply: N (normal); M (molar); mM (millimolar); µM (micromolar); mol (moles); mmol (millimoles); µmol (micromoles); nmol (nanomoles); pmol (picomoles); g (grams); mg (milligrams); µg (micrograms); ng (nanograms); 1 or L (liters); ml (milliliters); µl (microliters); cm (centimeters); mm (millimeters); µm (micrometers); nm (nanometers); °C (degrees Centigrade); SNHL (Sensorineural Hearing Loss); AISNHL, (Auto Immune Sensorineural Hearing Loss); IESCA (Inner Ear Supporting Cell Antigen); Mab (Monoclonal Antibody); HSP-70 (Heat Shock Protein); IF (Immunofluorescence); SDS PAGE (Sodium dodecyl sulfate polyacrylamide gel electrophoresis).

### EXAMPLE 1

### Isolation Of KHRI-3 MAb

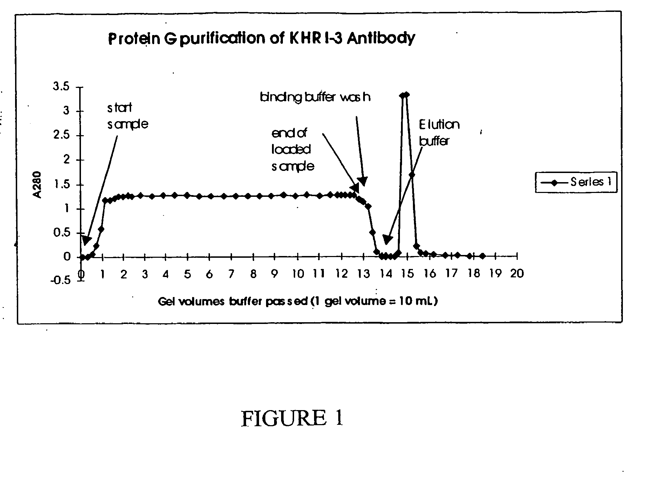

The preparation of hybridoma cells which produce KHRI-3 monoclonal antibodies was reported earlier (Zajic, G et al. (1991) Hearing Res 52:59-72). Hybridoma cells were cultured in a CELLMAX (CELLMAX^{®} Artificial Capillary System, Cellco, Inc., Germantown, MD) bioreactor. The bioreactor was inoculated with KHRI-3 hybridoma cells. The serum-free, antibiotic-free medium was changed to lactic acid production to maintain optimal cell growth and antibody production. Once the system was operating at optimal conditions, 45 ml of fluid containing antibody and excess cells was harvested every second day from the extra-capillary space. The IgG1 antibody binding capacity of a protein G affinity column was determined. An amount of bioreactor supernatant calculated to contain that quantity of antibody was loaded and the effluent was monitored for absorbance at A₂₈₀. After all of the sample was loaded, the column was washed with binding buffer. The elution buffer was applied when the protein content of the effluent reached zero absorbance and the second peak was collected in three fractions corresponding to the center and borders of the peak (Figure 1). Protein and IgG content of each fraction was assessed using Bradford assays, IgG ELISA and SDS-PAGE followed by Western blotting with anti-Ig antibody. Samples from the eluted antibody peak of eight column runs were pooled to yield 80.5 mg of antibody.

### EXAMPLE 2

### Purification and Characterization of the KHRI-3 Inner Ear Target Antigen

### A. Purification By Affinity Chromatography

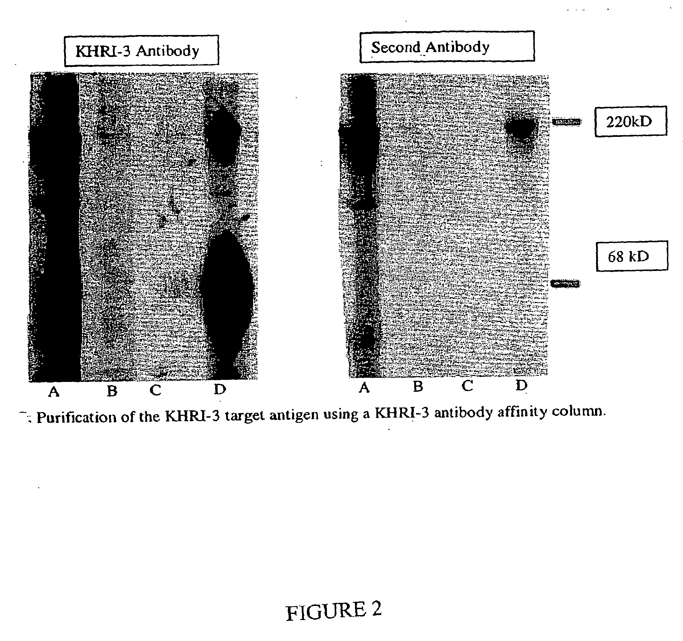

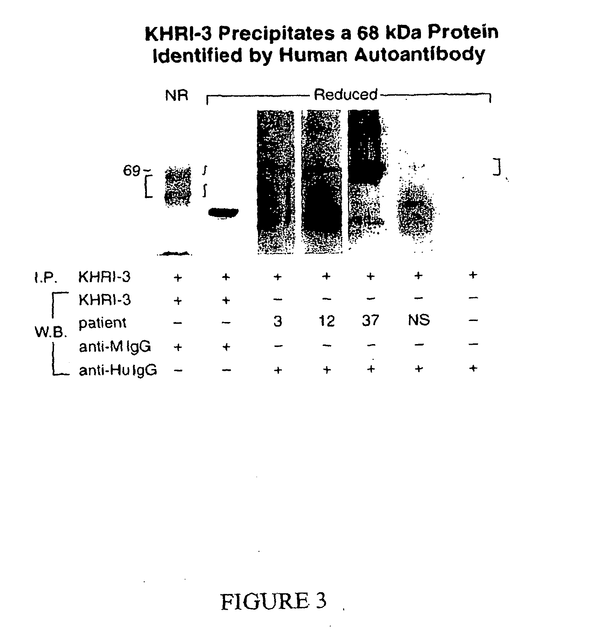

KHRI-3 MAb, affinity purified on protein G sepharose (as described in Example 1 above), was covalently linked to sepharose beads and used to affinity purify IESCA. Inner ear extract was passed onto the column after washing the column and was rinsed extensively with binding buffer. The samples coming off the column were monitored for protein using absorbance at 280 nm. When no protein was detected in the rinse buffer, the bound material was eluted with glycine-HCL (pH 2.0) buffer. The fractions were collected in twenty 1 ml fractions. Each fraction was monitored for absorbance at 280 nm. Pooled fractions (1-5, 6-10, 11-15 and 16-20) were concentrated to 100 µl, electrophoresed on a 7% SDS-PAGE gel, transferred to nitrocellulose and Western blotted using chemi luminescence. Fraction pool 16-20 alone contained KHRI-3 reactive protein as determined by Western blot (Figure 2). When the SDS-PAGE gel was run under reducing conditions a strong doublet was detected at 68 kD and 70 kD (Figure 3). Under non-reducing conditions a doublet was resolved at 65 kD and 68 kD. Stripping and reprobing the blot with anti-IgG antibody showed that the band was not due to free IgG leaching from the column.

### B. The KHRI-3 Antigen And Heat Shock Protein Are Distinct

The nature of the target antigen of human autoimmune sera has been suggested to be heat shock protein, specifically HSP-70 (Rauch et al. (1995) Am. J. Otology 16:648-652; Shin et al. (1997) Laryngoscope 107:222-227; Bloch et al.(1996) Arch. Otoloaryngol. Head Surg. 121:1167-1171). However, the inventors have concluded that the inner ear specific antigen and HSP-70 are not the same, based upon the following observations. First, HSP-70 is a ubiquitous protein, and antibodies to it would not necessarily target the inner ear. Second, antibodies to HSP-70 are found in the sera of many patients with other organ specific autoimmune diseases (Figuerdo et al. (1995) Clin. Immunol. Immunopathol. 79:252-255; Shingai et al. (1995) J. Hepatol. 23:382-390; Paggi et al. (1995) Endocr. Res. 21:555-567), yet it has been demonstrated that antibodies to more restricted antigens are the cause of specific autoimmune diseases (Anhalt et al. (1983) Arch Dermatol. 119:711-714; Wilkin (1990) N. Engl. J. Med. 323:1318-1324; Amagai et al. (1992) J. Clin. Invest. 90:919-926; Feldt-Rasmussen et al. (1991) Autoimmunity 9:245-254; Disher et al. (1997) Ann. NY Acad, Sci. 830:253-65; Mappouras et al. (1995) Clin. Exp. Immunol. 100:336-343; Chiovato et al. (1993) J. Clin. Endocrinol. Metab. 7:1700-1705; Li et al. (1995) J. Immunol 154:3328-3332). Third, the inventors have discovered that monoclonal antibody KHRI-3 can precipitate an antigen from inner ear extracts that reacts strongly with AISNHL patients' sera, but purified KHRI-3 does not precipitate a protein from these extracts that binds antibodies to HSP-70. Fourth, the inventors have also discovered that KHRI-3 and Western blot positive AISNHL human sera stain inner ear tissues with a similar pattern, but highly purified, high titered KHRI-3 antibody concentrated from hybridoma supernatant or produced in a bioreactor does not stain HSP-70 protein on Western blots.

Evidence that the KHRI-3 Antigen and HSP-70 are distinct. Monoclonal antibody to HSP-70 does not stain the guinea pig organ of Corti. These experiments were performed using immunofluorescence studies on surface preparations, both with and without tissue permeabilization. This difference in tissue expression demonstrates that HSP-70 is different from the antigen which binds KHRI-3, as KHRI-3 does bind to supporting cells on surface preparations, both *in vivo* and *in vitro.*

HSP-70 is present in the inner ear, as demonstrated by the observations that it can be immunoprecipitated from the inner ear and detected on Western blots using anti-HSP antibody. However, when proteins immunoprecipitated from guinea pig inner ear by anti HSP-70 and highly purified KHRI-3 monoclonal antibodies were Western blotted, KHRI-3 stained only the protein it precipitated, and not HSP-70. Similarly, anti-HSP-70 stained only the 70 kD protein it precipitated, and not the protein precipitated by KHRI-3 (Nair *et al*., Association for Research in Otolaryngology Feb 15-19, 1998).

### EXAMPLE 4

### Characterisation and Role in Hearing Loss of Antibody in Autoimmune Sensory Neural Hearing Loss Patients

This example describes the characterisation and role in hearing loss of antibodies to IESCA in AISNHL patients.

### A. Patient Selection

Patients with sudden onset hearing loss, fluctuating hearing and rapidly progressive unilateral or bilateral hearing loss were considered possible autoimmune patients. Sudden onset cases were included after review of the histories of several bilateral progressive cases demonstrated that these patients often reported an initial event of sudden hearing loss in one ear that subsequently progressed to involve the opposite ear. This group was included because they may represent the acute stage of early disease.

Hearing loss was defined as greater than 30 dB at any frequency or less than 85% speech discrimination in either one (unilateral) or both ears (bilateral). If the hearing loss developed within a 24 hour period and did not progress subsequently, the patient was considered to have sudden hearing loss. Either unilateral or bilateral hearing loss with greater than 10 dB progression within three months was classified as rapidly progressive (Moscicki et al. (1994) JAMA 272:611-616). An initial analysis of 91 patients has been reported (Disher et al. (1997) Ann. NY Acad. Sci. 830:253-265). Of the 91 patients, seventeen did not fit in these categories and were not included in the analysis. These seventeen patients included: four patients with vertigo and 1 with tinnitus, who were excluded because of insufficient hearing loss to fit the criteria; seven patients who were classified as having slowly progressive hearing loss (*i.e*., less than a 10 dB progression in three months); four patients who were not well classified and more data has been requested; and one patient who had stable hearing loss leaving 74 in the group.

### B. Methods

### Western Blot Analysis

The University of Michigan Committee on Use and Care of Animals approved all studies described in this report. The Unit for Laboratory Animal Medicine of the University of Michigan provided Veterinary care and housing. Guinea pigs (250-300 g) were anesthetized and decapitated. The temporal bones were removed, opened to reveal inner ear structures, and immediately placed in phosphate buffered saline (PBS) on ice. The organ of Corti and vestibular tissues were harvested and placed in lysis buffer (1% NP-40 in PBS pH 7.2 containing protease inhibitors (1mM PMSF, leupeptin 1 µg/mL, antipain 2 µg/mL, benzamidine 10 µl/mL, aprotinin 10 ku/mL, chymostatin 1 µg/mL, pepstatin 1 µg/mL)). The lysate was allowed to stand at 4°C for 30 minutes following homogenization. After low speed centrifugation (1000 rpm for 3 min) to remove bone, the lysate was mixed with sample buffer containing 0.0625 M tris-HCL pH 6.8, 2% SDS, 10% glycerol, and 0.005% bromophenol blue. For reducing conditions, the sample contained 5% (v/v) 2-mercaptoethanol.

The samples were boiled for 5 minutes, and separated on a 3% stacking and 7% polyacrylamide separating gel. The gel was electrophoresed for 90 minutes at constant current of 25 mA/gel for minigels and for 4 hours at constant current of 35 mA/gel for larger gels. Electrophoretic transfer to a nitrocellulose membrane was carried out at 35 V/gel overnight. The nitrocellulose paper was cut into strips and blocked with milk buffer (Blotto) (50 mM tris-HCl, pH 8.0, containing 5% nonfat dry milk, 2 mM CaCl2, and 5% Tween 20). All subsequent incubations were done in this buffer. Strips were incubated for 2 hours at room temperature with human serum diluted at 1:50, or KHRI-3 diluted to 10 ug/mL, washed 3 times for 10 minutes each, and incubated for 2 hours at room temperature with secondary antibody (goat anti-human IgG/IgM or anti-mouse IfG heavy and light chain-specific conjugated with horseradish peroxidase (Jackson ImmunoResearch Laboratories, Inc., West Grove, Pennsylvania), diluted at 1:500). The blots were developed in 4-chloro-1-napthol (0.5 mg/mL) in methanol-PBS, pH 7.6 (1:5) containing 0.05 % hydrogen peroxide.

### Immunofluorescence

Guinea pigs weighing 250-300 g were anesthetized, decapitated, and the bullas were fixed locally with 4% paraformaldehyde for 2 hours. After the cochleae were dissected free of the surrounding bone, the spiral ligament and tectorial membrane were removed. The central bony cochlae containing the organ of Corti were incubated in 3% normal goat serum for 1 hour. The specimens were washed 3 times for 5 minutes in PBS, and incubated in primary antibody (serum diluted at 1:50 or KHRI-3 10 ug/mL) overnight at 4 °C, washed three times for 5 minutes in PBS, and incubated for 45 min at room temperature in secondary antibody (Lissamine Rhodamine LRSC anti-Human IgG/IgM heavy and light chain-specific (Jackson ImmunoResearch Laboratories Inc, West Grove) or rhodamine (TRITC) conjugated to goat antimouse anti-IgG (Accurate Chemicals, Old Westbury, NY), diluted 1:200 in PBS (pH 7.4). The cochleae were washed in PBS, and then the organ of Corti was carefully peeled off the modiolus and mounted in GVA mounting media (Zymed Labs, San Francisco, CA).

### Immunoprecipitation and Western Blotting

The KHRI-3 antigen was immunoprecipitated from guinea pig cochlea and vestibular tissue extracts using KHRI-3 antibody. For each lane in an experiment, cochlear and vestibular tissue from both ears of a guinea pig were homogenized in 25 uL lysis buffer with protease inhibitors, as described above. The lysate (25 ul) was mixed with 50 ul of BSA and 150 ul of wash buffer containing 1% Nonidet P-40, 50 mM tris-HCl (pH 8), 150 mM NaCl, 0.1% sodium deoxycholate, 0.1% SDS, and 1 mM phenylmethylsulfonyl fluoride per sample. The samples were precleared twice with protein G-agarose for 30 minutes and incubated overnight at 4 °C with 200 ul of the KHRI-3 antibody from hybridoma supernatant. Antibody-antigen complexes were precipitated by incubation with protein G-agarose for 2 hours at 4 °C. The supernatant was removed after centrifugation at 5000 x g for 5 minutes. The precipitates were washed four times with wash buffer, centrifuged, resuspended in running buffer under reducing or nonreducing conditions, and separated by SDS-PAGE using a 7% gel. The gel was electrophoresed at 25 mA of constant current. The proteins were transferred as described above, and Western blotted and human sera or KHRI-3 antibody as described above.

### C. Reactivity of AISNHL Patient Sera with Guinea Pig Inner Ear Antigens Determined by Western Blots

Sera from the remaining 74 patients with rapidly progressive or sudden onset hearing loss were evaluated for reactivity with guinea pig inner ear antigens using Western blot (73 patients). The results of the analyisis, grouped according type of hearing loss and serological result, is shown in Table 1 (Disher et al. (1997) Ann. NY Acad. Sci. 830:253-265). Fifty-four patients (36 bilateral and 18 unilateral) had rapidly progressive hearing loss. Twenty patients (2 bilateral and 18 unilateral) had sudden hearing loss. Of the 54 patients with rapidly progressive hearing loss tested by Western blot, 28 (52%) stained positively for a 68-70 kD protein. Similarly, one-half of the sudden hearing loss patients, 9/19 (47%), tested positively. Of the 54 patients with rapidly progressive hearing loss, 50% (18/36) with bilateral involvement and 56% (10/18) with unilateral involvement were Western blot positive.

| **Table 1** | | | |
|---|---|---|---|
| Western Blot Results By Type Of Hearing Loss | | | |
| | **Type Of Hearing Loss** | | **Total** |
| | **Rapidly Progressing** | **Sudden** | |
| Number of Patients | 54 | 20 | 74 |
| Western Blot: No. Positive / No. Tested / % Positive | 28 / 54 / 52% | 9/19/47% | 37 / 73 / 51% |

### D. Location of AISNHL Patient Sera Binding to Guinea Pig Inner Ear Antigens by Immunofluorescence Staining

To determine the location of the inner ear antigen to which AISNHL patient sera binds, Western blot positive sera, Western blot negative sera, normal donor sera, and sera from previously untested patients was tested for immunofluorescence staining on surface preparations of guinea pig organ of Corti. Including the previously reported cases (as described in above), a total of 166 AISNHL patients have now enrolled in the study.

Sera from 158 AISNHL patients have been studied for antibody to a 68 kD inner ear protein by Western blot and from 143 AISNHL patients have been tested by immunofluorescence on organ of Corti surface preparations. Many sera stained supporting cells. The staining was distributed in punctate clusters over the surfaces of the supporting cells, including the surface of the pillar cells, the phalangeal processes of the outer pillar cells, and the phalangeal processes of the Deiters' cells. There was a very strong relationship between sera that were Western blot positive and those that stained supporting cells as shown in Table 2.

| **Table 2** | | | | |
|---|---|---|---|---|
| Results For Sera From Suspected Autoimmune Hearing Loss Patients By Both Assays | | | | |
| | | **Immunofluorescence** | | **Total** |
| | | **Positive** | **Negative** | |
| Western blot | Positive | 88 | 9 | 97 |
| | Negative | 22 | 24 | 46 |
| | Total | 110 | 33 | 143 |

Chi square analysis rejects the hypothesis of independence. The Western blot and immunoflourescence results are significantly related (p<0.001).

Most Western blot positive sera, 88/110 (80%), also were positive by immunofluorescence (Table 2). Similarly, 24/33 sera (73%) that were negative by Western blot were also negative by immunofluorescence. Chi square analysis rejects the hypothesis of independence between antibody to the 68 kD protein and staining of supporting cells, indicating that the Western blot and immunofluorescence results are significantly related (p< 0.001). Examples of staining have been published (Disher et al. (1997) Ann. NY Acad. Sci. 830:253-265).

This pattern of immunofluorescence staining exhibited by the human sera on surface preparations of guinea pig organ of Corti is of particular interest, because the patient sera replicate the staining pattern observed with the monoclonal antibody KHRI-3 (Zajic et al. (1991) Hearing Res.52:59-72; Ptok et al. (1991) Hearing Res. 57:79-90; Nair et al. (1995) Hearing Res. 83:101-113; Disher et al. (1997) Ann. NY Acad. Sci. 830:253-265).

### E. Antibodies of Normal Control Serum Donors

Thus far, sera from 20 normal subjects ranging in age from 20 to 52 years of age have been tested by Western blot and immunofluorescence, as described above in sections B and C. None were positive for a 68 kD protein band, and only one stained supporting cells. All control subjects have normal hearing. Table 3 shows the frequency of antibodies to inner ear antigens in the sera of normal hearing individuals.

| **Table 3** | | | |
|---|---|---|---|
| Frequency Of Antibodies To Inner Ear Antigens In The Sera Of Normal Hearing Individuals | | | |
| **Immunofluorescence To Supporting Cells** | **Western Blot On Inner Ear Extract** | | **Totals** |
| | **Number Of Sera Positive** | **Number Of Sera Negative** | |
| Number of sera positive | 0 | 1 | 1 |
| Number of sera negative | 0 | 19 | 19 |
| Totals | 0 | 20 | 20 |

### F. Human Inner Ear Absorbs Human Antibodies that Bind to Guinea Pig Inner Ear

To determine if the antigen detected by patient sera in guinea pig inner ear tissue is also present in human inner ear, absorption analysis was performed. Inner ear tissue removed from patients undergoing ablative inner ear surgery was used as the source of inner ear antigen, and white and red blood cells from the same donors were used as histocompatibility and blood group antigen controls. Equal volumes of either packed blood cells or inner ear tissue were mixed with an aliquot of immunofluorescence (IF) positive patient sera and then retested on guinea pig inner ear substrate. Absorption with inner ear but not blood cells removed the antibody reactivity to guinea pig inner ear (Disher et al. (1997) Ann. NY Acad. Sci. 830:253-265). In a second experiment not shown, a similar but less complete absorption was obtained.

### G. Western Blot Positive AISNHL Sera Stain An Inner Ear Protein Precipitated by KHRI-3

The similarity of the results observed from Western blotting and from immunofluroescence staining for both KHRI-3 and human sera, and previous observations that KHRI-3 can cause hearing loss (Nair et al. (1995) Hear. Res. 83:101-113; Nair et al. (1997) Hearing Res. 107:93-101) suggested that the human sera might bind to the same antigen as KHRI-3. To test this hypothesis, proteins precipitated from inner ear extracts by KHRI-3 were subjected to SDS PAGE, Western blotted and stained with patient sera. Thus far, 4/4 patients' sera that were positive for the 68-70 kD band by Western blot also stained a 70 kD band in the material immunoprecipitated by KHRI-3. All three normal sera controls tested are negative.

The KHRI-3 antibody binds best to non-reduced inner ear proteins. Under these non-reducing conditions, a doublet of 65 and 68 kD is resolved. Although KHRI-3 reacts poorly with reduced proteins, faint staining of the bands which shift to 68 and 70 kD under reducing conditions can be discerned. The human sera stain most strongly the upper band detected by KHRI-3 under reducing conditions. In contrast to KHRI-3, the human sera bind best to reduced proteins, indicating that the human antibodies and the murine antibody may identify different conformational epitopes on the same protein.

### H. The Presence of Antibody to Supporting Cells is Significantly Associated with Hearing Improvement after Steroid Treatment

Although this is not a clinical study, the response to steroid treatment in a subset of patients, for whom both an acute phase serum specimen and objective measurements of hearing before and after steroid therapy was available, was prospectively investigated. A total of 64 patients who were treated with steroids fit these criteria. Hearing improvement was based on the criteria of Moscicki *et al*. (Moscicki et al. (1994) JAMA 272-611-616) and was defined as greater than 10 dB threshold improvement at two consecutive frequencies and/or an improvement in speech discrimination of at least 20%.

In the patient subset, 67% of the patients were antibody positive by Western blot and 75% were positive by immunofluorescence staining. About half of the patients (33/64) demonstrated clinical improvement, and about half (31/64) did not. Of those who were Western blot positive, 53% (23/43) improved; similarly, 48% (10/21) of the Western blot negative patients showed hearing improvement (p=0.66). In contrast, 60% (29/48) of patients who were immunofluroescence positive had hearing improvement, as compared to improvement in only 25% (4/16) of those who were immunofluorescence negative (p=0.021). Table 4 shows the relationship between the presence of antibody to supporting cells in the sera and hearing improvement. In fact, 88% (29/33) of those who improved were positive for antibody to supporting cells by the immunofluorescence assay and only 12.1% (4/33) of those who improved were immunofluroescence negative. The presence of antibody to supporting cells is statistically significantly associated with hearing improvement after steroid therapy (Relative Risk: 2.4). Therefore, patients whose acute phase sera are immunofluorescence positive are greater than two times more likely to experience improved hearing with steroid treatment than patients whose sera are immunofluorescence negative.

| **Table 4** | | | |
|---|---|---|---|
| Relationship Between Antibody To Supporting Cells And Hearing Improvement | | | |
| | **Hearing Improvement** | **No Hearing Improvement** | **Total** |
| IF Positive | 29 (60%) | 19 (40%) | 48 (100%) |
| IF Negative | 4 (25%) | 12 (75%) | 16 (100%) |
| Total | 33 | 31 | 64 |

The difference is statistically significant, p=0.021 (Fisher Exact Test).

This data provides further support for the importance of the inner ear supporting cell antigen in the pathogenesis of autoimmune hearing loss. It is not surprising that a subset of the immunofluorescence positive patients fail to improve, since some may have already developed irreversible damage at the time therapy was initiated. Moreover, it is not surprising that detecting antibodies to IESCA by IF correlated better to treatment outcome than detecting a 68-72 kDa band on Western blotting. Western blotting determines antibodies to 68-72 kDa proteins, of which there could be more than one in the guinea-pig cochlear tissue. IF identifies a potentially unique binding pattern to cochlear supporting cells, observed in an animal model of antibody-induced hearing loss, as described above. The IF result identifies a cellular localization already linked directly to antibody induced hearing loss. A positive Western blotting result can reflect the presence of antibodies to irrelevant proteins of a similar weight, weakening specificity. In this regard, heat shock protein 70 also migrates in this region and as discussed above, HSP70 was thought to be the target of autoimmune hearing loss antibodies.

It is also possible that serum antibody titers were sufficient for detection of antibody by IF, but not by Western blotting. This possibility is supported by the observation that 25% of the patients who improved with steroid treatment were negative on Western blotting, but 86% of these patients were IF positive, thus identifying a group of steroid responders without detectable antibodies to the 68-72 kDa band. Further evidence is provided by the observations that some patients whose sera were negative on a western blot of total serum proteins became positive for a 68-72 kDa band when tested on a subset of proteins comprising concentrated antigen precipitated from inner ear extracts using KHRI-3-coupled beads. These observations indicates that a AISNHL-inducing antibody may in fact be present at low titer in serum from these patients, despite the negative result on a standard Western blot assay. This also suggests that in developing routine assays for detecting antibodies for IESCA or other AISNHL associated proteins, it might be useful to concentrate the antigen samples by immunoprecipitation, such as with KHRI-3-coupled beads, before Western blotting.

### EXAMPLE 5 Characterisation and Identification of IESCA

This example describes the characterization and identification of IESCA as CTL2.

### A. Materials and Methods

### Antibody production, purification, and coupling to sepharose beads

Serum free KHRI-3 was produced in the CELLMAX ® Artificial Capillary System according to the manufacturer's instruction (Nair TS et al. (1999) Hearing Res 129:50-60). In brief, the hybridoma was inoculated into the extra capillary space and fresh chemically defined, serum-free medium designed for hybridoma cultures (GIBCO, Grand Island, NY) was circulated through the capillary network. Excess cells and fluid containing antibody were removed by flushing the extra capillary space at regular intervals. The cells were removed by centrifugation and the supernatants containing the antibody were pooled. The pooled KHRI-3 antibody was affinity purified using protein G columns (Pierce, Rockford, IL) (Nair, T.S et al. (1997) Hear. Res. 107:93-101) following the kit protocol. Purified antibody was tested for purity by gel electrophoresis and antibody activity was tested by immunofluorescence (IF) as previously described (Nair, T.S. et al. (1995) Hear. Res. 83:101-113).

### Peptide synthesis and rabbit antisera to CTL2

A custom 19 amino acid peptide corresponding to an antigenic region of human CTL2 that fell within one of the regions identified in guinea pig IESCA by MS/MS amino acid sequencing was synthesized and used to raise rabbit antisera (Princeton Biomolecules, Langome, PA). Two rabbits (R228 and R229) were immunized with 500ug of the CTL2 peptide, boosted two times and bled 10 days later, boosted again and bled 10 days after immunization. The antisera were identified as bleeds 1, 2, and 3.

### Coupling of antibody to beads

Protein G-purified KHRI-3 IgG1 antibody and protein-A sepharose (Pierce), purified IgG from the rabbit antisera to CTL2 were coupled to cyanogen bromide activated Sepharose 4B beads (Sigma, St. Louis, MO) following the protocol supplied by the manufacturer. In general, purified antibody was coupled in a ratio of 5mg antibody to I gram of beads swollen to 4 ml after antibody coupling. The coupling reaction is efficient and little or no detectable antibody remained in the supernatant after coupling. Thus, the coupled beads contained approximately 1.25 mg/ml of antibody.

### Inner ear extracts

All studies described in this report were approved by the University of Michigan Committee on Use and Care of Animals and NIH NIDCD review committees (R01 DC02272 and R01 DC03686). The Unit for Laboratory Animal Medicine of the University of Michigan provided veterinary care and housing. Immediately after euthanasia, guinea pig temporal bones were collected; the bullae were opened and placed in PBS on ice. The bone of the otic capsule was quickly but carefully removed, the organ of Corti and vestibular tissues were collected in lysis buffer and immediately homogenized (Zajic, G. et al. (1991) Hear. Res. 52:59-72). Lysis buffer consists of 1% NP-40 in phosphate buffered saline pH 7.2(PBS) containing 1mM phenylmethylsulfonyl fluoride (PMSF) and a cocktail of freshly prepared protease inhibitors (Roche Diagnostics, Mannheim, Germany). After homogenization the lysates were allowed to stand on ice for 30 mins, and were then centrifuged at 13,000 rpm for 2 minutes to pellet the bony modiolus. The supernatant was collected and used as whole cell lysate.

### SDS-PAGE electrophoresis

Inner ear lysates were analyzed using sodium dodecylsulfate-polyacrylamide gel electrophoresis (SDS-PAGE). Cochlear extracts, immunoprecipitated antigen, and molecular weight standards were prepared using sample buffer containing 0.0625M Tris-HCl pH 6.8, 2% SDS, 10% glycerol, 0.005% bromophenol blue. For reducing conditions the sample buffer contained 5% (v/v) 2-mercaptoethanol. The samples were boiled for 5 minutes and separated on 3% stacking and 8 or 10% separating gels. Samples for MS-MS sequencing were isolated using 1mm thick gels. All the reagents used for sequencing studies were prepared in HPLC grade water.

### Immunoprecipitation

The KHRI-3 antigen and CTL2 antigen were immunoprecipitated from guinea pig cochlea and vestibular tissue extracts using KHRI-3 and anti CTL2 antibodies. For each lane in an experiment, cochlear and vestibular tissue from one ear were homogenized in 50ul lysis buffer with protease inhibitors as described earlier. The lysate was mixed with either 20ul of KHRI-3-CNBr beads or CNBr coupled CTL2 antibody (20ul/ear), and incubated overnight at 4°C; the precipitated proteins were recovered by centrifugation of the beads. In some experiments, CTL2 antigen was incubated overnight with anti CTL2 antibody, and the immune complexes then precipitated by addition of 30ul of protein A agarose (Sigma) for 2 hours at 4°C. The supernatants were removed after centrifugation at 5000 x g for 5 min. The precipitates were washed three times, resuspended in sample buffer for either reducing or non-reducing conditions, boiled, and subjected to electrophoresis.

### Western blotting

Proteins from electrophoresis gels were transferred to nitrocellulose membranes at constant voltage of 25 V in transfer buffer (20% methanol, 150 mM glycine, 20 mM tris buffer). The membranes were incubated in 5% dry milk in phosphate buffered saline containing 0.1% Tween (PBS - T) on a rocker for one hour at room temperature to block non-specific binding. All subsequent incubations were done in this buffer. The nitrocellulose strips were incubated for 2 hours at room temperature with primary antibody, either KHRI-3 monoclonal antibody diluted to 1ug/ml or anti CTL2 peptide antibody (rabbit antiserum) diluted 1:1000, and washed 3 times with PBS-T for 10 mins each time. After washing, the strips were incubated for 2 hr in affinity purified secondary antibodies (either rat-anti-mouse IgG heavy and light chain specific, or mouse-anti-rabbit) conjugated to horseradish peroxidase (Jackson ImmunoResearch Laboratories Inc., West Grove, PA.) diluted 1:5000 and 1:10000 respectively. The blots were developed with enhanced chemiluminescence (ECL) by applying a 1:1 mix of ECL reagents 1 and 2 for 1 minute, after which the blots were drained, covered with plastic wrap and exposed to X-ray film. Exposure time varied from 10 seconds to a few minutes, depending on the reaction intensity.

### Immunoaffinity purification and sequencing of Inner Ear Supporting Cell Antigen (IESCA)

To isolate and purify the IESCA for MS/MS peptide sequence analysis, both an immunoaffinity column and immunoprecipitation with the KHRI-3-CNBr beads were used.

In the first experiment peptide sequencing experiment, the IESCA was purified by immunoaffinity column purification. Protein extracts from six animals (12 ears) were divided into two aliquots and each was mixed with 1 ml of beads coupled to 1.25mg of purified KHRI-3 monoclonal antibody. The antibody-coupled beads were washed with binding buffer (lysis buffer, described above) and incubated with guinea pig cochlear lysate overnight at 4°C, with gentle rocking. The beads were then transferred to a 10 ml Poly-Prep Chromatography Column (Bio-Rad Richmond, CA). After the flow through was collected, the beads were washed with 10 bed volumes of binding buffer (Dulbecco's phosphate buffered saline (GIBCO, Grand Island, NY) containing 0.5% NP-40 and a cocktail of protease inhibitors (Roche Diagnostics, Mannheim, Germany). The antigen was eluted with 100mM glycine/HCl pH 2.8, containing 0.5% NP-40 and collected as 1 ml fractions. The first 5 fractions of the eluate were immediately neutralized with 1M sodium phosphate pH 8.0, pooled, and concentrated to 50uL using Millipore Ultrafree Centrifugal filters which were washed three times with PBS according to the manufacturer's instructions (resulting in a total volume of ~120uL). The concentrated sample was loaded into two lanes (50ul each) on a 1mm thick 8% SDS PAGE gel for electrophoresis under reducing conditions. Two additional lanes, each containing the equivalent of one ear, were prepared for western blotting. Two bands that migrated at 68 and 72 kDa, and corresponded to protein bands stained with the KHRI-3 antibody on the western blot were identified in each of the two lanes in the 1mm thick gel by colloidal Coomassie blue staining. These were carefully cut out of the gel and pooled. A control region of the same size was cut from an empty lane in the same gel as a background control. A second affinity purification experiment was performed using another 3 ears and these colloidal Coomassie blue-stained bands were pooled with those from the first experiment. The gel slices were washed 2 times for 3 min each time with 50% acetonitrile (CH₃CN) in water. The liquid was decanted, the slices were frozen and sent to the Microchemistry Facility of Harvard University for MS/MS peptide sequencing.

In a second peptide sequencing experiment, the IESCA was immunoprecipitated. Extracts from 12 ears were incubated overnight with 240ul of KHRI-3-CNBr beads (~300ug of purified antibody) overnight at 4°C and then washed with 5 times with 1ml of binding buffer each time. After the last wash, 180 ul of the beads corresponding to 9 ears were resuspended in 120ul of reducing buffer (1%SDS, glycerol, 2 mercaptoethanol) and boiled. After brief centrifugation to pellet the beads, the supernatant was removed and loaded in one lane of an 8% 1mm thick SDS-PAGE gel for electrophoresis and colloidal Coomassie blue staining. The remaining 60 ul of beads were resuspended in 90ul of reducing buffer, boiled, centrifuged and the supernatant was divided into three lanes for western blotting. As before, the colloidal Coomassie blue stained bands migrating at 68 and 72 kDa that corresponded to the bands stained by the KHRI-3 antibody were carefully cut out. This time, the 68 kDa band and the 72 kDa band were placed in separate tubes. As in the previous experiment, a control slice was collected from an empty lane. The gel slices were treated 2 times with 50% acetone-nitrile solution, decanted, frozen and sent for MS/MS sequencing.

### MS/MS sequencing

Gel slices containing bands of interest and control slices were subjected to dual Mass Spectrometric sequence at the Microchemistry Facility of Harvard University, Cambridge, MA. Proteins eluted from the gel slices were digested with trypsin, and passed through a single microcapillary reverse-phase HPLC column directly coupled to the nano-electrospray ionization source of an ion trap mass spectrometer. The peaks containing ~15 mer peptides were collected, passed into a collision cell, bombarded with argon or helium ions to break the peptides at the amide bonds, and the resulting fragments passed through the nano-electrospray ionization chamber and into the mass spectrometer. The resulting MS/MS spectra were then correlated with known mass and ion charge spectra using an algorithm that reconstructs the original peptide sequence and compares it to known proteins in the major databases.

### ELISA and immunofluorescence on surface preparations

Antisera were tested for approximate antibody titer by ELISA (Zajic, G. et al. (1991) Hear. Res. 52: 59-72), using 96 well plastic plates coated with 1ug CTL2 peptide. After coating for overnight at 4°C, the plates were washed, blocked with 5%goat serum, then incubated sequentially for 1 hour each with serial dilutions (1/10-1/320) of the rabbit anti-CTL2 sera, washed, and incubated with alkaline phosphatase-conjugated goat anti-rabbit IgG diluted 1/5000 as described for western blots. After a final wash with PBS, para-nitrophenylphosphate was used as the substrate and the absorbance at 415 nm was determined after 30 min.

### Immunofluorescence on guinea pig organ of Corti and human vestibular tissue

Guinea pigs were decapitated and the bullas with exposed otic capsule were fixed locally with 2% paraformaldehyde for 2 hours. The cochlea were dissected free of spiral ligament and tectorial membrane, permeabilized with 0.3% triton X-100, and incubated in 3% normal goat serum for one hour. Fresh normal human inner ear vestibular tissue was obtained from patients undergoing translabyrinthine procedures for removal of acoustic tumors. Patients all gave prior informed written consent that was approved by the University of Michigan Institutional Review Committee for the Protection of Human Research Subjects. The human tissues were prepared by fixation in 2% paraformaldehyde in phosphate buffer pH 7.2 for 2 hr. The specimens were washed three times with PBS, then the cupula was carefully removed and the crista ampularis was blocked with 3% goat serum and incubated overnight at 4°C in anti-CTL2 serum diluted 1:100. The tissue was then washed three times in PBS, and incubated for 45 minutes at room temperature in affinity-purified rhodamine (TRITC)-conjugated goat anti rabbit antibody from Jackson Immunoresearch Laboratories Inc. The tissues were washed in PBS, and then mounted in Prolong Antifade Mounting Media from Molecular Probes (Eugene, Oregon).

### RNA extraction

Total RNA was extracted from guinea pig cochlear tissue by immediately placing the bulla in ice-cold RNAlater solution, (Ambion, Austin, TX) and then quickly completing the dissection. The soft tissue was homogenized in the tissue lysis buffer provided in RNeasy Mini kit, (Qiagen, Chatworth, CA). The tissue was sheared by passing through an 18-gauge needle 5 times and then centrifuged through a QIAShredder, (Qiagen, Chatworth, CA), for efficient homogenization. The supernatant was collected and processed further using the RNeasy Mini kit according to the manufacturer's protocol. Human inner ear tissue was collected directly into RNAlater solution from patients undergoing translabyrinthine surgery who had given informed consent to use the tissue that would otherwise be discarded. The RNA was extracted from human tissue using the same extraction protocol. Total RNA was isolated from trypsinized, washed and pelleted human cell lines using the RNeasy midi kit (Qiagen, Chatworth, CA).

### Reverse-Transcription Polymerase Chain Reaction (RT-PCR)

For the RT reaction, 2 µg of total RNA was used to synthesize sscDNA. Both oligo dT and random primers were used with M-MLV reverse transcriptase (Invitrogen Life Technologies (Carlsbad, CA)) according to the manufacturer's instructions. To amplify the CTL2 cDNA, seven sets of sense and antisense primers were designed using the Primer 3 Input software (Howard Hughes Medical Institute, NIH, National Human Genome Research Institute) based on the human CTL2 gene sequence. To sequence the guinea pig CTL2 cDNA, a back translation algorithm (ExPasY, Swiss Institute of Bioinformatics Web Site) was used to deduce the guinea pig coding region from the amino acid sequence. This information was then was used to design initial primer sets, after which a walking primer strategy was used so that the next set of primers was designed based on the sequence of the product from a previous successful PCR reaction. Long-range guinea pig primers based on these known sequences were then designed. All eight primer sets are shown in Table 6 (below).

PCR was performed using 2µL of cDNA, 0.5 mM of dNTP, 0.2µM of each primer, in 10X buffer with MgCl and 0.625µL of *Taq* DNA Polymerase (Promega, Madison, WI]. After denaturation at 94°C for one minute, the reaction was carried out for 30 cycles at 94°C for 1 min, 58-62°C (depending on the primer set) for 1 min and 72°C for two minutes. For amplification of long sequences (i.e. greater than 1kb), the Clontech Long Distance cDNA amplification kit was used as described in the manufacturer's instructions. The elongation step at 72°C was increased to 3 minutes in each cycle. PCR products were visualized on a 1% agarose gel using ethidium bromide fluorescence. As a positive control, β-actin cDNA was amplified from both human and guinea pig using primers based on the human sequence. The PCR products were purified using the QIAquick pPCR purification kit (Qiagen, , Chatworth, CA), and submitted for sequencing to the University of Michigan DNA Sequencing Core.

### B. Results

### Immunoaffinity purification and MS/MS sequencing of the IESCA

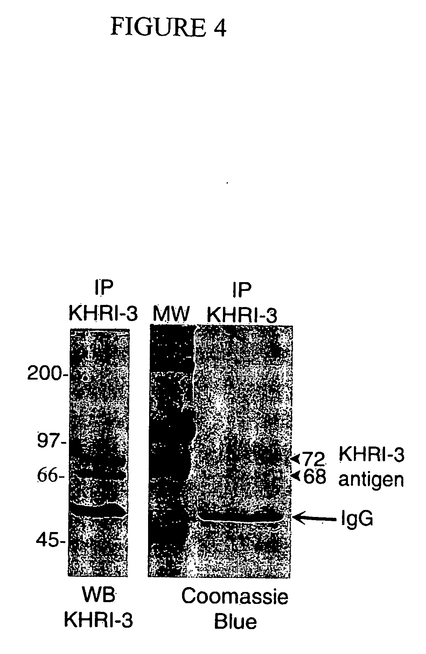

Immunoaffinity purification of the KHRI-3 defined IESCA was carried out as described in the methods under section A. The affinity-purified proteins were subjected to SDS-PAGE, and the 68 and 72 kDa bands were identified by western blot and colloidal Coomassie blue staining, as shown in Figure 4. In the first MS/MS sequencing run, the 68 and 72 kDa bands identified by colloidal Coomassie blue staining were cut from the gel and combined in a single tube for sequencing. A control slice from an empty lane of the same gel was used as a background control for contaminating proteins. In the second sequencing experiment, the 68 kDa and 72 kDa bands were cut out as before but sent as separate samples for independent sequencing.

In the first experiment, 6 peptides with sequence identity to human CTL2, a choline transporter-like protein (6) were reported (Table 6). The goals of the second experiment were to confirm the sequencing results and to determine if the 68 and 72 kDa bands were both derived from this protein. As can be seen in Table 5, multiple peptides with sequences identical to human CTL2 were identified in both the 68 and 72 kDa bands. In all, ten different peptides were identified in the three sequencing runs. The peptides are ordered from 1-10 based upon their relative location in the CTL2 protein. Most of the peptides are present in both the 68 and 72 kDa bands.

| | | | |
|---|---|---|---|
| Table 5. Peptide sequences obtained by MS/MS sequencing of the 68 and 72 kDa IESCA bands | | | |
| The letter in parentheses before each sequence is either K or R signifying that the preceding amino acid was either lysine (K) or arginine (R). | | | |

| Peptide | Sequencing Run 1 | Sequencing Run 2 | |
|---|---|---|---|
| | 68 & 72kDa | 68kDa | 72kDa |
| 1 | (K)YDPTFK (SEQ ID NO:5) | (K)YDPTFKGPIYNR (SEQ ID NO:6) | |
| 2 | (K)VIYPTDSR (SEQ ID NO:7) | (R)KVIYPTDSR (SEQ ID NO:8) | (R)KVIYPTDSR (SEQ ID NO:8) |
| 3 | (K)CASPLVLLEFQCPTPQICVEK (SEQ ID NO:9) | (K)CASPLVLLEFQCPTPQICVEK (SEQ ID NO:9) | (K)CASPLVLLEFQCPTPQICVEK (SEQ ID NO:9) |
| 4 | (K)GVAEVLR (SEQ ID NO:10) | | |
| 5 | | (R)VYLHLR (SEQ ID NO:11) | (R)VYLHLR (SEQ ID NO:11) |
| 6 | | | (R)ILIAIALIK (SEQ ID NO:12) |
| 7 | (R)CQFAFYGGESGYHR (SEQ ID NO:13) | (R)CQFAFYGGESGYHR (SEQ ID NO:13) | (R)CQFAFYGGESGYHR (SEQ ID NO:13) |
| 8 | (R)NAFFLLMR (SEQ ID NO: 14) | (R)NAFFLLMR (SEQ ID NO:14) | (R)NAFFLLMR (SEQ ID NO:14) |
| 9 | | | (K)VTDFLFLLGK (SEQ ID NO:15) |
| 10 | | (R)NDGSAERPYFMSSTLK (SEQ ID NO:16) | (R)NDGSAERPYFMSSTLK (SEQ ID NO: 16) |

### CTL2 Structure

A diagrammatic representation of the predicted structure of the CTL2 protein in the cell membrane is shown in Figure 5. This structure was predicted using Swiss Prot Expert Protein Analysis System (ExPASy) based on the various domains within the protein and the conformation with the lowest predicted entropy. The lowest entropy calculation of CTL2 is predicted to have ten helical transmembrane domains with both the N-terminal and C-terminal portions residing in the cytoplasmic domain. The human CTL2 open reading frame includes 22 exons mapped to human chromosome 19p13.1 (accession number AJ245621 O'Regan *et al*.) and encodes a protein of 706 amino acids. Each of the 10 guinea pig peptides identified by MS/MS sequencing (Table 5) are identical in humans and guinea pig. Colored regions that are labeled with circled numerals from 1 to 10 indicate the location of each peptide in the model. The fine black lines in the figure that cross the backbone of the protein show the relative locations of the boundaries of the 22 exons. Red and green circles in the backbone indicate the approximate locations of half-cysteine and arginine residues, respectively. Predicted N-glycosylation sites in the extracellular loops were determined using PROSITE (7) and are shown with black arrowheads.

### Rabbit antisera to CTL2 peptides ELISA and Immunofluorescence

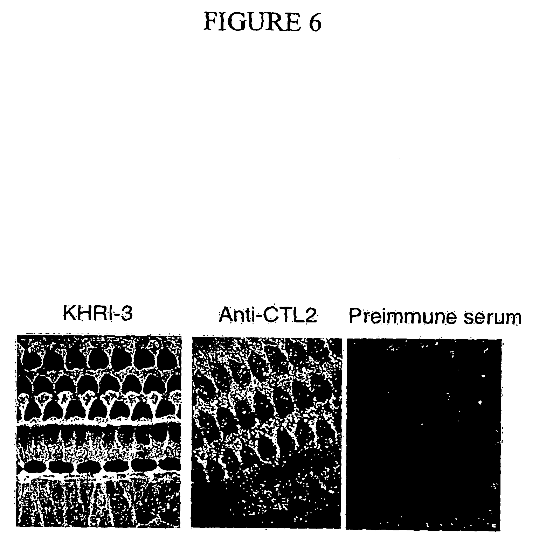

To determine if CTL2 and the IESCA defined by KHRI-3 are the same molecule, CTL2 specific antibodies were prepared by immunizing rabbits with a CTL2 peptide. A hydrophobicity and antigenicity plot of the entire protein was constructed using Lasergene-DNA Star software. From this plot, an antigenic segment in the N-terminal domain of CTL2 that includes peptide 1 (Green bar in figure 5, to the right of the N terminal sequence line) isolated from the guinea pig inner ear (Table 5) was selected. The full sequence of the synthesized peptide sequence is HGTPQKYDPTFKGPIYNR (SEQ ID NO:17), which corresponds to all of the amino acids encoded by exon 2 with overlap into exon 1 and 3. This synthetic peptide is comprised of the 13 amino acids in peptide 1 plus the next 5 upstream amino acids. A dark blue bar in Figure 5 adjacent to peptide 1 (to the left of the N terminal sequence line), shows the location of the synthetic peptide in the CTL2 protein. Rabbit pre-immune sera and antisera from each bleeding of two rabbits were tested by ELISA against the immunizing peptide. ELISA results indicated that all the post immune sera had strong binding to the CTL2 peptide, whereas pre-immune serum did not. The anti-CTL2 sera were tested by immunofluorescence staining of guinea pig inner ear surface preparations and compared to staining by the KHRI-3 antibody, as shown in Figure 6. The staining pattern obtained with the rabbit anti CTL2 serum is remarkably similar to the typical KHRI-3 binding pattern of strong staining of the phalangeal processes of the outer pillar cells, Deiters' cells, and other supporting cells. As shown, the pre-immune serum had no specific binding on guinea pig inner ear.

### Immunoprecipitation and western blotting

To determine if the IESCA identified by KHRI-3 is identical to the CTL2 antigen recognized by the rabbit antisera, immunoprecipitation and western blotting was used. Proteins were precipitated from guinea pig inner ear extracts with CNBr coupled KHRI-3 monoclonal antibody, subjected to SDS PAGE under non-reducing conditions, transferred to nitrocellulose filters, and western blotted with either KHRI-3 antibody or with the anti-CTL2 sera from rabbits R228 and R229. As shown in Figure 7A, when guinea pig inner ear extracts were immunoprecipitated with KHRI-3 and then Western blotted with the KHRI-3 antibody or with either of the rabbit anti-CTL2 antisera, identical staining patterns were obtained with all three antibody reagents.

To further confirm the identity of the molecules identified by the murine and rabbit antibodies, a second immunoprecipitation of guinea pig inner ear extracts was carried out using anti-CTL2 serum (R229) coupled to CNBr beads, KHRI-3 antibody coupled to CNBr beads, and rabbit and mouse IgG coupled to CNBr beads as a controls. The immunoprecipitates were electrophoresed along with whole cochlear lysates (WCL) and then the blots were probed with either KHRI-3 antibody (left panel) or with the R229 anti-CTL2 antiserum (right panel). As shown in Figure 7B, both KHRI-3 and the rabbit anti CTL2 antibodies bind to 68 and 72kDa bands in the whole cell lysate and also in the immunoprecipitates, indicating that both reagents identify the same proteins. In contrast, there was no such staining of material in the mock precipitates from either the rabbit or mouse IgG conjugates. In addition to the 68 and 72 kDa bands identified by KHRI-3, the anti-CTL2 antibody stains a faint 80kD band in the lanes loaded with whole cell lysate.

### RT-PCR Results

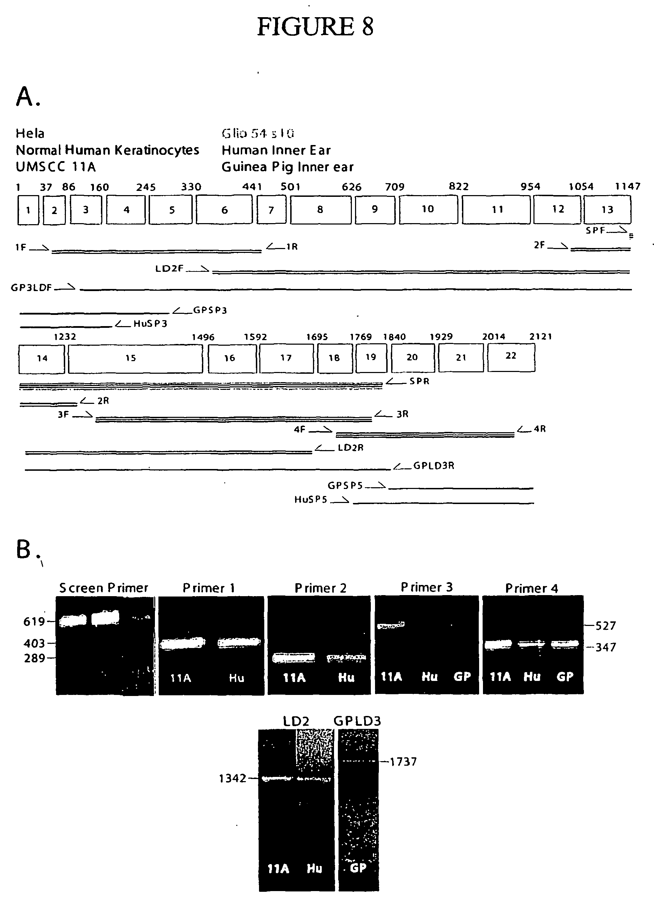

To detect CTL2 messenger RNA expression, total RNA was screened from four human tumor cell lines, human keratinocytes, and inner ear from guinea pig and humans. The first primers were designed using Primer3 Output from the Whitehead Institute MIT web site. These primers, SPF and SPR (screen primers forward and screen primers reverse) (Table 6 and Figure 8), were used to screen a series of human cell lines for CTL2 expression. cDNAs from normal human keratinocytes (NHK), a glioblastoma (Glio54s10), a neuroblastoma, (SJNB20) and a squamous cell carcinoma (UM-SCC-11A), all resulted in a 619 bp PCR product (Figure 8B left panel) with sequence identity to the predicted CTL2 sequence. However, this primer set did not work with RNA from guinea pig inner ear. Nevertheless, the knowledge that CTL2 is expressed in some human cells allowed the use of RNA from UM-SCC-11A (shown as 11A in the figure) for testing additional primers based on the peptide sequences obtained from MS/MS sequencing from the guinea pig protein.

Four sets of PCR primers were designed that should amplify both human and guinea pig CTL2. In primer set 1, the forward primer corresponds to sequences in peptide 1, and the reverse primer to peptide 4 (Tables 5, 6, and Figure 8A). In primer set 2, the forward primer is located in peptide 6, and the reverse primer is in peptide 7. Primer set 3 begins in peptide 7 and ends in peptide 9, and primer set 4 begins in peptide 8 and ends in peptide 10. Results of representative RT-PCR reactions with human inner ear RNA (Hu) and guinea pig inner ear RNA (GP) are shown in Figure 8B. All four sets of primers amplified products from RNA from human inner ear tissue and from UM-SCC-11A. Sequencing of the PCR products from all four primer sets revealed that the products are identical to the expected CTL2 sequence in both human tissues.

To complete the human sequence, long distance primers LD2F and LD2R were designed within the regions amplified by primer sets 1 and 4 (Figure 8A). These primers amplified cDNA from both human inner ear and UM-SCC-11A, giving the correct human CTL2 sequence for both samples. Primer sets 3 and 4 also produced PCR products from guinea pig inner ear RNA (Figure 8B), yielding sequences that are homologous to human CTL2, 89% and 86% respectively.

No PCR products were obtained from guinea pig inner ear with primer sets 1 and 2. Because the PCR primers were based on the human amino acid sequence within peptides expressed in the guinea pig, this failure was attributed to species differences in codon preference between guinea pig and human RNA. To overcome this problem, we designed a new guinea pig specific reverse primer was designed, based upon the guinea pig cDNA sequence obtained with primer set 4. This primer, GPLD3R (guinea pig long distance reverse), begins at nucleotide 1851 (Figure 8A). A new forward primer was designed within peptide three beginning at nucleotide 115 (GPLD3F). This primer set successfully amplified a 1737 bp segment from guinea pig inner ear cDNA (last panel, Figure 8B). The guinea pig PCR product was sequenced from 3' to 5' (86% homology to human CTL2) and 5' to 3' (85% homology to human CTL2).

At this point the expressed inner ear human and guinea pig sequences were known except for the 3' and 5' ends. Using the known sequences for both species, a final set of guinea pig specific primers (called GPSP3 and GPSP5) and a final set of human primers (called HuSP3 and HuSP5) were designed to obtain the remaining 3' and 5' sequences using rapid amplification of cDNA ends (RACE). With these primers, the 3' and 5'guinea pig sequences were obtained resulting in the complete sequence of the guinea pig CTL2 cDNA sequence (as shown in Figure 11, SEQ ID NO:3; the encoded amino acid sequence for guinea pig CTL2 is shown in Figure 12, SEQ ID NO:4), and confirmed that the expressed product in human inner ear is identical to the expected CTL2 sequence.

From these results, it can be concluded that CTL2 or a closely related homologue is expressed in inner ear in both humans and guinea pig and in cultured human epithelial cells.

| | | | |
|---|---|---|---|
| Table 6. PCR primer sets for sequencing human and guinea pig CTL2 cDNAs | | | |

| Primer set | Forward 5' - 3' (SPF) | Reverse 5' - 3' (SPR) | Product size (bp) |
|---|---|---|---|
| Screen primers | TGTGGGATACGTCATGTGCT (SEQ ID NO:18) | GCAGCATTTGAGACAGGTCA (SEQ ID NO:19) | 619 |
| 1 | CCACTTTCAAAGGACCCATTTAC (SEQ ID NO:20) | CGAAGCACCTCAGCCACTC (SEQ ID NO:21) | 403 |
| 2 | CTCATCGCGATTGCACTCAT (SEQ ID NO:22) | CCGACTCACCACCGTAGAAG (SEQ ID NO:23) | 289 |
| 3 | GCCTTCTACGGTGGTGAGTC (SEQ ID NO:24) | CCCAACAGGAAGAGGAAGTC (SEQ ID NO:25) | 527 |
| 4 | ATGCCTTCTTCCTGCGCAT (SEQ ID NO:26) | TTTCTTGAGGGTGGAAGACAT (SEQ ID NO:27) | 347 |
| GP 5' RACE GPSP3 | | GGGCTGGCACACTTTACAAT (SEQ ID NO:28) | |
| GP 3' RACE GPSP5 | GGGATTCTGGCTTTCTTCTT (SEQ ID NO:29) | | |
| Long Distance 2 | CTACCTCACGTACCTGAATGCTC (SEQ ID NO:30) | AATCATGATGTAGGCATTCCTATTAGG (SEQ ID NO:31) | |
| Guinea Pig LD | AGGTGATCTACCCCACTGATAGC (SEQ ID NO:32) | GGGACCCAGTAATAGTTGAGAGG (SEQ ID NO:33) | 1737 |
| RACE 3' HSP5 | TCATCAGAGTGGCTGTCCTG (SEQ ID NO:34) | | 359 |
| RACE 5' HSP3 | | TTTCGAGGGTCTCCATGAGT (SEQ ID NO:35) | 185 |

To confirm the expression of the CTL2 protein in the human inner ear, tissue from the vestibular system of a patient undergoing a translabyrinthine surgical procedure was obtained and used immunofluorescence to detect expression. The results showed bright punctate staining of the supporting cells surrounding the sensory cells. This staining pattern is very similar to the pattern of expression of the IESCA detected by KHRI-3 antibody in guinea pig vestibular tissue as reported previously by Ptok (Ptok, M. et al. (1993) Hear. Res. 66, 245-252).

Although the invention has been described in connection with specific preferred embodiments, it should be understood that the invention as claimed should not be unduly limited to such specific embodiments. Indeed, various modifications of the described modes for carrying out the invention which are obvious to those skilled in chemistry, and molecular biology or related fields are intended to be within the scope of the following claims.

## Claims

1. A method for predicting the response of an autoimmune sensorineural hearing loss patient to immunosuppressive therapeutic treatment with steroids, comprising:
a. providing a glycoprotein from the inner-ear organ of Corti reactive with an antibody associated with sensorineural hearing loss, wherein the glycoprotein is encoded by a nucleic acid sequence of SEQ ID NO.: 1 or an amino acid sequence of SEQ ID NO.: 2; and serum from a patient suspected of having autoimmune sensorineural hearing loss;
b. contacting the glycoprotein with the serum *in vitro* or *ex vivo*; and
c. detecting binding of an antibody in the serum to the glycoprotein under conditions sufficient to effect binding of the antibody to the glycoprotein, where the response of an autoimmune sensorineural hearing loss patient to therapeutic treatment with steroids is correlated to the presence of an antibody in the patient serum which binds to the glycoprotein.

2. A kit for assaying for the presence of an antibody associated with autoimmune sensorineural hearing loss in a patient, where the kit comprises an immunopurified glycoprotein from the inner-ear organ of Corti reactive with an antibody associated with sensorineural hearing loss, wherein the glycoprotein is encoded by a nucleic acid sequence of SEQ ID NO.: 1 or an amino acid sequence of SEQ ID NO.: 2; and an antibody that binds to the glycoprotein.

3. The method of Claim 1, wherein a patient with an antibody that binds to the glycoprotein is more likely to have improvement in hearing after steroid therapy than a patient without the antibody that binds to the glycoprotein.

4. The kit of Claim 2, wherein the antibody is KHRI-3.

## Patentansprüche

1. Ein Verfahren zur Prognose der Reaktion eines an autoimmunen sensorineuralen Hörverlust leidenden Patienten auf eine immunsuppresive therapeutische Behandlung mit Steroiden, umfassend:
a. die Bereitstellung eines Glykoproteins aus dem inneren Corti-Organ, welches mit einem Antikörper reagiert, der mit sensorineuralen Hörverlust assoziiert ist, wobei das Glykoprotein durch die Nukleinsäuresequenz SEQ ID Nr.: 1 oder die Aminosäuresequenz SEQ ID Nr.: 2 kodiert wird; und Serum von einem Patienten, bei dem der Verdacht auf autoimmunen sensorineuralen Hörverlust besteht;
b. in Kontakt bringen des Glykoproteins mit dem Serum *in vitro* oder *ex vivo*; und
c. die Detektion der Bindung eines Antikörpers aus dem Serum an das Glykoprotein unter Bedingungen, die hinreichend sind um eine Bindung des Antikörpers an das Glykoprotein herbeizuführen, wobei die Reaktion eines an autoimmunen sensorineuralen Hörverlust leidenden Patienten auf eine immunsuppresive therapeutische Behandlung mit Steroiden korreliert ist mit der Anwesenheit eines Antikörpers in dem Patientenserum, der an das Glykoprotein bindet.

2. Ein Kit zur Untersuchung der Anwesenheit eines mit autoimmunen sensorineuralen Hörverlust assoziierten Antikörpers bei einem Patienten, wobei der Kit ein immungereinigtes Glykoprotein aus dem inneren Corti-Organ, welches mit einem Antikörper reagiert, der mit sensorineuralen Hörverlust assoziiert ist, wobei das Glykoprotein durch die Nukleinsäuresequenz SEQ ID Nr.: 1 oder die Aminosäuresequenz SEQ ID Nr.: 2 kodiert wird; und einem an das Glykoprotein bindenden Antikörper umfasst.

3. Das Verfahren nach Anspruch 1, wobei der Patient mit dem an das Glykoprotein bindenden Antikörper eine höhere Erfolgsaussicht auf besseres Hören nach der Stereoidbehandlung hat, als ein Patient ohne den an das Glykoprotein bindenden Antikörper.

4. Der Kit nach Anspruch 2, wobei der Antikörper KHRI-3 ist.

## Revendications

1. Procédé de prévision de la réponse d'un patient souffrant d'une perte d'audition sensoneurinale auto-immune à un traitement thérapeutique immunosuppresseur avec des stéroïdes, comprenant :
a. la fourniture d'une glycoprotéine de l'organe de Corti de l'oreille interne réactive à un anticorps associé à une perte d'audition sensoneurinale, la glycoprotéine étant codée par une séquence d'acides nucléiques de SEQ ID N° : 1 ou une séquence d'acides aminés de SEQ ID N° : 2 ; et de sérum d'un patient suspecté de présenter une perte d'audition sensoneurinale auto-immune ;
b. la mise en contact de la glycoprotéine avec le sérum *in vitro* ou *ex vivo* ; et
c. la détection de la liaison d'un anticorps dans le sérum à la glycoprotéine dans des conditions suffisantes pour effectuer une liaison de l'anticorps à la glycoprotéine, dans lequel la réponse d'un patient souffrant d'une perte d'audition sensoneurinale auto-immune au traitement thérapeutique avec des stéroïdes est corrélée à la présence d'un anticorps dans le sérum du patient qui se lie à la glycoprotéine.

2. Kit pour tester la présence d'un anticorps associé à une perte d'audition sensoneurinale auto-immune chez un patient, le kit comprenant une glycoprotéine immunopurifiée de l'organe de Corti de l'oreille interne, réactive à un anticorps associé à une perte d'audition sensoneurinale, la glycoprotéine étant codée par une séquence d'acides nucléiques de SEQ ID N° : 1 ou une séquence d'acides aminés de SEQ ID N° : 2, et un anticorps qui se lie à la glycoprotéine.

3. Procédé selon la revendication 1, dans lequel un patient présentant un anticorps qui se lie à la glycoprotéine est plus susceptible d'avoir une amélioration de l'audition après un traitement aux stéroïdes qu'un patient qui ne présente pas l'anticorps qui se lie à la glycoprotéine.

4. Kit selon la revendication 2, dans lequel l'anticorps est KHRI-3.
